# EUROPEAN PATENT SPECIFICATION

(11) **EP 0 935 657 B1**
(45) Date of publication and mention of the grant of the patent: **29.08.2007**
(21) Application number: 97949348.3
(22) Date of filing: 28.10.1997
(51) Int. Cl.: C12N 15/12, C12N 15/62, C07K 14/72, A61K 38/17, A01K 67/027

(54) **MODIFIED STEROID HORMONE RECEPTORS**
MODIFIZIERTE STEROID-HORMON REZEPTOREN
RECEPTEURS D'HORMONES STEROIDIENNES MODIFIES

(30) Priority: 29.10.1996 US 29964 P
(43) Date of publication of application: 18.08.1999
(73) Proprietor: BAYLOR COLLEGE OF MEDICINE, Houston, TX 77381 (US)
(72) Inventor: O'MALLEY, Bert, Houston, TX 77079 (US); TSAI, Ming-Jer, Houston, TX 77005 (US); TSAI, Sophia, Y., Houston, TX 77005 (US); WANG, Yaolin, Iselin, NJ 08830 (US)
(74) Representative: Williams, Richard Andrew Norman
(86) International application number: PCT/US1997/019607
(87) International publication number: WO 1998/018925

(56) References cited:
- EP-A- 0 371 820
- WO-A-90/07517
- WO-A-90/14356
- WO-A-92/22567
- WO-A-93/23431
- WO-A-96/40911
- VINCENT LAUDET, DOMINIQUE ST¹HELIN: "Les récepteurs nucléaires" POUR LA SCIENCE, PARIS, vol. 183, January 1993, pages 32-39, XP002058341
- KELLENDONK ET AL.: "Regulation of Cre recombinase activity by the synthetic steroid RU 486" NUCLEIC ACIDS RESEARCH., vol. 24, no. 8, OXFORD GB, pages 1404-1411, XP002071703
- VELDSCHOLTE J ET AL.: "Anti-androgens and the mutated androgen receptor of LNCaP cells: differential effects on binding affinity, heat-shock protein interaction, and transcription activation." BIOCHEMISTRY., vol. 31, 1992, EASTON, PA US, pages 2393-2399, XP002071704
- LANZ RB;RUSCONI S: "A conserved carboxy-terminal subdomain is important for ligand interpretation and transactivation by nuclear receptors." ENDOCRINOLOGY, vol. 135, no. 5, 1994, pages 2183-2195, XP002071705
- LEWIN, BENJAMIN: "Genes V" 1994 , OXFORD UNIVERSTITY PRESS , OXFORD, GB XP002058342 205070 see page 889, column 1, line 31 - page 897, column 2, line 14
- MALCHOFF D M ET AL: "A MUTATION OF THE GLUCOCORTICOID RECEPTOR IN PRIMARY CORTISOL RESISTANCE" JOURNAL OF CLINICAL INVESTIGATION, vol. 91, no. 5, May 1993, pages 1918-1925, XP000610522
- WURTZ J -M ET AL: "A CANONICAL STRUCTURE FOR THE LIGAND-BINDING DOMAIN OF NUCLEAR RECEPTORS" NATURAL STRUCTURAL BIOLOGY, vol. 3, 1996, pages 87-94, XP000610199
- LANZ, R.B. ETAL.;: "Active, interactive, and inactive steroid receptos mutants" STEOIDS, no. 59, September 1994, pages 148-152, XP002071954
- WANG Y. ET AL.: "A regulatory system for use in gene transfer" PROC.NATL.ACAD.SCI.USA, vol. 91, 1994, pages 8180-8184, XP002121654
- BRASELMANN S. ET AL.: "A selective transcriptional induction system for mammalian cells based on Gal4-estrogen receptor fusion proteins" PROC.NATL.ACAD.SCI.USA, vol. 90, 1993, pages 1657-1661, XP002062028
- WANG ET AL.: "Positive and negative regulation of gene expression in eukaryotic cells with an inducible transcriptional regulator" GENE THERAPY, vol. 4, 1997, pages 432-441, XP002130261
- LIPKIN ET AL.: "Constitutive retinoid receptors expressed from adenovirus vectors that specifically activate chromosomal target genes required for differentiation of promyelocytic leukemia and teratocarcinoma cells" J.VIROL., vol. 70, no. 10, October 1996 (1996-10), pages 7182-7189,

### SEQUENCE LISTING

(1) GENERAL INFORMATION:
   (i) APPLICANT: O'Malley, Bert W. Tsai, Ming-Jer Ledebur, Harry C. Jr. Kittle, Joseph D. Jr.
   (ii) TITLE OF INVENTION: MODIFIED STEROID HORMONES FOR GENE THERAPY AND METHODS FOR THEIR USE
   (iii) NUMBER OF SEQUENCES: 14
   (iv) CORRESPONDENCE ADDRESS:
      (A) ADDRESSEE: Lyon & Lyon
      (B) STREET: 633 West Fifth Street Suite 4700
      (C) CITY: Los Angeles
      (D) STATE: California
      (E) COUNTRY: U.S.A.
      (F) ZIP: 90071-2066
   (v) COMPUTER READABLE FORM:
      (A) MEDIUM TYPE: 3.5" Diskette, 1.44 Mb storage
      (B) COMPUTER: IBM Compatible
      (C) OPERATING SYSTEM: IBM P.C. DOS 5.0
      (D) SOFTWARE: Word Perfect 5.1
   (vi) CURRENT APPLICATION DATA:
      (A) APPLICATION NUMBER: To Be Assigned
      (B) FILING DATE: Herewith
      (C) CLASSIFICATION:
   (vii) PRIOR APPLICATION DATA:
      (A) APPLICATION NUMBER: 08/479,913
      (B) FILING DATE: June 7, 1995

      (A) APPLICATION NUMBER: 07/939,246
      (B) FILING DATE: September 2, 1992
   (viii) ATTORNEY/AGENT INFORMATION:
      (A) NAME: Warburg, Richard J.
      (B) REGISTRATION NUMBER: 32,327
      (C) REFERENCE/DOCKET NUMBER: 222/085
   (ix) TELECOMMUNICATION INFORMATION:
      (A) TELEPHONE: (213) 489-1600
      (B) TELEFAX: (213) 955-0440
      (C) TELEX: 67-3510
(2) INFORMATION FOR SEQ ID NO: 1:
   (i) SEQUENCE CHARACTERISTICS:
      (A) LENGTH: 6177 base pairs
      (B) TYPE: nucleic acid
      (C) STRANDEDNESS: double
      (D) TOPOLOGY: linear
   (ii) MOLECULE TYPE: nucleic acid
   (xi) SEQUENCE DESCRIPTION: SEQ ID NO: 1:
   CTAGAGTCGA CCTGCAGCCC AAGCTCTCGA GGGATCCTGA GAACTTCAGG GTGAGTTTGG   60
   GGACCCTTGA TTGTTCTTTC TTTTTCGCTA TTGTAAAATT CATGTTATAT GGAGGGGGCA  120
   AAGTTTTCAG GGTGTTGTTT AGAATGGGAA GATGTCCCTT GTATCACCAT GGACCCTCAT  180
   GATAATTTTG TTTCTTTCAC TTTCTACTCT GTTGACAACC ATTGTCTCCT CTTATTTTCT  240
   TTTCATTTTC TGTAACTTTT TCGTTAAACT TTAGCTTGCA TTTGTAACGA ATTTTTAAAT  300
   TCACTTTTGT TTATTTGTCA GATTGTAAGT ACTTTCTCTA ATCACTTTTT TTTCAAGGCA  360
   ATCAGGGTAT ATTATATTGT ACTTCAGCAC AGTTTTAGAG AACAATTGTT ATAATTAAAT  420
   GATAAGGTAG AATATTTCTG CATATAAATT CTGGCTGGCG TGGAAATATT CTTATTGGTA  480
   GAAACAACTA CATCCTGGTC ATCATCCTGC CTTTCTCTTT ATGGTTACAA TGATATACAC  540
   TGTTTGAGAT GAGGATAAAA TACTCTGAGT CCAAACCGGG CCCCTCTGCT AACCATGTTC  600
   ATGCCTTCTT CTTTTTCCTA CAGCTCCTGG GCAACGTGCT GGTTGTTGTG CTGTCTCATC  660
   ATTTTGGCAA AGAATTCACT CCTCAGGTGC AGGCTGCCTA TCAGAAGGTG GTGGCTGGTG  720
   TGGCCAATGC CCTGGCTCAC AAATACCACT GAGATCTTTT TCCCTCTGCC AAAAATTATG  780
   GGGACATCAT GAAGCCCCTT GAGCATCTGA CTTCTGGCTA ATAAAGGAAA TTTATTTTCA  840
   TTGCAATAGT GTGTTGGAAT TTTTTGTGTC TCTCACTCGG AAGGACATAT GGGAGGGCAA  900
   ATCATTTAAA ACATCAGAAT GAGTATTTGG TTTAGAGTTT GGCAACATAT GCCATATGCT  960
   GGCTGCCATG AACAAAGGTG GCTATAAAGA GGTCATCAGT ATATGAAACA GCCCCCTGCT 1020
   GTCCATTCCT TATTCCATAG AAAAGCCTTG ACTTGAGGTT AGATTTTTTT TATATTTTGT 1080
   TTTGTGTTAT TTTTTTCTTT AACATCCCTA AAATTTTCCT TACATGTTTT ACTAGCCAGA 1140
   TTTTTCCTCC TCTCCTGACT ACTCCCAGTC ATAGCTGTCC CTCTTCTCTT ATGAACTCGA 1200
   GGAGCTTTTT GCAAAAGCCT AGGCCTCCAA AAAAGCCTCC TCACTACTTC TGGAATAGCT 1260
   CAGAGGCCGA GGCGGCCTCG GCCTCTGCAT AAATAAAAAA AATTAGTCAG CCATGGGGCG 1320
   GAGAATGGGC GGAACTGGGC GGAGTTAGGG GCGGGATGGG CGGAGTTAGG GGCGGGACTA 1380
   TGGTTGCTGA CTAATTGAGA CTGCATTAAT GAATCGGCCA ACGCGCGGGG AGAGGCGGTT 1440
   TGCGTATTGG GCGCTCTTCC GCTTCCTCGC TCACTGACTC GCTGCGCTCG GTCGTTCGGC 1500
   TGCGGCGAGC GGTATCAGCT CACTCAAAGG CGGTAATACG GTTATCCACA GAATCAGGGG 1560
   ATAACGCAGG AAAGAACATG TGAGCAAAAG GCCAGCAAAA GGCCAGGAAC CGTAAAAAGG 1620
   CCGCGTTGCT GGCGTTTTTC CATAGGCTCC GCCCCCCTGA CGAGCATCAC AAAAATCGAC 1680
   GCTCAAGTCA GAGGTGGCGA AACCCGACAG GACTATAAAG ATACCAGGCG TTTCCCCCTG 1740
   GAAGCTCCCT CGTGCGCTCT CCTGTTCCGA CCCTGCCGCT TACCGGATAC CTGTCCGCCT 1800
   TTCTCCCTTC GGGAAGCGTG GCGCTTTCTC AATGCTCACG CTGTAGGTAT CTCAGTTCGG 1860
   TGTAGGTCGT TCGCTCCAAG CTGGGCTGTG TGCACGAACC CCCCGTTCAG CCCGACCGCT 1920
   GCGCCTTATC CGGTAACTAT CGTCTTGAGT CCAACCCGGT AAGACACGAC TTATCGCCAC 1980
   TGGCAGCAGC CACTGGTAAC AGGATTAGCA GAGCGAGGTA TGTAGGCGGT GCTACAGAGT 2040
   TCTTGAAGTG GTGGCCTAAC TACGGCTACA CTAGAAGGAC AGTATTTGGT ATCTGCGCTC 2100
   TGCTGAAGCC AGTTACCTTC GGAAAAAGAG TTGGTAGCTC TTGATCCGGC AAACAAACCA 2160
   CCGCTGGTAG CGGTGGTTTT TTTGTTTGCA AGCAGCAGAT TACGCGCAGA AAAAAAGGAT 2220
   CTCAAGAAGA TCCTTTGATC TTTTCTACGG GGTCTGACGC TCAGTGGAAC GAAAACTCAC 2280
   GTTAAGGGAT TTTGGTCATG AGATTATCAA AAAGGATCTT CACCTAGATC CTTTTAAATT 2340
   AAAAATGAAG TTTTAAATCA ATCTAAAGTA TATATGAGTA AACTTGGTCT GACAGTTACC 2400
   AATGCTTAAT CAGTGAGGCA CCTATCTCAG CGATCTGTCT ATTTCGTTCA TCCATAGTTG 2460
   CCTGACTCCC CGTCGTGTAG ATAACTACGA TACGGGAGGG CTTACCATCT GGCCCCAGTG 2520
   CTGCAATGAT ACCGCGAGAC CCACGCTCAC CGGCTCCAGA TTTATCAGCA ATAAACCAGC 2580
   CAGCCGGAAG GGCCGAGCGC AGAAGTGGTC CTGCAACTTT ATCCGCCTCC ATCCAGTCTA 2640
   TTAATTGTTG CCGGGAAGCT AGAGTAAGTA GTTCGCCAGT TAATAGTTTG CGCAACGTTG 2700
   TTGCCATTGC TACAGGCATC GTGGTGTCAC GCTCGTCGTT TGGTATGGCT TCATTCAGCT 2760
   CCGGTTCCCA ACGATCAAGG CGAGTTACAT GATCCCCCAT GTTGTGCAAA AAAGCGGTTA  2820
   GCTCCTTCGG TCCTCCGATC GTTGTCAGAA GTAAGTTGGC CGCAGTGTTA TCACTCATGG 2880
   TTATGGCAGC ACTGCATAAT TCTCTTACTG TCATGCCATC CGTAAGATGC TTTTCTGTGA 2940
   CTGGTGAGTA CTCAACCAAG TCATTCTGAG AATAGTGTAT GCGGCGACCG AGTTGCTCTT 3000
   GCCCGGCGTC AATACGGGAT AATACCGCGC CACATAGCAG AACTTTAAAA GTGCTCATCA 3060
   TTGGAAAACG TTCTTCGGGG CGAAAACTCT CAAGGATCTT ACCGCTGTTG AGATCCAGTT 3120
   CGATGTAACC CACTCGTGCA CCCAACTGAT CTTCAGCATC TTTTACTTTC ACCAGCGTTT 3180
   CTGGGTGAGC AAAAACAGGA AGGCAAAATG CCGCAAAAAA GGGAATAAGG GCGACACGGA 3240
   AATGTTGAAT ACTCATACTC TTCCTTTTTC AATATTATTG AAGCATTTAT CAGGGTTATT 3300
   GTCTCATGAG CGGATACATA TTTGAATGTA TTTAGAAAAA TAAACAAATA GGGGTTCCGC 3360
   GCACATTTCC CCGAAAAGTG CCACCTGACG TCTAAGAAAC CATTATTATC ATGACATTAA   3420
   CCTATAAAAA TAGGCGTATC ACGAGGCCCT TTCGTCTTCA AGCTGCCTCG CGCGTTTCGG   3480
   TGATGACGGT GAAAACCTCT GACACATGCA GCTCCCGGAG ACGGTCACAG CTTGTCTGTA   3540
   AGCGGATGCC GGGAGCAGAC AAGCCCGTCA GGGCGCGTCA GCGGGTGTTG GCGGGTGTCG   3600
   GGGCGCAGCC ATGACCCAGT CACGTAGCGA TAGCGGAGTT GGCTTAACTA TGCGGCATCA   3660
   GAGCAGATTG TACTGAGAGT GCACCATATC GACGCTCTCC CTTATGCGAC TCCTGCATTA   3720
   GGAAGCAGCC CAGTAGTAGG TTGAGGCCGT TGAGCACCGC CGCCGCAAGG AATGGTGCTG   3780
   GCTTATCGAA ATTAATCGAC TCACTATAGG GAGACCCGAA TTCGAGCTCG CCCCGTTACA   3840
   TAACTTACGG TAAATGGCCC GCCTGGCTGA CCGCCCAACG ACCCCCGCCC ATTGACGTCA   3900
   ATAATGACGT ATGTTCCCAT AGTAACGCCA ATAGGGACTT TCCATTGACG TCAATGGGTG   3960
   GAGTATTTAC GGTAAACTGC CCACTTGGCA GTACATCAAG TGTATCATAT GCCAAGTACG   4020
   CCCCCTATTG ACGTCAATGA CGGTAAATGG CCCGCCTGGC ATTATGCCCA GTACATGACC   4080
   TTATGGGACT TTCCTACTTG GCAGTACATC TACGTATTAG TCATCGCTAT TACCATGGTG   4140
   ATGCGGTTTT GGCAGTACAT CAATGGGCGT GGATAGCGGT TTGACTCACG GGGATTTCCA   4200
   AGTCTCCACC CCATTGACGT CAATGGGAGT TTGTTTTGGC ACCAAAATCA ACGGGACTTT   4260
   CCAAAATGTC GTAACAACTC CGCCCCATTG ACGCAAATGG GCGGTAGGCG TGTACGGTGG   4320
   GAGGTCTATA TAAGCAGAGC TCGTTTAGTG AACCGTCAGA TCGCCTGGAG ACGCCATCCA   4380
   CGCTGTTTTG ACCTCCATAG AAGACACCGG GACCGATCCA GCCTCCGCGG GATCTTGGTG   4440
   GCGTGAAACT CCCGCACCTC TTCGGCCAGC GCCTTGTAGA AGCGCGTATG GCTTCGTGGG   4500
   GATCCCCCAA AGAATCCTTA GCTCCCCCTG GTAGAGACGA AGTCCCTGGC AGTTTGCTTG   4560
   GCCAAGGGAG GGGGAGCGTA ATGGACTTTT ATAAAAGCCT GAGGGGAGGA GCTACAGTCA   4620
   AGGTTTCTGC ATCTTCGCCC TCAGTGGCTG CTGCTTCTCA GGCAGATTCC AAGCAGCAGA   4680
   GGATTCTCCT TGATTTCTCG AAAGGCTCCA CAAGCAATGT GCAGCAGCGA CAGCAGCAGC   4740
   AGCAGCAGCA GCAGCAGCAG CAGCAGCAGC AGCAGCAGCA GCAGCAGCCA GGCTTATCCA   4800
   AAGCCGTTTC ACTGTCCATG GGGCTGTATA TGGGAGAGAC AGAAACAAAA GTGATGGGGA   4860
   ATGACTTGGG CTACCCACAG CAGGGCCAAC TTGGCCTTTC CTCTGGGGAA ACAGACTTTC   4920
   GGCTTCTGGA AGAAAGCATT GCAAACCTCA ATAGGTCGAC CAGCGTTCCA GAGAACCCCA   4980
   AGAGTTCAAC GTCTGCAACT GGGTGTGCTA CCCCGACAGA GAAGGAGTTT CCCAAAACTC   5040
   ACTCGGATGC ATCTTCAGAA CAGCAAAATC GAAAAAGCCA GACCGGCACC AACGGAGGCA   5100
   GTGTGAAATT GTATCCCACA GACCAAAGCA CCTTTGACCT CTTGAAGGAT TTGGAGTTTT   5160
   CCGCTGGGTC CCCAAGTAAA GACACAAACG AGAGTCCCTG GAGATCAGAT CTGTTGATAG   5220
   ATGAAAACTT GCTTTCTCCT TTGGCGGGAG AAGATGATCC ATTCCTTCTC GAAGGGAACA   5280
   CGAATGAGGA TTGTAAGCCT CTTATTTTAC CGGACACTAA ACCTAAAATT AAGGATACTG   5340
   GAGATACAAT CTTATCAAGT CCCAGCAGTG TGGCACTACC CCAAGTGAAA ACAGAAAAAG   5400
   ATGATTTCAT TGAACTTTGC ACCCCCGGGG TAATTAAGCA AGAGAAACTG GGCCCAGTTT   5460
   ATTGTCAGGC AAGCTTTTCT GGGACAAATA TAATTGGTAA TAAAATGTCT GCCATTTCTG   5520
   TTCATGGTGT GAGTACCTCT GGAGGACAGA TGTACCACTA TGACATGAAT ACAGCATCCC   5580
   TTTCTCAGCA GCAGGATCAG AAGCCTGTTT TTAATGTCAT TCCACCAATT CCTGTTGGTT   5640
   CTGAAAACTG GAATAGGTGC CAAGGCTCCG GAGAGGACAG CCTGACTTCC TTGGGGGCTC   5700
   TGAACTTCCC AGGCCGGTCA GTGTTTTCTA ATGGGTACTC AAGCCCTGGA ATGAGACCAG   5760
   ATGTAAGCTC TCCTCCATCC AGCTCGTCAG CAGCCACGGG ACCACCTCCC AAGCTCTGCC   5820
   TGGTGTGCTC CGATGAAGCT TCAGGATGTC ATTACGGGGT GCTGACATGT GGAAGCTGCA   5880
   AAGTATTCTT TAAAAGAGCA GTGGAAGGAC AGCACAATTA CCTTTGTGCT GGAAGAAACG   5940
   ATTGCATCAT TGATAAAATT CGAAGGAAAA ACTGCCCAGC ATGCCGCTAT CGGAAATGTC   6000
   TTCAGGCTGG AATGAACCTT GAAGCTCGAA AAACAAAGAA AAAAATCAAA GGGATTCAGC   6060
   AAGCCACTGC AGGAGTCTCA CAAGACACTT CGGAAAATCC TAACAAAACA ATAGTTCCTG   6120
   CAGCATTACC ACAGCTCACC CCTACCTTGG TGTCACTGCT GGAGGTGATT GAACCCG      6177
(2) INFORMATION FOR SEQ ID NO: 2:
   (i) SEQUENCE CHARACTERISTICS:
      (A) LENGTH: 98 base pairs
      (B) TYPE: nucleic acid
      (C) STRANDEDNESS: single
      (D) TOPOLOGY: linear
   (xi) SEQUENCE DESCRIPTION: SEQ ID NO: 2:
   GTACGTTTAA ACGCGGCGCG CCGTCGACCT GCAGAAGCTT ACTAGTGGTA CCCCATGGAG   60
   ATCTGGATCC GAATTCACGC GTTCTAGATT AATTAAGC                           98
(2) INFORMATION FOR SEQ ID NO: 3:
   (i) SEQUENCE CHARACTERISTICS:
      (A) LENGTH: 98 base pairs
      (B) TYPE: nucleic acid
      (C) STRANDEDNESS: single
      (D) TOPOLOGY: linear
   (xi) SEQUENCE DESCRIPTION: SEQ ID NO: 3:
   GGCCGCTTAA TTAATCTAGA ACGCGTGAAT TCGGATCCAG ATCTCCATGG GGTACCACTA 60
   GTAAGCTTCT GCAGGTCGAC GGCGCGCCGC GTTTAAAC 98
(2) INFORMATION FOR SEQ ID NO: 4:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (xi) SEQUENCE DESCRIPTION: SEQ ID NO: 4:
      GATCTCGGTC TCCAACAGCA ACAGCAACAG CAACAGCAAC AGGGTCTTCT G 51
(2) INFORMATION FOR SEQ ID NO: 5:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (xi) SEQUENCE DESCRIPTION: SEQ ID NO: 5:
      GATCCAGAAG ACCCTGTTGC TGTTGCTGTT GCTGTTGCTG TTGGAGACCG A 51
(2) INFORMATION FOR SEQ ID NO: 6:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO: 6:
      AATTCCCCGA GGCGGCAGCT GAAATCATCA CCAATCAGAT CT 42
(2) INFORMATION FOR SEQ ID NO: 7:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO: 7:
      TATGCCTTAC CATGTGGC 18
(2) INFORMATION FOR SEQ ID NO: 8:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO: 8:
      TTGGTCGACA AGATCATGCA TTATC 25
(2) INFORMATION FOR SEQ ID NO: 9:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO: 9:
      TTGTCGACCC GCAGTACAGA TGAAGTTG 28
(2) INFORMATION FOR SEQ ID NO: 10:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO: 10:
      TTGGTCGACC CAGCAATAAC TTCAGACATC 30
(2) INFORMATION FOR SEQ ID NO: 11:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO: 11:
      CGACAGATCT GGCTCCTGAG CAAAGAGAA 29
(2) INFORMATION FOR SEQ ID NO: 12:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 12:
      CCAGGGATCC TCTCCTTGCT GCAA 24
(2) INFORMATION FOR SEQ ID NO: 13:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO: 13:
      TCTAGTCGAC GATGGCTCCT GAGCAAAGAG AAG 33
(2) INFORMATION FOR SEQ ID NO: 14:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (xi) SEQUENCE DESCRIPTION: SEQ ID NO: 14:
      CCAGGGATCC TATCCTTGCT GCAACAG 27

## Description

This invention relates to gene therapy, whereby modified steroid receptors regulate the expression of genes within tissue. In particular, the modified steroid receptors contain a DNA binding domain, one or more transregulatory domains, and a ligand binding domain and are capable of binding a non-natural ligand.

### Background of the Invention

The following description of the background of the invention is provided to aid in the understanding of the invention but is not admitted to describe or constitute prior art to the invention.

Intracellular receptors are a superfamily of related proteins that mediate the nuclear effects of steroid hormones, thyroid hormone and vitamins A and D (Evans, Science 240:889-895 (1988)). The cellular presence of a specific intracellular receptor defines that cell as a target for the cognate hormone. The mechanisms of action of the intracellular receptors are related in that they remain latent in the cytoplasm or nuclei of target cells until exposed to a specific ligand (Beato, Cell 56:335-344 (1989); O'Malley, et al., Biol. Reprod. 46:163-167 (1992)). Interaction with hormone then induces a cascade of molecular events that ultimately lead to the specific association of the activated receptor with other proteins or regulatory elements of target genes. The resulting positive or negative effects on regulation of gene transcription are determined by the cell-type and promoter-context of the target gene.

In the case of steroid hormones and steroid receptors, such complexes are responsible for the regulation of complex cellular events, including activation or repression of gene transcription. For example, the ovarian hormones, estrogen and progesterone, are responsible, in part, for the regulation of the complex cellular events associated with differentiation, growth and functioning of female reproductive tissues. Likewise, testosterone is responsible for the regulation of complex cellular events associated with differentiation growth and function of male reproductive tissues.

In addition, these hormones play important roles in development and progression of malignancies of the reproductive endocrine system. The reproductive steroids estrogen, testosterone, and progesterone are implicated in a variety of hormone-dependent cancers of the breast (Sunderland, et al., J. Clin. Oncol. 9:1283-1297 (1991)), ovary (Rao, et al., Endocr. Rev. 12:14-26 (1991)), endometrium (Dreicer, et al., Cancer Investigation 10:27-41, (1992)), and possibly prostate (Daneshgari, et al., Cancer 71:1089-1097 (1993)). In addition, the onset of post-menopausal osteoporosis is related to a decrease in production of estrogen (Barzel, Am. J. Med. 85:847-850 (1988)).

In addition, corticosteroids are potent and well-documented mediators of inflammation and immunity. They exert profound effects on the production and release of numerous humoral factors and the distribution and proliferation of various cellular components associated with the immune and inflammatory responses. For example, steroids are able to inhibit the production and release of cytokines (IL-1, IL-2, IL-3, IL-6, IL-8, TNF-α, IFN-γ), chemical mediators (eicosinoids, histamine), and enzymes (MMPs) into tissues, and directly prohibit the activation of macrophages and endothelial cells. Due to the global down-regulation of these physiological events, corticosteroids have a net effect of suppressing the inflammatory response and have therefore been used extensively to treat a variety of immunological and inflammatory disorders (rheumatoid arthritis, psoriasis, asthma, allergic rhinitis, etc.).

Besides the therapeutic benefits, however, there are some severe toxic side effects associated with steroid therapy. These include peptic ulcers, muscle atrophy, hypertension, osteoporosis, headaches, etc. Such side effects have hindered the utilization of steroids as therapeutic agents.

In general, the biological activity of steroid hormones is mediated directly by a hormone and tissue-specific intracellular receptor. Ligands are distributed through the body by the hemo-lymphatic system. The hormone freely diffuses across all membranes but manifests its biological activity only in those cells containing the tissue-specific intracellular receptor.

In the absence of ligand, the inactive steroid hormone receptors such as the glucocorticoid ("GR"), mineral corticoid ("MR"), androgen ("AR") progesterone ("PR") and estrogen ("ER") receptors are sequestered in a large complex consisting of the receptor, heat-shock proteins ("hsp") 90, hsp70 and hsp56 and other proteins as well (Smith, et al., Mol. Endo. 7:4-11 (1993)). The cellular localization of the physiologically inactive form of the oligomeric complex has been shown to be either cytoplasmic or nuclear (Picard, et al., Cell Regul. 1:291-299 (1992); Simmons, et al., J. Biol. Chem. 265:20123-20130 (1990)).

Upon binding its agonist or antagonist ligand, the receptor changes conformation and dissociates from the inhibitory heteromeric complex (Allan, et al., J. Biol. Chem. 267:19513-19520 (1992); Allan, et al., P.N.A.S. 89:11750-11754 (1992)). In the case of GR and other related systems such as AR, MR, and PR, hormone binding elicits a dissociation of heat shock and other proteins and the release of a monomeric receptor from the complex (O'Malley, et al., Biol. Reprod. 46:163-167 (1992)). Studies from genetic analysis and *in vitro* protease digestion experiments show that conformational changes in receptor structure induced by agonists are similar but distinct from those induced by antagonists (Allan, et al., J. Biol. Chem. 267:19513-19520 (1992); Allan, et al., P.N.A.S. 89:11750-11754 (1992); Vegeto, et al., Cell 69:703-713 (1992)). However, both conformations are incompatible with hsp-binding.

Following the conformation changes in receptor structure, the receptors are capable of interacting with DNA. Studies suggest that the DNA binding form of the receptor is a dimer. In the case of GR homodimers (Tsai, et al., Cell 55:361-369 (1988)), this allows the receptor to bind to specific DNA sites in the regulatory region of target gene promoters (Beato, Cell 56:335-344 (1989)). These short nucleotide sketches are arranged as palindromic, inverted or repeated repeats *(Id.).* Specificity is determined by the sequence and the spacing of the repeated sequences (Tsai and O'Malley, Ann. Rev. Biochem. 63:451-486 (1994)). Following binding of the receptor to DNA, the hormone is responsible for mediating a second function that allows the receptor to interact specifically with the transcription apparatus. Such interaction could either provide positive or negative regulation of gene expression, *i.e*., steroid receptors are ligand-binding transcription factors, capable of not only activating but also repressing the expression of specific genes. Studies have shown, however, that repression does not require DNA binding.

For instance, when bound to their intracellular receptors, corticosteroids can affect the transcription of a variety of genes whose products play key roles in the establishment and progression of an inflamed situation. Such genes include those encoding for cytokines, chemical mediators and enzymes. Transcription of these genes can be repressed or activated depending on the transcription factors and/or regulatory sequences controlling the expression of the gene. Presently there are numerous reports documenting the effect of glucocorticoid on the expression of various genes at the transcriptional level.

In particular, the glucocorticoid receptor is a member of a family of ligand-dependent transcription factors capable of both positive and negative regulation of gene expression (Beato, FASEB J. 5:2044-2051 (1991); Pfahl, Endocr. Rev. 14:651-658, (1993); Schule, et al., Trends Genet. 7:377-381 (1991)). In its inactivated form, the GR is part of a large heteromeric complex which includes hsp90 as well as other proteins (Denis, et al., J. Biol. Chem. 262:11803-11806 (1987); Howard, et al., J. Biol. Chem. 263:3474-3481 (1988); Mendel, et al., J. Biol. Chem. 261:3758-3763 (1986); Rexin, et al., J. Biol. Chem. 267:9619-9621 (1992); Sanchez, et al., J. Biol. Chem. 260:12398-12401 (1985)), and hsp56 (Lebea, et al., J. Biol. Chem. 267:4281-4284 (1992); Pratt, J. Steroid Biochem. Mol. Biol. 46:269-279 (1993); Sanchez, J. Biol. Chem. 265:22067-22070 (1990); Yem, J. Biol. Chem. 267:2868-2871, (1992)). Binding of agonist stimulates receptor activation, dissociation from hsp90 and the other proteins (Denis, et al., Nature 333:686-688 (1988); Sanchez, et al., J. Biol. Chem. 262:6986-6991 (1987)), and nuclear translocation, prerequisites for both transactivation and transrepression.

Cloning of several members of the steroid receptor superfamily has facilitated the reconstitution of hormone-dependent transcription in heterologous cell systems and facilitated delineation of the GR activation and repression mechanisms. Subsequently, *in vivo* and *in vitro* studies with mutant and chimeric receptors have demonstrated that steroid hormone receptors are modular proteins organized into structurally and functionally defined domains. Deletion mutants of the GR have determined that the transactivation domain is located at the N-terminal amino acid sequence positioned between amino acids 272 and 400 (Jonat, et al., Cell 62:1189-1204 (1990)). A well defined 66 amino acid DNA binding domain ("DBD") has been identified and studied in detail, using both genetic and biochemical approaches (Lucibello, et al., EMBO J. 9:2827-2834 (1990)). The ligand or hormone binding domain ("LBD"), located in the carboxyl-terminal portion of the receptor, consists of about 300 amino acids (Kerppola, et al., Mol. Cell. Biol. 13:3782-3791 (1993)). The LBD has not been amenable to detailed site-directed mutagenesis, since this domain appears to fold into a complex tertiary structure, creating a specific hydrophobic pocket which surrounds the effector ligand when bound. This feature creates difficulty in distinguishing among amino acid residues that affect the overall structure of the LBD domain from those involved in a direct contact with the ligand. The LBD also contains sequences responsible for receptor dimerization, nuclear localization, hsp interactions and transactivation sequences of the receptor (Fuller, et al, FASEB J. 5:3092-3099 (1991)).

The mechanism of gene activation is generally better understood than that of repression. For transactivation, a ligand-induced conformational change, comparable to that inferred to be necessary for activation of the progesterone (Allan, et al., Proc. Natl. Acad. Sci. USA 89:11750-11754 (1992)) and estrogen (Beekman, et al., Mol. Endocrinol. 7:1266-1274 (1993)) receptors, is required for efficient activation of the transcription activating function of the receptor (Hollenberg and Evans, Cell 55:899-906 (1988); Webster, et al., Cell 54:199-207, (1988)). Furthermore, the conformational change is required for interaction of the receptor with other components of the transcription apparatus. Transactivation is mediated by a receptor dimer bound to a glucocorticoid response element ("GRE"). Such transactivation occurs exclusively by homodimerization. This is mainly achieved by a region in the second zinc finger of the receptor known as the D-loop (Umesono, et al., Cell 57:1139-1146 (1989); Dahlman-Wright, et al., J. Biol. Chem. 266:3107-3112 (1991)). The resulting homodimers then bind to the palindromic GRE to initiate the transcriptional activation process (Evans, Science 240:889-895 (1988); Cato, et al., J. Steroid Biochem. Mol. Biol. 43:63-68 (1992)).

Transrepression, on the other hand, appears to be mediated by the monomeric form of the receptor through interactions with other transcriptional factors, including AP-1 and NF_{K}-B, preventing them from carrying out their function as transcriptional activators (Hoeck, et al., EMBO J. 13:4087-4095 (1994)). Studies also show transrepression by the dimeric form of the receptor. In the case of the monomeric pathway, studies suggest that AP-1 prevents hormone-dependent activation of GR-regulated promoters through a mutually inactive complex formed either by a direct protein-protein interaction of the receptor and AP-1 or through a third partner (Miner, et al., Cell Growth Differ. 2:525-530 (1991); Pfahl, Endocrine Rev. 14:651-658 (1993)). Such transrepression of AP-1 and NF_{K}-B mediated by the monomeric form of the receptor depends on the presence of the DNA binding domain. It does not depend on the ability of the receptor to bind DNA. In the case of the dimeric form of the receptor, several studies suggest mechanisms for such GR-mediated transrepression include GR binding to a sequence overlapping a cis-acting element for another trans-acting factor, thereby displacing it from, or preventing its binding to, its cognate element (Akerblom, et al., Science 241:350-353 (1988); Drouin, et al., Mol. Cell. Biol. 9:5305-5314 (1989); Oro, et al., Cell 55:1109-1114, (1988); Stromstedt, et al., Mol. Cell. Biol. 11:3379-3383, (1991)).

As noted above, GR-mediated transrepression attributed to direct or indirect interaction of the GR with other trans-acting factors, results in inhibition of their activity and/or ability to bind to DNA (Celada, et al., J. Exp. Med. 177:691-698 (1993); Diamond, et al., Science 299:1266-1272 (1990); Gauthier, et al., Embo J. 12:5089-5096 (1993); Jonat, et al., Cell 62:1189-1204 (1990); Kutoh, et al., Mol. Cell Biol. 12:4955-4969 (1992); Lucibello, et al., Embo J. 9:2827-2834 (1990); Ray, et al., Proc. Natl. Acad. Sci. USA 91:752-756 (1994); Schule, et al., Cell. 62:1217-1226 (1990); Tverberg, et al., J. Biol. Chem. 267:17567-17573 (1992); Yang-Yen, et al., Cell 62:1205-1215 (1990); Lucibello, et al., EMBO J. 9:2827-2834 (1990)). These models require ligand binding to stimulate receptor activation, dissociation from hsp90, and nuclear translocation. It is not clear whether these mechanisms are dependent on the same ligand-induced conformational change needed for transactivation. However, a transactivation-defective mutant represses the AP-1 dependent promoter suggesting that the transactivation function of the receptor is not required for the repression of AP-1 activity (Yang-Yen, et al., Cell 62:1205-1215 (1990)). Furthermore, similar studies also suggest that the transactivation function of the receptor is not required for the repression of NF_{K}-B activity.

In attempts to decipher the transrepression mechanism, studies have reviewed the role of the bound ligand in GR-mediated repression of AP-1-responsive genes containing a tetradecanoyl phorbol acetate ("TPA") response element. Repression of these genes has been proposed to be the result of the direct interaction of the GR with c-Jun (Diamond, et al., Science 249:1266-1272 (1990); Lucibello, et al., EMBO J. 9:2827-2834 (1990); Schule, et al., Cell 62:1217-1226 (1990); Touray, et al., Oncogene 6:1227-1234 (1991); Yang-Yen, et al., Cell 62:1205-1215 (1990)) or c-Fos (Kerppola, et al., Mol. Cell. Biol. 13:3782-3791 (1992)) which are components of the AP-1 transcription complex. The GR DNA-binding domain is necessary for this interaction, since most mutations in this domain result in the loss of repressor activity *in vivo* (Diamond et al., Science 249:1266-1272 (1990); Jonat et al., Cell 62:1189-1204 (1990); Lucibello et al., EMBO J. 9:2827-2834 (1990); Schule et al., Cell 62:1217-1226 (1990); Yang-Yen et al., Cell 62:1205-1215 (1990)).

The DNA-binding domain is also necessary for inhibition of *in vitro* transcription from the collagenase promoter and inhibition of Jun-Fos heterodimer binding to the collagenase TPA response element (Mordacq et al., Genes Dev. 3:760-769 (1989)). However, deletion or truncation of the ligand-binding domain also results in a significant loss of repressor activity (Jonat et al., Cell 62:1189-1204 (1990); Schule et al., Cell 62:1217-1226 (1990); Yang-Yen et al., Cell 62:1205-1215 (1990)), suggesting that the ligand-binding domain may contribute to, or modulate, the inhibition of AP-1 activity.

Further studies examining the role of the ligand in GR-mediated transrepression of the collagenase promoter found efficient receptor-mediated transrepression with ligand-free mutant GR in which the first cysteine residue of the proximal zinc finger was replaced with tyrosine (Liu et al., Mol. Cell. Bio. 15:1005-1013 (1995)). Such studies suggest that neither retention of the ligand nor direct binding of the receptor to DNA is required, i.e., that transrepression of AP-1 activity by GR is ligand independent.

The expression of most mammalian genes is intricately regulated *in vivo* in response to a wide range of stimuli, including physical (pressure, temperature, light), electrical (e.g. motor and sensory neuron signal transmission) as well as biochemical (ions, nucleotides, neurotransmitters, steroids and peptides) in nature. While the mechanism of transcriptional regulation of gene expression has been extensively studied (McKnight, Genes Dev. 10:367-381 (1996)), progress on achieving target gene regulation in mammalian cells, without interfering with endogenous gene expression, has been limited. Currently, most strategies for target gene activation or repression are performed in a constitutive manner. Such uncontrolled regulation of gene expression is not ideal physiologically, and can even be deleterious to cell growth and differentiation. In contrast, use of the yeast GAL4 DNA binding domain in this invention does not interfere with endogenous genes since that chimeric regulator will only recognize target gene constructs containing the GAL4 binding sequence.

Several inducible systems have been employed for controlling target gene expression. These inducible agents include heavy metal ions (Mayo et al., Cell 29:99-108 (1982)), heat shock (Nover et al. CRC Press 167-220 (1991)), isopropyl β-D-thiogalactoside (β-gal) (Baim et al. Proc. Natl. Acad. Sci. 88:5072-5076 (1981)), and steroid hormones such as estrogen (Braselmann et al. Proc. Natl. Acad. Sci. 90:1657-1661 (1993)) and glucocorticoids (Lee et al. Nature 294:228-232 (1981)). However, many of these inducers are either toxic to mammalian cells or interfere with endogenous gene expression (Figge et al. Cell 52:713-722 (1988)).

Utilizing a bacterial tetracycline-responsive operon element, Gossen et al. developed a model for controlling gene expression with a tetracycline-controlled transactivator (tTA and rtTA) (Gossen et al. Proc. Natl. Acad. Sci. 89:5547-5551 (1992); Gossen et al. Science 268:1766-1769 (1995)). No *et al.* recently reported a three-component system consisting of a chimeric GAL4-VP16-ecdysone receptor, its partner retinoid X receptor (RXR), and a target gene. They demonstrated its application in activating reporter gene expression in an ecdysone-dependent manner (No et al. Proc. Natl. Acad. Sci. 93:3346-3351 (1996). The invention described herein has advantages over the No and Gossen models, as the chimeric regulator recognizes only the target gene constructs and not endogenous genes, and the system is only activated in the presence of an exogenous compound, but not in the presence of any endogenous molecules.

WO 93/23431 (Baylor College of Medicine) describes mutant proteins of steroid hormone receptors, including progesterone receptor. The modified receptors can distinguish between an antagonist and an agonist. Such modified receptors are proposed to be used as a molecular switch for regulating expression n gene therapy.

Lipkin et al. (1996) J. Virol. 70: 7182-7189 describes fusion proteins between a retinoic acid receptor (RAR) or an activated retinoid X (RXR) receptor and a VP16 transactivation domain.

Wang Y. et al (1994) P.N.A.S. 91: 8180-8184 describes a chimeric protein with a VP16-derived transactivating domain, a Gal 4 DNA-binding domain, and a mutant progesterone receptor-ligand binding domain responsive to RU486. The transregulatory domain is of the N-terminus.

### Summary of the Invention

Accordingly, the present invention provides a modified steroid hormone receptor protein, wherein said receptor protein responds to a conventional antagonist of a wild-type progesterone receptor protein counterpart with an agonistic response, and wherein said modified receptor protein comprises a DNA binding domain, a transregulatory domain, and a mutated progesterone receptor ligand binding region, characterized in that the transregulatory domain is heterologous to the ligand binding domain and is located in a C-terminal position in said modified steroid hormone receptor protein.

The progesterone receptor ligand binding region may be mutated by an alteration of a primary sequence in about 13 to 42 carboxyl terminal amino acids of a wild-type progesterone receptor ligand binding domain. More preferably the progesterone receptor ligand binding region may consist essentially of amino acids selected from the group consisting of 640 through 914, 640 through 917 or 640 through 920 of a progesterone receptor ligand binding domain.

The transregulatory domain is preferably capable of transactivating or transrepressing gene expression.

In certain embodiments the DNA binding domain may be replaced with a DNA binding domain that is not associated normally with the progesterone ligand binding domain. Preferably the DNA binding domain is a GAL-4 DNA binding domain.

The transregulatory domain may be a transactivation domain that is different from a transactivation domain that is naturally associated with the mutated steroid hormone receptor protein ligand binding domain.

A preferred transregulatory domain is a transrepression domain.

Other preferred said transregulatory domains comprise a Krüppel-associated box-A (KRAB) transrepressing domain, e.g. wherein the KRAB transrepression domain is a Kid-1 or a Kox1 KRAB transrepression domain.

The mutated progesterone receptor ligand binding domain may bind a synthetic antagonist selected from the group consisting of: RU486, Org31806 and Org 31376.

In preferred embodiments, the said mutated progesterone receptor ligand binding region is capable of responding to RU486 at a concentration as low as 0.01 nM.

The modified steroid hormone receptor protein preferably comprises in order from N-terminus to C-terminus, the DNA binding domain, the mutated progesterone receptor ligand binding region, and the transregulatory domain.

The invention provides a nucleic acid sequence encoding a modified steroid hormone receptor protein as described herein. The nucleic acid sequence may further comprise a tissue specific promoter.

The invention includes a vector containing a nucleic acid sequence as described herein.

The invention also includes a cell transfected or transformed with a vector as described herein.

The invention also provides a method of making a transformed cell *in situ* comprising the step of contacting said cell with a vector as described herein for sufficient time to transform said cell, wherein said transformed cell expresses a modified steroid hormone receptor protein encoded by said vector.

In another aspect, the invention provides the use of a vector as described herein in the manufacture of a medicament for delivering the nucleic acid sequence encoding the modified steroid hormone receptor protein to a mammal in *vivo.*

In a further aspect, the invention provides the use of a vector as described herein in the manufacture of a medicament for delivering the nucleic acid sequence encoding the modified steroid hormone receptor protein to a mammal *in vivo* for regulating expression of neurite growth factor.

The invention also includes a transgenic non-human animal whose cells contain the nucleic acid or the vector as described herein.

Construction of novel modified steroid hormone receptors which regulate the expression of nucleic acid sequences is described herein, and surprisingly these modifications allow control of the transactivation and transrepressing functions of the modified steroid hormone receptor. Such modifications unexpectedly allow the receptors to bind various ligands whose structures differ dramatically from the naturally-occurring ligands (for example, non-natural ligands, anti-hormones and non-native ligands) and thereby provide a substantial improvement over prior attempts to control or regulate target gene expression.

These modifications are generated in the ligand binding domain of the GR and eliminate the ability of the GR to bind its natural ligand. These modified steroid receptors exhibit normal transactivation and transrepression activity; however, stimulation of such activity occurs via activation by a non-natural and exogenously or endogenously applied ligand. Modifications are also generated in the ligand binding domain of the PR and eliminate the ability of PR to bind its natural ligand. Replacement of the GR binding domain with the modified PR binding domain allows the stimulation of GR responsive gene expression via non-natural ligands.

Other modifications to the GR ligand binding domain in conjunction with modifications to the DNA binding domain of GR eliminate the ability of steroid hormones to initiate transactivation by its natural ligand. Instead, such modifications allow the modified receptor to bind non-natural ligands and stimulate the transrepression of gene expression but not transactivation. Likewise, using the same ligand binding domain modification in conjunction with modifications to the transregulatory domain allows the modified receptor to bind non-natural ligands and stimulate transactivation but not transrepression of gene expression.

Other modifications remove the ligand binding domain completely to create a constitutively active steroid receptor. Such modifications cause continual transactivation and transrepression effects on the regulation of gene transcription. In addition, modifications that selectively eliminate either transactivation or transrepression functions are incorporated into the constitutively active steroid receptor thereby constitutively transrepressing or transactivating gene expression. Furthermore, other modifications use a ligand binding domain which recognizes its natural ligand or if modified recognizes a non-natural ligand, but is fused with a DNA binding domain and transregulatory domains not associated normally with the ligand binding domain. Such a construct is capable of regulating the expression of a gene not normally associated with the ligand binding domain in a wild type receptor protein.

These modified receptors can be expressed by specially designing DNA expression vectors to control the level of expression of recombinant gene products. The steroid receptor family of gene regulatory proteins is an ideal set of such molecules. These proteins are ligand activated transcription factors whose ligands can range from steroids to retinoids, fatty acids, vitamins, thyroid hormones and other presently unidentified small molecules. These compounds bind to receptors and either activate or repress transcription.

These receptors are modified to allow them to bind various ligands whose structure is either naturally occurring or differs from naturally occurring ligands. By screening receptor mutants, receptors can be selected that respond to ligands which do not activate the host cell endogenous receptor. Thus, regulation of a desired transgene can be achieved using a ligand which binds to and regulates a customized receptor. This occurs only with cells that have incorporated and express the modified receptor.

Taking advantage of the abilities of the modified steroid hormone receptor to effect regulation of gene expression, these gene constructs can be used as therapeutic gene medicines, for gene replacement, and in gene therapy. These modified receptors are useful in gene therapy where the level of expression of a gene, whether transactivation or repression, is required to be controlled. The number of diseases associated with inappropriate production or responses to hormonal stimuli highlights the medical and biological importance of these constructs.

The properties of the modified steroid hormone receptors allow most or all of the deleterious effects of steroids to be avoided while generally maintaining their therapeutic benefits. In particular, administration of steroids typically causes toxicity problems. The deleterious effects of steroids can be attributed to the *in vivo* transactivation or transrepression of certain genes. These toxic effects may well be the result of both transactivation and transrepression, or be primarily attributable to one of them. The present invention includes the use of modified GR molecules as gene medicines for the replacement of steroid therapy. These synthetic receptors retain functions similar to those of the endogenous receptors, but by responding to alternative ligands, eliminate some of the toxic side effects attributable to currently used steroid therapy.

This ability of the GR constructs to avoid steroid toxicity but still exhibit therapeutic effects allows the constructs to be used for treating numerous diseases, including arthritis, asthma, senile dementia or Parkinson's disease. Furthermore, the constructs can be used for preventing or treating diseases in which inappropriate production or responses to hormonal stimuli exists, *e.g.,* hormone-dependent cancers of the breast, ovary, endometrium, prostate, and post-menopausal osteoporosis. The constructs also can be used in conjunction with co-transfected expression vectors so as to operate as a gene switch. For detailed description of gene switch, see, U.S. Patent No. 5,364,792, Vegeto et al.

The constructs described herein can be used for gene replacement therapy in humans and for creating non-human transgenic animal models used for studying human diseases. The transgenic models can be used as well for assessing and exploring novel therapeutic avenues to treat effects of chemical and physical carcinogens and tumor promoters. The above constructs can also be used for distinguishing steroid hormone receptor antagonists and steroid hormone receptor agonists. Such recognition of antagonist or agonist activity can be performed using cells transformed with the above constructs. Thus, in view of the above, various aspects of the invention will now be described.

Described herein is a modified glucocorticoid receptor fusion protein. The fusion protein receptor contains a GR with its ligand-binding domain replaced with a mutated PR ligand-binding domain. This fusion protein is capable of being activated by the binding of a non-natural ligand, but not by natural or synthetic glucocorticoid or other natural or synthetic steroids. The fusion protein includes a glucocorticoid receptor region which comprises a DNA binding domain and one or more transregulatory domains. The transregulatory domains are capable of transactivating or transrepressing glucocorticoid responsive gene expression.

In addition to the glucocorticoid receptor region, the fusion protein also includes a mutated progesterone ligand binding region which is capable of binding a non-natural ligand. The mutated ligand binding region is preferably mutated by deletion of about 16 to 42 carboxyl terminal amino acids of a progesterone receptor ligand binding domain. The mutated progesterone receptor ligand binding region preferably comprises, consists essentially of, or consists of about amino acids 640 through 891 of a progesterone receptor. Other preferred embodiments comprise, consist essentially of, or consist of amino acids 640-917, amino acids 640-920 or amino acids 640-914. One skilled in the art will recognize that various mutations can be created to achieve the desired function.

The term "fusion protein" as used herein refers to a protein which is composed of two or more proteins (or fragments thereof) where each protein occurs separately in nature. The combination can be between complete amino acid sequences of the protein as found in nature, or fragments thereof. In the case of a glucocorticoid-progesterone fusion protein receptor, the fusion protein is preferably composed of portions of the glucocorticoid receptor and the progesterone receptor. This combination can include the complete amino acid sequence of each protein or fragments thereof. For example, the glucocorticoid-progesterone fusion protein may include the ligand binding domain of progesterone and the DNA binding domain and transregulatory domains of the glucocorticoid receptor. This is only an example and not meant to be limiting.

The term "non-natural ligand" as used herein refers to compounds which can normally bind to the ligand binding domain of a receptor, but are not the endogenous ligand. "Endogenous" as used herein refers to a compound originating internally within mammalian cells. The receptor is not exposed to the ligand unless it is exogenously supplied. "Exogenous" as used herein refers to a compound originating from external sources and not normally present within mammalian cells. This also includes ligands or compounds which are not normally found in animals or humans. Non-natural also includes ligands which are not naturally found in the specific organism (man or animal) in which gene therapy is contemplated. These ligands activate receptors by binding to the modified ligand binding domain. Activation can occur through a specific ligand-receptor interaction whether it is through direct binding or through association in some form with the receptor. "Natural ligand" as used here refers to compounds which normally bind to the ligand binding domain of a receptor and are endogenous. The receptor in this case is exposed to the ligand endogenously. Natural ligands include steroids, retinoids, fatty acids, vitamins, thyroid hormones, as well as synthetic variations of the above. This is meant to be only an example and non-limiting.

The term "ligand" as referred to herein means any compound which activates the receptor, usually by interaction with the ligand binding domain of the receptor. Ligand includes a molecule or an assemblage of molecules capable of specifically binding to a modified receptor. The term "specifically binding" means that a labeled ligand bound to the receptor can be completely displaced from the receptor by the addition of unlabeled ligand, as is known in the art.

Examples of non-natural ligands and non-native ligands may be found in PCT Publication WO 96/40911.

The term "binding" or "bound" as used herein refers to the association, attaching, connecting, or linking through covalent or non-covalent means, of a ligand, whether non-natural or natural, with a corresponding receptor. The ligand and receptor interact at complementary and specific within sites on a given structure. Binding includes, but is not limited to, components which associate by electrostatic binding, hydrophobic binding, hydrogen binding, intercalation or forming helical , structures with specific sites on nucleic acid molecules.

The term "glucocorticoid receptor" refers to a steroid hormone receptor which responds to a glucocorticoid ligand. The glucocorticoid receptor is part of the steroid hormone receptor superfamily which are known steroid receptors whose primary sequence suggests that they are related to each other. Representative examples of such receptors include the estrogen, progesterone, Vitamin D, chicken ovalbumin upstream promoter transfactor, ecdysone, Nurr-1 and orphan receptors, glucocorticoid-α, glucocorticoid-β, mineralocorticoid, androgen, thyroid hormone, retinoic acid, and retinoid X. These receptors are composed of DNA binding domains, ligand binding domains, as well as transregulatory domains.

The glucocorticoid receptor is a ligand-dependent transcription factor capable of both positive and negative regulation of gene expression. Interaction of the receptor with a ligand induces a cascade of molecular events that ultimately lead to the specific association of the activated receptor with regulatory elements of target genes. In an inactive form such receptors form a large complex comprising the receptor, heat shock proteins and other proteins.

The term "glucocorticoid receptor region" refers to a fragment or part of the complete glucocorticoid receptor as defined above. A glucocorticoid receptor region may retain complete or partial activity of the natural receptor protein. For example, a glucocorticoid receptor region might contain only the DNA binding domain and the transregulatory domains and not the ligand binding domain, or vice versa. This is only an example and not meant to be limiting.

The term "ligand binding domain" or "ligand binding region" as used herein refers to that portion of a steroid hormone receptor protein which binds the appropriate hormone or ligand and induces a cascade of molecular events that ultimately leads to the specific association of the activated receptor with regulatory elements of target genes. This includes, but is not limited to, the positive or negative effects on regulation of gene transcription. Binding of ligand to the ligand binding domain induces a conformation change in the receptor structure. The conformational change includes the dissociation of heat shock proteins and the release of a monomeric receptor from the receptor complex, as well as a different tertiary or 3-dimensional structure. The conformational change that occurs is specific for the steroid receptor and ligand that binds to the ligand binding domain.

For example, for glucocorticoid receptors, the conformation change that occurs when glucocorticoid hormone binds allows homodimerization, *i.e*., dimerization between two identical GR molecules. However, heterodimerization can occur with other steroid receptors, *i.e*., dimerization with two molecules such as GR and ER. Such dimerization allows the receptor to bind with DNA or induce the regulatory effect by binding other transcription factors.

The term "DNA binding domain" as used herein refers to that part of the steroid hormone receptor protein which binds specific DNA sequence in the regulatory regions of target genes. This domain is capable of binding short nucleotide stretches arranged as palindromic, inverted or repeated repeats. Such binding, will activate gene expression depending on the specific ligand and the conformational changes due to such ligand binding. For repression, DNA binding is not needed.

The term "transregulatory domain" as used herein refers to those portions of the steroid hormone receptor protein which are capable of transactivating or transrepressing gene expression. This would include different regions of the receptor responsible for either repression or activation, or the regions of the receptor responsible for both repression and activation. Such regions are spatially distinct. The above is only an example and meant to be non-limiting. For transrepression, this domain under one mechanism is involved with dimerization which in turn causes a protein/protein interaction to prevent or repress gene expression. Such regulation occurs when the receptor is activated by the ligand binding to the ligand binding domain. The conformational change of the receptor is capable of forming a dimer with a discrete portion of the transregulatory domain to repress gene expression. In addition, repression can occur through a monomeric form of the receptor, however, DNA binding is not necessary (see below).

The terms "transactivation," "transactivate," or "transactivating" refer to a positive effect on the regulation of gene transcription due to the interaction of a hormone or ligand with a receptor causing the cascade of molecular events that ultimately lead to the specific association of the activated receptor with the regulatory elements of the target genes. Transactivation can occur from the interaction of non-natural as well as natural ligands. Agonist compounds which interact with steroid hormone receptors to promote transcriptional response can cause transactivation. Such positive effects on transcription include the binding of an activated receptor to specific recognition sequences in the promoter of target genes to activate transcription. The activated receptors are capable of interacting specifically with DNA. The hormone- or ligand-activated receptors associate with specific DNA sequences, or hormone response elements, in the regulatory regions of target genes. Transactivation alters the rate of transcription or induces the transcription of a particular gene(s). It refers to an increase in the rate and/or amount of transcription taking place.

The terms "transrepress," "transrepression" or "transrepressing" as used herein refer to the negative effects on regulation of gene transcription due to the interaction of a hormone or ligand with a receptor inducing a cascade of molecular events that ultimately lead to the specific association of the activated receptor with other transcription factors such as NF_{K}-B or AP-1. Transrepression can occur from the interaction of non-natural as well as natural ligands. Antagonist and agonist compounds which interact with steroid hormone receptor can cause transrepression. Once the ligand binds to the receptor, a conformational change occurs. Transrepression can occur via two different mechanisms, *i.e*., through the dimeric and monomeric form of the receptor. Use of the monomeric form of the receptor for transrepression depends on the presence of the DNA binding domain but not on the ability of the receptor to bind DNA. Use of the dimeric form of the receptor for transrepression depends on the receptor binding response elements overlapping cis-element(s). Transrepression alters the rate of transcription or inhibits the transcription of a particular gene. Transrepression decreases the rate and/or the amount of transcription taking place.

The term "progesterone receptor" as used herein also refers to a steroid hormone receptor which responds to or is activated by the hormone progesterone. Progesterone is part of the steroid hormone receptor superfamily as described above. The progesterone receptor can exist as two distinct but related forms that are derived from the same gene. The process for generation of the products may be alternate initiation of transcription, splicing differences, or transcription termination. These receptors are composed of DNA binding, ligand binding, as well as transregulatory domains. The progesterone receptor is also a ligand-dependent transcription factor capable of regulating gene expression. Interaction of the progesterone receptor with a ligand induces a cascade of molecular events that ultimately lead to the specific association of the activated receptor with regulatory elements of target genes.

The term "modified," "modification," "mutant" or "mutated" refers to an alteration of the receptor from its naturally occurring wild-type form. This includes alteration of the primary sequence of a receptor such that it differs from the wild-type or naturally-occurring sequence. The mutant steroid hormone receptor protein as used in the present invention can be a mutant of any member of the steroid hormone receptor superfamily. For example, a steroid receptor can be mutated by deletion of amino acids on the carboxyl terminal end of the protein. Generally, a deletion of from about 1 to about 120 amino acids from the carboxyl terminal end of the protein provides a mutant steroid hormone receptor useful in the present invention. A person having ordinary skill in this art will recognize, however, that a shorter deletion of carboxyl terminal amino acids will be necessary to create useful mutants of certain steroid hormone receptor proteins. Other mutations or deletions can be made in other domains of the steroid receptor of interest, such as the DNA binding domain or the transregulatory domain.

For example, a mutant of. the progesterone receptor protein will contain a carboxyl terminal amino acid deletion of approximately 1 to 60 amino acids. In an embodiment of the present invention, 42 carboxyl terminal amino acids are deleted from the progesterone receptor protein. Likewise, a mutation of one or more amino acids in the DNA binding domain or the transregulatory domains can change the regulation of gene expression.

One skilled in the art will recognize that a combination of mutations and/or deletions are possible to gain the desired response. This would include double point mutations to the same or different domains. In addition, mutation also includes "null mutations" which are genetic lesions to a gene locus that totally inactivate the gene product.

Examples of mutations are described in PCT Publication WO 96/40911.

The term mutation also includes any other derivatives. The term "derivative" as used herein refers to a peptide or compound produced or modified from another peptide or compound of a similar structure. Such a derivative may be a "chemical derivative," "fragment," "variant," "chimera," or "hybrid" of the complex. A derivative retains at least a portion of the function of the protein (for example reactivity with an antibody specific for the complex, enzymatic activity or binding activity mediated through noncatalytic domains) which permits its utility in accordance with the present invention.

A derivative may be a complex comprising at least one "variant" polypeptide which either lacks one or more amino acids or contain additional or substituted amino acids relative to the native polypeptide. The variant may be derived from a naturally occurring complex component by appropriately modifying the protein DNA coding sequence to add, remove, and/or to modify codons for one or more amino acids at one or more sites of the C-terminus, N-terminus, and/or within the native sequence. It is understood that such variants having added, substituted and/or additional amino acids retain one or more characterizing portions of the native complex. A functional derivative of complexes comprising proteins with deleted, inserted and/or substituted amino acid residues may be prepared using standard techniques well-known to those of ordinary skill in the art.

A "chemical derivative" of the complex contains additional chemical moieties not normally a part of the protein. Such moieties may improve the molecule's solubility, absorption, biological half life, and the like.

The term "modified" or "modification" as used herein refers to a change in the composition or structure of the compound or molecule. However, the activity of the derivative, modified compound, or molecule is retained, enhanced, or increased relative to the activity of the parent compound or molecule. This would include the change of one amino acid in the sequence of the peptide or the introduction of one or more non-naturally occurring amino acids or other compounds. This includes a change in a chemical body, a change in a hydrogen placement, or any type of chemical variation. In addition, "analog" as used herein refers to a compound that resembles another structure. Analog is not necessarily an isomer. The above are only examples and are not limiting.

The term "nucleic acid sequence," "gene," "nucleic acid" or "nucleic acid cassette" as used herein refers to the genetic material of interest which can express a protein, or a peptide, or RNA after it is incorporated transiently, permanently, or episomally into a cell. The nucleic acid can be positionally and sequentially oriented in a vector with other necessary elements such that the nucleic acid can be transcribed and, when necessary, translated into protein in the cells.

The term "genetic material" as used herein refers to contiguous fragments of DNA or RNA. The genetic material which is introduced into targeted cells can be any DNA or RNA. For example, the nucleic acid can be: (1) normally found in the targeted cells, (2) normally found in targeted cells but not expressed at physiologically appropriate levels in targeted cells, (3) normally found in targeted cells but not expressed at optimal levels in certain pathological conditions, (4) not normally found in the targeted cells, (5) novel fragments of genes normally expressed or not expressed in targeted cells, (6) synthetic modifications of genes expressed or not expressed within targeted cells, (7) any other DNA which may be modified for expression in targeted cells and (8) any combination of the above.

The term "gene expression" or "nucleic acid expression" as used herein refers to the gene product of the genetic material from the transcription and translation process. Expression includes the polypeptide chain translated from an mRNA molecule which is transcribed from a gene. If the RNA transcript is not translated, e.g., rRNA, tRNA, the RNA molecule represents the gene product.

A glucocorticoid-progesterone fusion protein receptor can be expressed as a cell surface, cytoplasmic or nuclear protein. By "cell surface protein" it is meant that a protein is wholly or partially spanning the cell membrane when expressed and which also is exposed on the surface of the cell. By cytoplasmic protein it is meant that a protein is contained completely within the cytoplasm, and does not span the nucleus or cell surfaces. As for "nuclear protein" it is meant that the protein is wholly or partially spanning the nuclear membrane when expressed and is exposed to the cell cytoplasm, or may be contained completely within the cell nucleus, not attached to the nuclear membrane and not exposed to cell cytoplasm.

A modified receptor protein as described herein may include a mutated progesterone ligand binding region of amino acids 640 through 914 of a progesterone receptor ligand binding domain. Another modified receptor protein as described herein may contain a transregulatory domain located in the N-terminal region of the mutated progesterone ligand binding domain. In yet another modified receptor protein as described herein there may be a transregulatory domain located in the C-terminal region of the mutated progesterone ligand binding domain. Thus, the transregulatory domain can be located either in the C-terminal or N-terminal direction of the ligand binding domain.

A modified receptor protein as described herein may include a GAL4 DNA. binding domain: In another modified receptor protein as described herein may be a Krüppel-associated box-A (KRAB) transrepressing domain. The terms "GAL4 DNA binding domain" and "KRAB transrepressing domain" are used as conventionally understood in the art and encompass functional equivalents of such sequences that retain the ability to bind DNA or retain the transrepressing activity.

In another modified receptor as described herein there may be a mutated progesterone receptor ligand binding region capable of binding RU486 at a concentration as low as 0.01 nM. In still another embodiment, a modified steroid hormone receptor protein responds to a conventional antagonist of the wild-type steroid hormone receptor protein counterpart with an agonistic response. Those skilled in the art will understand that "binding" can be measured by several conventional methods in the art, such as binding constants and that a protein "response" can also be measured using conventional techniques in the art, such as measurement of induced transcription levels.

As used herein the term "constitutively" refers to the ability to continually activate or repress gene expression without the need for a ligand.

Examples of mutated transregulatory domains are described in PCT Publication WO 96/40911.

Examples of mutated DNA binding domain are described in PCT publication WO 96/40911.

The nucleic acid is the genetic material which can express a protein, or a peptide, or RNA after it is incorporated transiently, permanently or episomally into a cell.

Vectors are capable of expressing the nucleic acid transiently, permanently or episomally into a cell or tissue.

The term "vector" as used herein refers to a construction comprised of genetic material designed to direct transformation of a targeted cell. A vector contains multiple genetic elements positionally and sequentially oriented with other necessary elements such that the nucleic acid in a nucleic acid cassette can be transcribed and when necessary translated in the transfected cells. The term vector as used herein can refer to nucleic acid, e.g., DNA derived from a plasmid, cosmid, phagemid or bacteriophage, into which one or more fragments of nucleic acid may be inserted or cloned which encode for particular proteins. The term "plasmid" as used herein refers to a construction comprised of extrachromosomal genetic material, usually of a circular duplex of DNA which can replicate independently of chromosomal DNA. The plasmid does not necessarily replicate.

The vector can contain one or more unique restriction sites, and may be capable of autonomous replication in a defined host or organism such that the cloned sequence is reproduced. The vector molecule can confer some well-defined phenotype on the host organism which is either selectable or readily detected. The vector may have a linear or circular configuration. The components of a vector can contain but is not limited to a DNA molecule incorporating: (1) DNA; (2) a sequence encoding a therapeutic or desired product; and (3) regulatory elements for transcription, translation, RNA processing, RNA stability, and replication.

The purpose of the vector is to provide expression of a nucleic acid sequence in cells or tissue. Expression includes the efficient transcription of an inserted gene or nucleic acid sequence. Expression products may be proteins, polypeptides, or RNA. The nucleic acid sequence can be contained in a nucleic acid cassette. Expression of the nucleic acid can be continuous, constitutive, or regulated. The vector can also be used as a prokaryotic element for replication of plasmid in bacteria and selection for maintenance of plasmid in bacteria.

In the present invention the preferred vector comprises the following elements linked sequentially at an appropriate distance to allow functional expression: a promoter, a 5' mRNA leader sequence, a translation initiation site, a nucleic acid cassette containing the sequence to be expressed, a 3' mRNA untranslated region, and a polyadenylation signal sequence. As used herein the term "expression vector" refers to a DNA vector that contains all of the information necessary to produce a recombinant protein in a heterologous cell.

In addition, the term "vector" as used herein can also include viral vectors. A "viral vector" in this sense is one that is physically incorporated in a viral particle by the inclusion of a portion of a viral genome within the vector, *e.g*., a packaging signal, and is not merely DNA or a located gene taken from a portion of a viral nucleic acid. Thus, while a portion of a viral genome can be present in a vector of the present invention, that portion does not cause incorporation of the vector into a viral particle and thus is unable to produce an infective viral particle.

A vector as used herein can also include DNA sequence elements which enable extra-chromosomal (episomal) replication of the DNA. Vectors capable of episomal replication are maintained as extra-chromosomal molecules and can replicate. These vectors are not eliminated by simple degradation but continue to be copied. These elements may be derived from a viral or mammalian genome. These provide prolonged or "persistent" expression as described below.

The term "persistent expression" as used herein refers to introduction of genes into the cell together with genetic elements which enable episomal (*i.e*., extra-chromosomal) replication. This can lead to apparently stable transformation of the cell without the integration of the novel genetic material into the chromosome of the host cell.

"Stable expression" as used herein relates to the integration of genetic material into chromosomes of the targeted cell where it becomes a permanent component of the genetic material in that cell. Gene expression after stable integration can permanently alter the characteristics of the cell and its progeny arising by replication leading to stable transformation.

A related aspect of the present invention features a transfected cell containing a vector which contains nucleic acid sequence, for a modified receptor as described herein. As used herein the term "transfected" or "transfection" refers to the incorporation of foreign DNA into any cells by exposing them to such DNA. This would include the introduction of DNA by various delivery methods, *e.g*., via vectors or plasmids.

Methods of transfection may include microinjection, CaPO₄ precipitation, liposome fusion (*e.g*., lipofection), electroporation or use of a gene gun. Those are only examples and are meant not to be limiting. The term "transfection" as used herein refers to the process of introducing DNA (*e*.*g*., DNA expression vector) into a cell. Following entry into the cell, the transfected DNA may: (1) recombine with the genome of the host; (2) replicate independently as an episome; or (3) be maintained as an episome without replication prior to elimination. Cells may be naturally able to uptake DNA. Particular cells which are not naturally able to take up DNA require various treatments, as described above, in order to induce the transfer of DNA across the cell membrane.

A related aspect of the present invention features a transformed cell with a vector containing a nucleic acid sequence for a modified receptor as discussed above. As used here in the term "transformed" or "transformation" refers to transient, stable or permanent changes in the characteristics (expressed phenotype) of a cell by the mechanism of gene transfer. Genetic material is introduced into a cell in a form where it expresses a specific gene product or alters the expression or effects of endogenous gene products.

The term "stable" as used herein refers to the introduction of gene(s) into the chromosome of the targeted cell where it integrates and becomes a permanent component of the genetic material in that cell. Gene expression after stable transformation can permanently alter the characteristics of the cell leading to stable transformation. An episomal transformation is a variant of stable transformation in which the introduced gene is not incorporated in the host cell chromosomes but rather is replicated as an extrachromosomal element. This can lead to apparently stable transformation of the characteristics of a cell. "Transiently" as used herein refers to the introduction of a gene into a cell to express the nucleic acid, e.g., the cell express specific proteins, peptides or RNA, etc. The introduced gene is not integrated into the host cell genome and is accordingly eliminated from the cell over a period of time. Transient expression relates to the expression of a gene product during a period of transient transfection. Transient expression also refers to transfected cells with a limited life span.

Transformation can be performed by *in vivo* techniques or *ex vivo* techniques as deserved in PCT Publication WO 96/40911. Transformation can be tissue specific to regulate expression of the nucleic acid predominantly in the tissue or cell of choice.

Transformation of the cell may be associated with production of a variety of gene products including protein and RNA. Such products are described in PCT Publication WO 96/40911. The product expressed by the transformed cell depends on the nucleic acid of the nucleic acid cassette. In the present invention the nucleic acid to be expressed is a fusion protein as referenced above, or variations thereof or any of the other receptor proteins disclosed herein.

In one embodiment the transformed cell is a muscle cell. The term "muscle" refers to myogenic cells including myoblasts, skeletal, heart and smooth muscle cells. The muscle cells or tissue can be *in vivo*, in vitro or tissue culture and capable of differentiating into muscle tissue. In another embodiment, the transformed cell is a lung cell. The term "lung cell" as used herein refers to cells associated with the pulmonary system. The lung cell can also be in vivo, in vitro or tissue culture.

In still another embodiment, the transformed cell is a cell associated with the joints. The term "cells associated with the joints" refers to all of the cellular and non-cellular materials which comprise the joint (e.g., knee or elbow) and are involved in the normal function of the joint or are present within the joint due to pathological conditions. These include material associated with: the joint capsule such as synovial membranes, synovial fluid, synovial cells (including type A cells and type B synovial cells); the cartilaginous components of the joint such as chondrocyte, extracellular matrix of cartilage; the bony structures such as bone, periosteum of bone, periosteal cells, osteoblast, osteoclast; the immunological components such as inflammatory cells, lymphocytes, mast cells, monocytes, eosinophil; other cells like fibroblasts; and combinations of the above. Once transformed these cells express the fusion protein. One skilled in the art will quickly realize that any cell is capable of undergoing transformation and within the scope of this invention.

An aspect of the present invention features methods for transforming a cell with a vector-containing nucleic acid encoding for a modified receptor. This method includes the steps of transforming a cell in situ by contacting the cell with the vector for a sufficient amount of time to transform the cell. As discussed above, transformation can be *in vivo* or *ex vivo.* Once transformed the cell expresses the mutated glucocorticoid receptor. This method includes methods of introducing and methods of incorporating the vector. "Incorporating" and "introducing" as used herein refer to uptake or transfer of the vector into a cell such that the vector can express the therapeutic gene product within a cell as discussed with transformation above.

The modified receptors described herein may be used in a method comprising the steps of transforming a cell with a vector containing a nucleic acid encoding for the modified receptor of interest. The transformed cells are able to express the mutated receptor. The receptor is capable of regulating by a non-natural ligand the expression of glucocorticoid responsive genes, whether such regulation is transactivation or transrepression. The term "glucocorticoid responsive genes" as used herein refers to genes whose expression is regulated by the activation of the glucocorticoid receptor. Such regulation includes both positive and negative regulation of gene expression. This also includes GRE (glucocorticoid response element) controlled genes.

This method of use includes methods of gene replacement using the fusion protein, methods of gene therapy using the fusion protein and methods of administering the fusion protein in which the same steps are used. "Gene replacement" as used herein means supplying a nucleic acid sequence which is capable of being expressed in vivo in an animal and thereby providing or augmenting the function of an endogenous gene which is missing or defective in the animal.

Described herein are methods for administration as discussed above. Such methods include methods for administering a supply of polypeptide, protein or RNA to a human, animal or to tissue culture or cells. These methods of use of the above-referenced vectors comprises the steps of administering an effective amount of the vectors to a human, animal or tissue culture. The term "administering" or "administration" as used herein refers to the route of introduction of a vector or carrier of DNA into the body. The vectors of the above methods and the methods discussed below may be administered by various routes. Administration may be intravenous, intratissue injection, topical, oral, or by gene gun or hypospray instrumentation. Administration can be directly to a target tissue, e.g. direct injection into synovial cavity or cells, or through systemic delivery. These are only examples and are nonlimiting.

Administration will include a variety of methods, as described in PCT Publication WO 96/40911. See, also, WO 93/18759. The preferred embodiment is by direct injection. Routes of administration include intramuscular, aerosol, oral, topical, systemic, ocular, intraperitoneal, intrathecal and/or fluid spaces.

The term "effective amount" as used herein refers to sufficient vector administered to humans, animals or into tissue culture cells to produce the adequate levels of polypeptide, protein, or RNA. One skilled in the art recognizes that the adequate level of protein polypeptide or RNA will depend on the intended use of the particular vector. These levels will be different depending on the type of administration, treatment or vaccination as well as intended use.

A method of using the mutated receptors described herein uses RU486 as the non-natural ligand to regulate gene expression. This ligand is capable of binding the mutated progesterone ligand binding domain and activating the transregulatory domains of the receptor. RU486 is capable of activating or repressing the appropriate progesterone responsive genes. This is only an example and is not meant to be limiting. Those skilled in the art will recognize that other non-natural ligands can be used.

A method for treating asthma is described. This method includes the transformation of cells associated with the lungs or pulmonary system with the above referenced vectors. The vectors contain nucleic acid which encodes the fusion protein. Once expressed in the lung cells the mutated receptor is capable of transactivating or transrepressing the expression by a non-natural ligand of the appropriate glucocorticoid responsive genes and/or GRE controlled transgenes.

The above methods of treatment invoke use of RU486 as the non-natural ligand. The transactivation and transrepression can occur when the mutated progesterone receptor is activated by RU486. The genes that are transrepressed or transactivated in response to ligand binding to the fusion protein are described above.

An aspect of the present invention features a transgenic non-human animal whose cells contain the vectors of the present invention. These cells include germ or somatic cells. Non-human transgenic animal models can be used for understanding of molecular carcinogenesis and disease, assessing and exploring novel therapeutic avenues for effects by potential chemical and physical carcinogens and tumor promoters.

By "transgenic animal" is meant an animal whose genome contains an additional copy or copies of the gene from the same species or it contains the gene or genes of another species, such as a gene encoding for a mutated glucocorticoid receptor introduced by genetic manipulation or cloning techniques, as described herein and as known in the art. The transgenic animal can include the resulting animal in which the vector has been inserted into the embryo from which the animal developed or any progeny of that animal. The term "progeny" as used herein includes direct progeny of the transgenic animal as well as any progeny of succeeding progeny. Thus, one skilled in the art will readily recognize that if two different transgenic animals have been made each utilizing a different gene or genes and they are mated, the possibility exists that some of the resulting progeny will contain two or more introduced genes. One skilled in the art will readily recognize that by controlling the matings, transgenic animals containing multiple introduced genes can be made.

Other features and advantages of the invention will be apparent from the following description of the preferred embodiments thereof and from the claims.

### Brief Description of the Drawings

Figure 1 shows the mutagenesis and screening strategy used in the present experiments.
Figure 2 illustrates the functional and structural characterization of the UP-1 mutant.
Figure 3 shows a western analysis of the mutant human progesterone receptor.
Figure 4 shows the transcriptional activity and hormone binding analysis of wild type and mutant human progesterone receptor constructs.
Figure 5 shows the specificity of transcriptional activity of the mutant human progesterone receptor.
Figure 6 depicts the transient transfection of mutant human progesterone human receptor into mammalian cells.

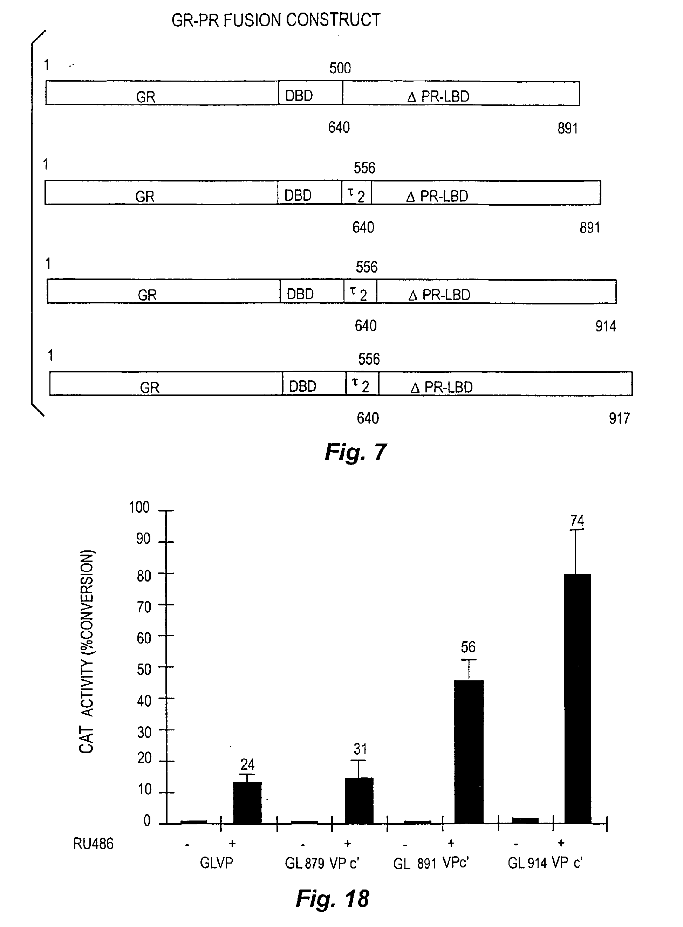

Figure 7 depicts the GR-PR fusion constructs.

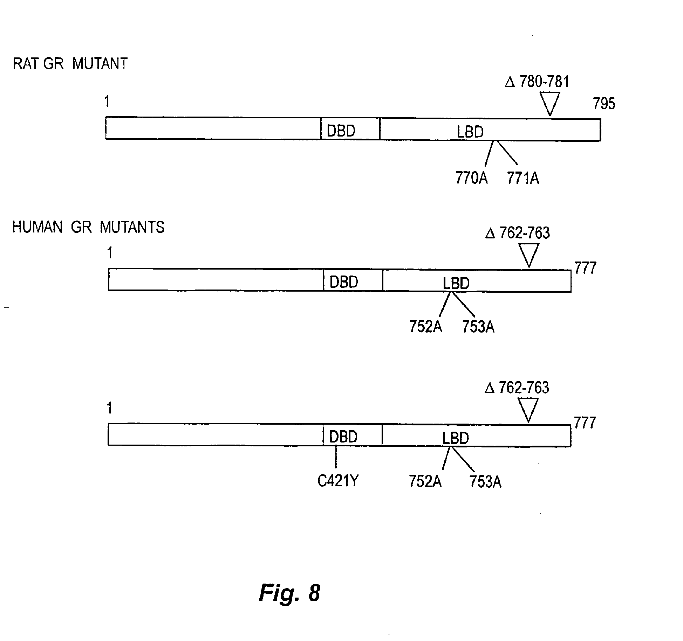

Figure 8 depicts the Rat and Human GR double point mutation constructs.
Figure 9 illustrates the nucleic acid sequence encoding a plasmid pGR0403R expressing a constitutively active mutant GR protein.
Figure 10 depicts plasmid pGR0403R expressing a constitutively active mutant GR protein.
Figure 11 illustrates the amount of CAT protein produced in response to ligand binding to mutant human and rat GR and the respective wild type receptors.

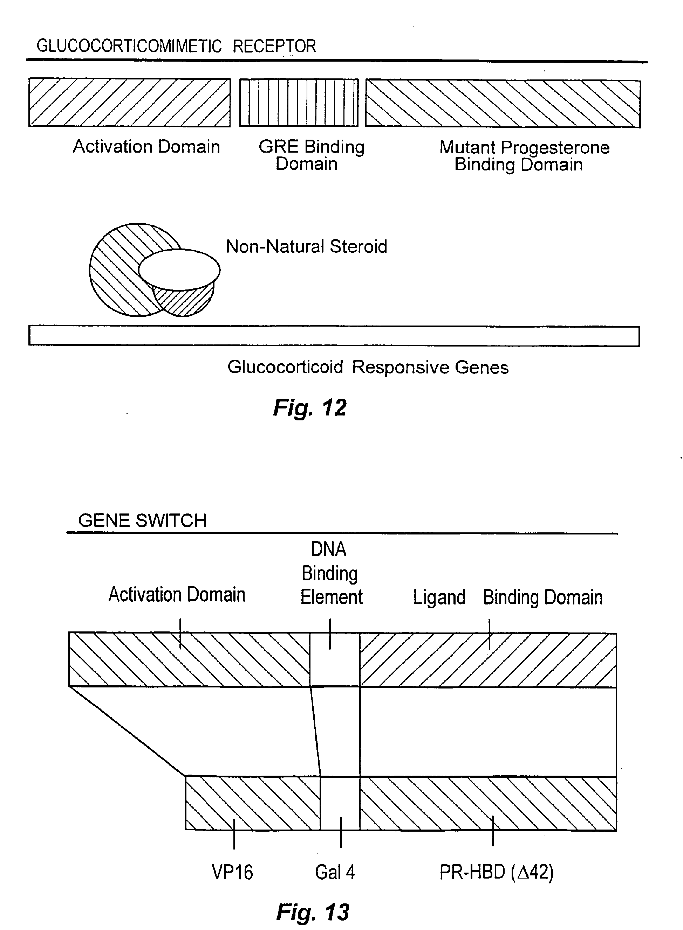

Figure 12 is a schematic representation of the fusion protein with an activation transregulatory domain.
Figure 13 is a schematic representation of the gene switch.
Figure 14 is a schematic representation of GLVP and its derivatives containing an additional transactivation domain.
Figure 15 is a schematic representation of the effect of various lengths of poly-Q insertion on GLVP transactivation potential.
Figure 16 is a schematic representation that an additional copy of the VP16 activation domain into GLVP does not further increase its transactivation potential.
Figure 17 is a diagram of the original chimeric GLVP and its C-terminally extended derivatives.
Figure 18 is a diagram of the transcriptional activation of GLVP versus its C-terminally located VP16 activation domain and various extensions of the hPR-LBD.
Figure 19 is a diagram of the inducible repressors and reporters constructs.

The drawings are not necessarily to scale. Certain features of the invention may be exaggerated in scale or shown in schematic form in the interest of clarity and conciseness.

### Detailed Description of the Invention

Described herein are modified proteins of steroid hormone receptors. Steroid hormone receptors which may be modified include any of those receptors which comprise the steroid hormone receptor superfamily. Representative examples of such receptors include the estrogen, progesterone, glucocorticoid, mineralocorticoid, androgen, thyroid hormone, retinoic acid, retinoid X and Vitamin D3 receptors.

The modified steroid hormone receptor proteins of the present invention include a steroid receptor region made up of a DNA binding domain, one or more transregulatory domains and a mutated steroid receptor ligand binding region capable of binding a non-natural ligand.

The DNA binding domain contains the receptor regulating sequence and binds DNA. Such a domain may be a yeast GAL4 DNA binding domain. The ligand binding domain binds the specific compound which will activate the receptor, for example RU486.

Several different functional domains have been characterized in transcription factors; they can be either acidic (VP16, GAL4), glutamine-rich (SP1, Oct-1, Oct2A), proline-rich (Oct3/4), or serine- and threonine-rich (Pit1) (Wegner et al., Curr. Opin. in Cell Biol. 5:488-498 (1993)). It is known that different types of transcriptional activation domains interact with different coactivators of the general transcriptional machinery. When different activation domains are fused together in a transactivator, they can synergize with each other to increase its transcriptional potential. Recently, Gerber, *et al*. demonstrated that insertion of either a poly-glutamine (poly-Q) or poly-proline (poly-P) stretch within the GAL4-VP16 enhances the activation of GAL4-VP16 (Gerber et al., Science 263:808-811 (1994)).

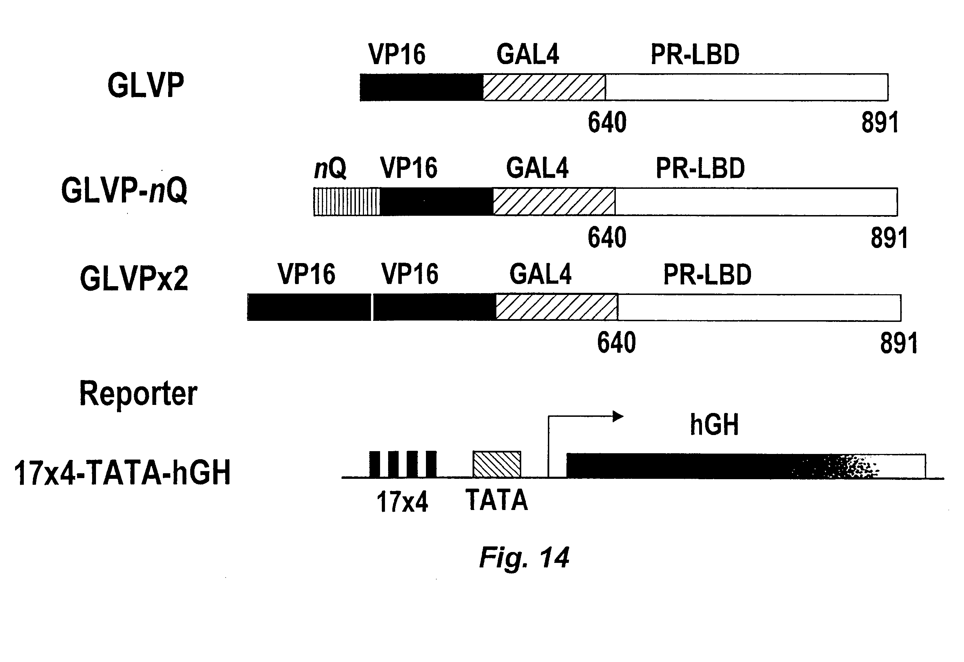

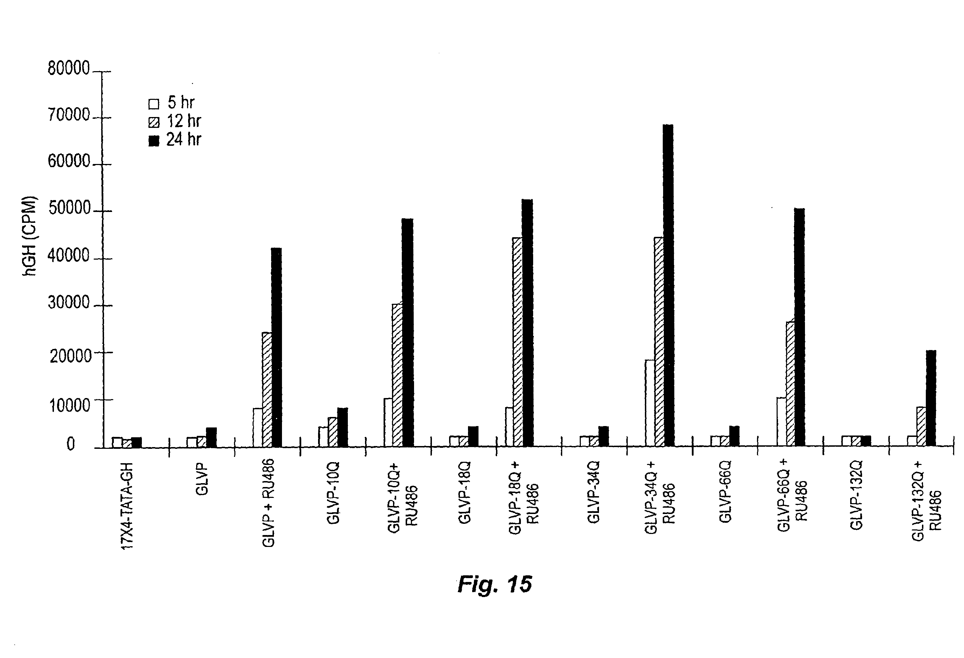

In order to increase the potency of the GLVP regulator, varying lengths of poly-Q stretches encoded by the triplet repeats (CAG)n were inserted into the N-terminus of the GLVP regulator (Figure 14). Transactivation analysis of the various sizes of poly-Q insertions in the GLVP indicate that addition of 10-34Q increases transcriptional activity of the regulator on the reporter gene (17x4-TATA-hGH), while further extension of poly-Q from a 66Q-oligomer to a 132Q-oligomer results in decreased activation of target gene (Figure 15). These experiments demonstrated that a combination of different types of functional domains of appropriate strength further improves the activation potential of the GLVP chimeric regulator.

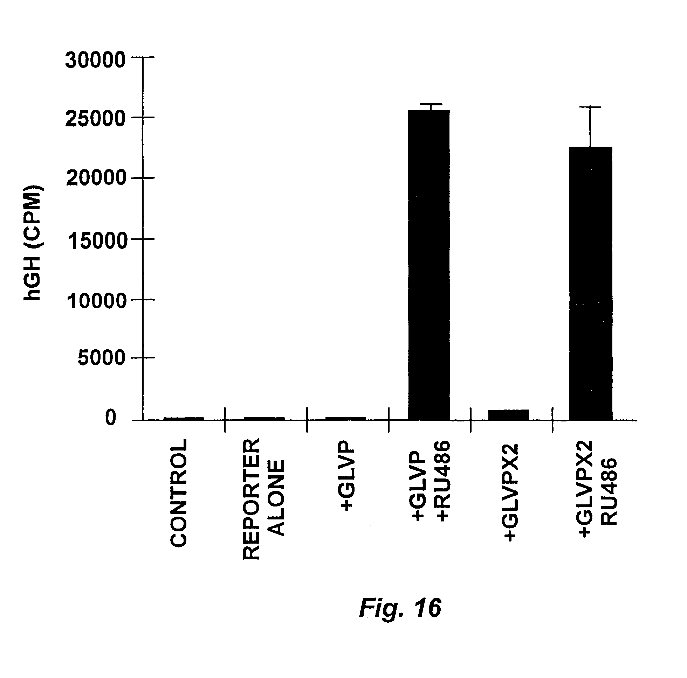

To understand whether additional activation domains of the same type would also increase the activation potential of the chimeric regulator, GLVPx2 with 2 copies of VP16 activation domain at the N-terminus was constructed (Figure 14). As shown in Figure 16, further addition of the same type of transactivation domain (VP16) did not increase the activation potential of the regulator.

The original GLVP can efficiently activate target gene expression containing stronger promoters such as the thymidine kinase (tk) promoter. To further enhance the transcriptional activity of GLVP, a more potent RU486-inducible gene regulator was generated. This new gene regulator responds to RU486 at a concentration even lower than that used by the original GLVP. At this concentration, RU486 does not have any anti-progesterone or anti-glucocorticoid activity. The inducible system has been used successfully to produce secreted NGF from a reporter gene in an RU486 dependent manner to induce neurite outgrowth in co-cultured PC12 cells (of rat adrenal pheochromocytoma). This RU486-controllable ligand binding domain can also be converted to an inducible repressor for shutting down target gene expression. Individual domains within a chimeric fusion protein have been shown to influence each other's function in a position-dependent context.

Transcriptional regulation of gene expression has been intensively studied over the past decade (McKnight, Genes Dev. 10:367-381; Goodrich et al., Curr. Opin. in Cell Biol. 6:403-409; Pugh, Curr. Opin. in Cell Biol. 8:303-311). It is generally believed that transcription factors selectively bind to their recognition sequences on DNA (promoters and enhancers) and directly interact with the TBP-associated factors (TAFs), coactivators, or corepressors to activate or repress transcriptional activity. Nuclear hormone receptors, such as steroid, thyroid, retinoid and orphan receptors, are an unique class of inducible transcription factors that can modulate their respective target genes in response to their cognate ligands. Recently, several coactivators (SRC-1) (Onate et al., Science 270:1354-1357 (1995)), CBP (Kamei et al., Cell 85:403-414 (1996)), and corepressors (N-CoR, SMART) (Hörlein et al., Nature 377:397-404 (1995); Chen et al., Nature 377:454-457 (1995)), that mediate nuclear hormone receptor activation of target genes have been identified. These studies suggest that multiple protein factors are involved in the complex process of transcriptional regulation of gene expression.

Mutagenesis studies of the hPR ligand binding domain have demonstrated that extension of the LBD deletion from amino acid position 891 to 914 increases the activation potential of the chimeric regulator. Addition of this short stretch of 23 amino acids increases the PR-LBD's dimerization potential and subsequent binding to its response element. Further extension of the hPR-LBD from residue 917 to 928 results in a decrease of transactivation, suggesting that this region may serve as a repressor interacting domain. In fact, when this 12 amino acid stretch is ligated to the GAL4 DNA binding domain, it is sufficient to confer transcriptional repression of a target gene, suggesting that these 12 amino acids might interact with a yet unidentified cellular co-repressor.

Many chimeric proteins have been constructed in recent years in order to combine different functional domains of various proteins into one versatile chimera. While it is clear that each protein domain can function independently, relatively little is known about how individual domains modulate each other's function within a chimeric protein. The activation potential of VP16 is influenced by its relative position within the chimeric regulator. The C-terminally located VP16 chimeric regulator GL₉₁₄VP_{C'} effectively activates target gene expression containing a minimal promoter at an RU486 concentration 10-fold lower than its N-terminally located VP16 counterpart, GL₉₁₄VP. At this concentration, RU486 is expected to have no interference with endogenous gene expression.

This new inducible system will afford an improved margin of safety and further contribute to its application for gene regulation *in vivo.* Mutational studies revealed that the chimeric regulator GL₉₁₄VP_{C'} is about 8 to 10 times more potent than our originally described regulator GLVP and responds at a lower ligand concentration. Furthermore, within a chimeric protein, individual functional domains, such as those involved in transactivation, DNA binding and ligand binding, can modulate each other's function, depending on their relative positions.

Protein-protein interaction studies suggest that different types of transactivation or transrepression domains interact with their respective TAFs or coactivator, corepressor molecules within the RNA polymerase II preinitiation complex to alter gene transcription (Pugh, Curr. Opin. in Cell Biol. 8:303-311; Goodrich et al., Cell 75:519-530 (1993)). Glutamine rich stretches have been identified in various transcriptional factors (SP1, Oct-1 and androgen receptor) although their precise function is unknown (Wegner et al., Curr. Opin. in Cell Biol. 5:488-498 (1993); Gerber et al., Science 263:808-811 (1994)). Expanded regions of triplet CAG repeats have been implicated in several neurodegenerative diseases such as Huntington's, Kennedy's, dentatorubral-pallidoluysian atrophy (DRPLA), and hereditary spinocerebellar ataxias (SCA1) (Kuhl et al., Curr. Opin. in Genet. Dev. 3:404-407 (1993); Ross et al., Trends in Neurosci 16:254-260 (1993)); Ashley et al., Annu. Rev. Genet. 29:703-728 (1995)).

Recently, several groups have isolated proteins responsible for the above mentioned neurodegenerative diseases and confirmed that they indeed contain long poly-glutamine (Q) stretches encoded by the expanded CAG repeats (Servadio et al., Nature Genet. 10:94-98 (1995); Yazawa et al., Nature Genet. 10:99-103 (1995); Trottier et al., Nature Genet. 10:104-110 (1995)). To further understand the role of poly-Q stretches in transcriptional regulation, various lengths of poly-Q was inserted in the N-terminus of GLVP.

Addition of a 10-34 oligomer of poly-Q results in synergistic transcriptional activation, while expanded CAG triplet repeats beyond 66 oligomeric glutamines do not further increase the transactivation potential of chimeric regulator GLVP. These observations suggest that structural and conformational changes might be involved in proteins encoded by the expanded CAG triplet repeat as compared with the regular length poly-Q which encoded by 10-30 repeats of CAG in normal protein. These results suggest that a neurological disease with expanded CAG repeats (>40 mer) may not be due to aberrant high transcriptional potential but rather due to an influence on other aspects of cell function (Burke et al., Nature Medecine 2:347-350 (1996)).

A transcription factor can either activate or repress gene expression depending on the promoter/enhancer context of its particular target DNA and the coregulator proteins with which it interacts (Kingston et al. Genes Dev. 10:905-920 (1996)). For example, in the absence of thyroid hormone (T3), the thyroid hormone receptor (TR) normally binds to its recognition sequence on DNA and represses target gene activation through interactions with corepressors (Baniahmad et al., Mol. Cell. Biol. 15:76-86 (1995); Shibata et al. (unpublished) (1996); Chen et al., Nature 377:454-457 (1995)). In the presence of T3, the co-repressor is released from the receptor and coactivators are recruited to enhance gene expression. Many transcription factors, such as p53, WT-1, YY1, Rel, can also act as dual activators and repressors depending on the DNA template and protein co-factors with which they interact.

The *Drosophila* zinc finger transcription factor, Krüppel, is encoded by a gap gene and is essential for organogenesis during later stages of the development. Through *in vitro* protein-protein interaction studies, Sauer et al. have demonstrated that the Kruppel protein can act as a transcriptional activator at low protein concentration (monomeric form) by interacting with TFIIB. However, at higher protein concentration, Kruppel forms a dimer and directly interacts with TFIIEβ resulting in transcriptional repression. Several Krüppel related proteins recently have been identified in mammalian cells (Witzgall et al., Mol. Cell. Biol. 13:1933-42 (1993); Witzgall et al., Proc. Natl. Acad. Sci. 91:4514-4518 (1994); Margolin et al., Proc. Natl. Acad. Sci. 91:4509-4513 (1994)). One of them, *Kid-1,* was isolated from rat kidney and contains a highly conserved region of ~75 amino acids at the N-terminus termed Krüppel-associated box (KRAB).

It has been shown that the KRAB domain can act as a potent repressor when fused to a yeast GAL4 DNA binding domain or TetR (Deuschle et al., Mol. Cell. Biol. 15:1907-1914 (1995)). Replacement of the VP16 transcriptional activation domain with the *Kid-1* KRAB repression domain, converted a regulatable transactivator into a regulatable repressor. By exchanging the GAL4 DNA binding domain with the DNA binding domain of another protein, repression of a target gene (e.g., tumor proliferation gene) may be achieved in response to ligand RU486. Recently, Deuschle et al. reported that the KRAB domain isolated from *Kox1* zinc finger protein, which shares extensively homology with that of *Kid-1,* interacts with a 110 kDa adaptor protein termed SMP1 (silencing-mediating protein 1). The characteristics and mechanism of this adaptor protein have yet to be determined. Recently, a KRAB-associated protein-1 (KAP-1) was identified which binds to the KRAB domain and functions as a transcriptional co-repressor (Friedman et al., Gene and Dev. 10:2067 (1996)).

Using the newly modified GL₉₁₄VP_{C'}, regulation of neurite outgrowth in PC12 cells via RU486 controllable expression of NGF was achieved. This novel inducible system can be employed to analyze biological function in a temporal manner. For example, the role of a growth factor could be assessed at a particular stage of development and the sequential relationship of *in vivo* cell death and proliferation could be delineated in a manner not possible with constitutive expression of the test gene.

Tissue specific regulation of gene expression in transgenic mice utilizing this inducible system was demonstrated. RU486 inducible regulator may be used to create an inducible gene knockout (temporal and/or spatial) in transgenic mice which could circumvent an embryonic lethality resulting from use of current gene knockout techniques. Combinatorial inclusion of other inducible systems such as the tetracycline or ecdysone system with the RU486 inducible system may allow biologists one day to modulate complex biological processes which involve multiple levels of control.

The following are examples of the present invention using the mutated steroid receptors for gene therapy. It will be readily apparent to one skilled in the art that various substitutions and modifications may be made to the invention disclosed herein without departing from the scope and spirit of the invention. Thus, these examples are offered by way of illustration and are not intended to limit the invention in any manner.

The following are specific examples of preferred embodiments of the present invention. These examples demonstrate how the molecular switch mechanisms of the present invention can be used in construction of various cellular or animal models and how such molecular switch mechanisms can be used to transactivate or transrepress the regulation of gene expression.

### Methods of Use

### Cell Transformation

One embodiment of the present invention includes cells transformed with nucleic acid encoding for the mutated receptor. Once the cells are transformed, the cells will express the protein, polypeptide, or RNA encoded for by the nucleic acid, Cells include but are not limited to joints, lungs, muscle and skin. This is not intended to be limiting in any manner.

The nucleic acid which contains the genetic material of interest is positionally and sequentially oriented within the host or vectors such that the nucleic acid can be transcribed into RNA and, when necessary, be translated into proteins or polypeptides in the transformed cells. A variety of mutated glucocorticoid proteins and polypeptides can be expressed by the sequence in the nucleic acid cassette in the transformed cells.

Transformation can be done either by *in vivo* or *ex vivo* techniques. One skilled in the art will be familiar with such techniques for transformation. Transformation by *ex vivo* techniques includes co-transfecting the cells with DNA containing a selectable marker. This selectable marker is used to select those cells which have become transformed. Selectable markers are well known to those who are skilled in the art.

For example, one approach to gene therapy for muscle diseases is to remove myoblasts from an affected individual, genetically alter them *in vitro,* and reimplant them into a receptive locus. The *ex vivo* approach includes the steps of harvesting myoblasts cultivating the myoblasts, transducing or transfecting the myoblasts, and introducing the transfected myoblasts into the affected individual.

The myoblasts may be obtained in a variety of ways. They may be taken from the individual who is to be later injected with the myoblasts that have been transformed or they can be collected from other sources, transformed and then injected into the individual of interest.

Once the *ex vivo* myoblasts are collected, they may be transformed by contacting the myoblasts with media containing the nucleic acid transporter and maintaining the cultured myoblasts in the media for sufficient time and under conditions appropriate for uptake and transformation of the myoblasts. The myoblasts may then be introduced into an appropriate location by injection of cell suspensions into tissues. One skilled in the art will recognize that the cell suspension may contain: salts, buffers or nutrients to maintain viability of the cells; proteins to ensure cell stability; and factors to promote angiogenesis and growth of the implanted cells.

In an alternative method, harvested myoblasts may be grown *ex vivo* on a matrix consisting of plastics, fibers or gelatinous materials which may be surgically implanted in an appropriate location after transduction. This matrix may be impregnated with factors to promote angiogenesis and growth of the implanted cells. Cells can then be reimplanted.

### Administration

Administration as used herein refers to the route of introduction of a vector or carrier of DNA into the body. Administration may include intravenous, intramuscular, topical, or oral methods of delivery. Administration can be directly to a target tissue or through systemic delivery.

In particular, the present invention can be used for treating disease or for administering the formulated DNA expression vectors capable of expressing any specific nucleic acid sequence. Administration can also include administering a regulatable vector discussed above. Such administration of a vector can be used to treat disease. The preferred embodiment is by direct injection to the target tissue or systemic administration.

A second critical step is the delivery of the DNA vector to the nucleus of the target cell where it can express a gene product. In the present invention this is accomplished by formulation. The formulation can consist of purified DNA vectors or DNA vectors associated with other formulation elements such as lipids, proteins, carbohydrates, synthetic organic or inorganic compounds. Examples of such formulation elements include, but are not limited to, lipids capable of forming liposomes, cationic lipids, hydrophilic polymers, polycations (e.g., protamine, polybrene, spermidine, polylysine), peptide or synthetic ligands recognizing receptors on the surface of the target cells, peptide or synthetic ligands capable of inducing endosomal lysis, peptide or synthetic ligands capable of targeting materials to the nucleus, gels, slow release matrices, soluble or insoluble particles, as well as other formulation elements not listed. This includes formulation elements for enhancing the delivery, uptake, stability, and/or expression of genetic material into cells.

The delivery and formulation of any selected vector construct will depend on the particular use for the expression vectors. In general, a specific formulation for each vector construct used will focus on vector uptake with regard to the particular targeted tissue, followed by demonstration of efficacy. Uptake studies will include uptake assays to evaluate cellular uptake of the vectors and expression of the tissue specific DNA of choice. Such assays will also determine the localization of the target DNA after uptake, and establish the requirements for maintenance of steady-state concentrations of expressed protein. Efficacy and cytotoxicity can then be tested. Toxicity will not only include cell viability but also cell function.

DNA uptake by cells associated with fluid spaces have the unique ability to take up DNA from the extracellular space after simple injection of purified DNA preparations into the fluid spaces. Expression of DNA by this method can be sustained for several months.

Incorporating DNA by formulation into particulate complexes of nanometer size that undergo endocytosis increases the range of cell types that will take up foreign genes from the extracellular space.

Formulation can also involve DNA transporters which are capable of forming a non-covalent complex with DNA and directing the transport of the DNA through the cell membrane. This may involve the sequence of steps including endocytosis and enhanced endosomal release. It is preferable that the transporter also transport the DNA through the nuclear membrane. See, *e.g*.,

Woo et al., WO 93/18759 entitled "A DNA Transporter System and Method of Use".

In addition, delivery can be cell specific or tissue specific by including cell or tissue specific promoters. Furthermore, mRNA stabilizing sequences (3' UTR's) can be used to provide stabilized modified receptor molecules. Such stabilizing sequences increase the half-life of mRNAs and can be cell or tissue specific. The above is discussed in more detail in U.S. Patent 5,238,422 (Schwartz et al.).

In a preferred method of administration involving a DNA transporter system, the DNA transporter system has a DNA binding complex with a binding molecule capable of non-covalently binding to DNA which is covalently linked to a surface ligand. The surface ligand is capable of binding to a cell surface receptor and stimulating entry into the cell by endocytosis, pinocytosis, or potocytosis. In addition, a second DNA binding complex is capable of non-covalently binding to DNA and is covalently linked to a nuclear ligand. The nuclear ligand is capable of recognizing and transporting a transporter system through a nuclear membrane. Additionally, a third DNA binding complex may be used which is also capable of non-covalently binding to DNA. The third binding molecule is covalently linked to an element that induces endosomal lysis or enhanced release of the complex from the endosome after endocytosis. The binding molecules can be spermine, spermine derivatives, histones, cationic peptides and/or polylysine. See also Szoka, C.F., Jr. et al., Bioconjug. Chem. 4:85-93 (1993); Szoka, F.C., Jr. et al., P.N.A.S., 90:893-897 (1993).

Transfer of genes directly has been very effective. Experiments show that administration by direct injection of DNA into joint tissue results in expression of the gene in the area of injection. Injection of plasmids containing the mutated receptors into the spaces of the joints results in expression of the gene for prolonged periods of time. The injected DNA appears to persist in an unintegrated extrachromosomal state. This means of transfer is the preferred embodiment.

The formulation used for delivery may also be by liposomes or cationic lipids. Liposomes are hollow spherical vesicles composed of lipids arranged in a similar fashion as those lipids which make up the cell membrane. They have an internal aqueous space for entrapping water soluble compounds and range in size from 0.05 to several microns in diameter. Several studies have shown that liposomes can deliver nucleic acids to cells and that the nucleic acid remains biologically active. Cationic lipid formulations such as formulations incorporating DOTMA has been shown to deliver DNA expression vectors to cells yielding production of the corresponding protein. Lipid formulations may be non-toxic and biodegradable in composition. They display long circulation half-lives and recognition molecules can be readily attached to their surface for targeting to tissues. Finally, cost effective manufacture of liposome-based pharmaceuticals, either in a liquid suspension or lyophilized product, has demonstrated the viability of this technology as an acceptable drug delivery system. See Szoka, F.C., Jr. et al., Pharm. Res., 7:824-834 (1990); Szoka, F.C., Jr. et al., Pharm. Res., 9:1235-1242 (1992).

The chosen method of delivery should result in nuclear or cytoplasmic accumulation and optimal dosing. The dosage will depend upon the disease and the route of administration but should be between 1-1000 *µ*g/kg of body weight. This level is readily determinable by standard methods. It could be more or less depending on the optimal dosing. The duration of treatment will extend through the course of the disease symptoms, possibly continuously. The number of doses will depend upon disease, the formulation and efficacy data from clinical trials.

With respect to vectors, the pharmacological dose of a vector and the level of gene expression in the appropriate cell type includes but is not limited to sufficient protein or RNA to either: (1) increase the level of protein production; (2) decrease or stop the production of a protein; (3) inhibit the action of a protein; (4) inhibit proliferation or accumulation of specific cell types; and (5) induce proliferation or accumulation of specific cell types. As an example, if a protein is being produced which causes the accumulation of inflammatory cells within the joint, the expression of this protein can be inhibited, or the action of this protein can be interfered with, altered, or changed.

### Persistent Expression Using Episomal Vectors

In each of the foregoing examples, transient expression of recombinant genes induces the desired biological response. In some diseases more persistent expression of recombinant genes is desirable. This is achieved by adding elements which enable extrachromosomal (episomal) replication of DNA to the structure of the vector. Vectors capable of episomal replication are maintained as extrachromosomal molecules and can replicate. These sequences will not be eliminated by simple degradation but will continue to be copied. Episomal vectors provide prolonged or persistent, though not necessarily stable or permanent, expression of recombinant genes in the joint. Persistent as opposed to stable expression is desirable to enable adjustments in the pharmacological dose of the recombinant gene product as the disease evolves over time.

### Formulations for Gene Delivery into Cells of the Joint

Initial experiments used DNA in formulations for gene transfer into cells of the joint. This DNA is taken up by synovial cells during the process of these cells continually resorbing and remodeling the synovial fluid by secretion and pinocytosis. Gene delivery is enhanced by packaging DNA into particles using cationic lipids, hydrophilic (cationic) polymers, or DNA vectors condensed with polycations which enhance the entry of DNA vectors into contacted cells. Formulations may further enhance entry of DNA vectors into the body of the cell by incorporating elements capable of enhancing endosomal release such as certain surface proteins from adenovirus, influenza virus hemagglutinin, synthetic GALA peptide, or bacterial toxins. Formulations may further enhance entry of DNA vectors into the cell by incorporating elements capable of binding to receptors on the surface of cells in the joint and enhancing uptake and expression. Alternatively, particulate DNA complexed with polycations can be efficient substrates for phagocytosis by monocytes or other inflammatory cells. Furthermore, particles containing DNA vectors which are capable of extravasating into the inflamed joint can be used for gene transfer into the cells of the joint. One skilled in the art will recognize that the above formulations can also be used with other tissues as well.

### Induction of "Steroid Response" by Gene Transfer of Steroid Receptors into Cells of the Joint

Current therapy for severe arthritis involves the administration of pharmacological agents including steroids to depress the inflammatory response. Steroids can be administered systemically or locally by direct injection into the joint space.

Steroids normally function by binding to receptors within the cytoplasm of cells. Formation of the steroid-receptor complex changes the structure of the receptor so that it becomes capable of translocating to the nucleus and binding to specific sequences within the genome of the cell and altering the expression of specific genes. Genetic modifications of the steroid receptor can be made which enable this receptor to bind non-natural steroids. Other modifications can be made to create a mutated steroid receptor which is "constitutively active" meaning that it is capable of binding to DNA and regulating gene expression in the absence of steroid in the same way that the natural steroid receptor regulates gene expression after treatment with natural or synthetic steroids.

Of particular importance is the effect of glucocorticoid steroids such as cortisone, hydrocortisone, prednisone, or dexamethasone which are effective drugs available for the treatment of arthritis. One approach to treating arthritis is to introduce a vector in which the nucleic acid cassette expresses a genetically modified steroid receptor into cells of the joint, *e.g.*, a genetically modified steroid receptor which mimics the effect of glucocorticoid but does not require the presence of glucocorticoid for effect. This is achieved by expression of a fusion receptor protein discussed above or other mutated glucocorticoid receptors such as ones which are constitutively active within cells of the joint. This induces the therapeutic effects of steroids without the systemic toxicity of these drugs.

Alternatively, construction of a steroid receptor which is activated by a novel, normally-inert steroid enables the use of drugs which would affect only cells taking up this receptor. These strategies obtain a therapeutic effect from steroids on arthritis without the profound systemic complications associated with these drugs. Of particular importance is the ability to target these genes differentially to specific cell types (for example synovial cells versus lymphocytes) to affect the activity of these cells.

The steroid receptor family of gene regulatory proteins is an ideal set of such molecules. These proteins are ligand activated transcription factors whose ligands can range from steroids to retinoic acid, fatty acids, vitamins, thyroid hormones and other presently unidentified small molecules. These compounds bind to receptors and either activate or repress transcription.

Progesterone receptor fusion proteins can be modified to allow them to bind various ligands whose structure differs from naturally occurring ligands. For example, small C-terminal alterations in amino acid sequence, including truncation, result in altered affinity of ligand binding to the progesterone receptor. By screening receptor mutants, receptors can be customized to respond to ligands which do not activate the host cell endogenous receptors.

A person having ordinary skill in the art will recognize, however, that various mutations, for example, a shorter deletion of carboxy terminal amino acids, will be necessary to create useful mutants of certain steroid hormone receptor proteins. Steroid hormone receptors which may be mutated are any of those receptors which comprise the steroid hormone receptor superfamily, such as receptors including the estrogen, progesterone, glucocorticoid-α, glucocorticoid-β, mineral corticoid, androgen, thyroid hormone, retinoic acid, and Vitamin D3 receptors.

### Direct DNA Delivery to Muscle

Diseases that result in abnormal muscle development, due to many different reasons can be treated using the above modified progesterone receptors. These diseases can be treated by using the direct delivery of genes encoding for the mutated progesterone receptor resulting in the production of mutated receptor gene product. Genes which can be repressed or activated have been outlined in detail above.

### Direct DNA Delivery to the Lungs

Current therapy for severe asthma involves the administration of pharmacological agents including steroids to inhibit the asthma response. Steroids can be administered systemically or locally by direct instillation or delivery into the lungs.

Of particular importance is the effect of glucocorticoid steroids such as cortisone, hydrocortisone, prednisone, or dexamethasone which are the most important-effective drugs available for the treatment of asthma. One approach to treating asthma is to introduce a vector in which the nucleic acid cassette expresses a genetically modified steroid receptor into cells of the lungs, *e.g*., a genetically modified steroid receptor which mimics the effect of glucocorticoid but does not require the presence of glucocorticoid for effect. This is achieved by expression of the fusion proteins discussed above or other mutated glucocorticoid receptors such as ones which are constitutively active within cells of the lungs. This induces the therapeutic effects of steroids without the systemic toxicity of these drugs.

Alternatively, construction of a steroid receptor which is activated by a novel, normally-inert steroid enables the use of drugs which would affect only cells taking up this receptor. These strategies obtain a therapeutic effect from steroids on asthma without the profound systemic complications associated with these drugs. Of particular importance is the ability to target these genes differentially to specific cell types (for example alveoli of the lungs) to affect the activity of these cells.

The steroid receptor family of gene regulatory proteins is an ideal set of such molecules. -These proteins are ligand-activated transcription factors whose ligands can range from steroids to retinoids, fatty acids, vitamins, thyroid hormones, and other presently unidentified small molecules. These compounds bind to receptors and either up-regulate or down-regulate transcription.

The preferred receptor of the present invention is the modified progesterone receptor. These receptors can be modified to allow them to bind various ligands whose structure differs from naturally occurring ligands. For example, small C-terminal alterations in amino acid sequence, including truncation, result in altered affinity of the ligand and altered function. By screening receptor mutants, receptors can be customized to respond to ligands which do not activate the host cells own receptors.

A person having ordinary skill in the art will recognize, however, that various mutations, for example, a shorter deletion of carboxy terminal amino acids, will be necessary to create useful mutants of certain steroid hormone receptor proteins. Steroid hormone receptors which may be mutated are any of those receptors which comprise the steroid hormone receptor superfamily, such as receptors including the estrogen, progesterone, glucocorticoid-α, glucocorticoid-β, mineral corticoid, androgen, thyroid hormone, retinoic acid, and Vitamin D3 receptors.

### Examples

While the present invention is disclosed by reference to the details for the following examples, it is to be understood that this disclosure is intended in an illustrative rather than limiting sense, as it is contemplated that modifications will readily occur to those skilled in the art.

### Mutagenesis and Characterization of the Ligand Binding Domain of Human Progesterone Receptor

### Example 1

### Yeast Strain

The *Saccharomyces cerevisiae* strain BJ3505 (MATα, pep4:HIS3, prb1-Δ1.6R, his3Δ200, lys2-801, trpl-Δ101, ura3-52, gal2, (CUP1)) was used (Yeast Genetic Stock Center, Berkeley, CA). All yeast transformations were carried out following the lithium acetate transformation protocol (Ito, et al., J. Bacteriol. 153:163-168, 1983).

The PCR reactions were carried out using YEphPR-B DNA template (a YEp52AGSA-derived yeast expression plasmid containing the cDNA of hPR form-B (Misrahi, et al., Biochem. Bioph. Res. Comm. 143:740-748, 1987) inserted downstream of the yeast methallothionein-CUP1 promoter) and using three different sets of primers. In order to decrease the fidelity of the second strand polymerization reaction, buffer conditions of 1.5 mM MgCl₂, 0.1 mM dNTPs and pH 8.2 were used. About 2000 primary transformants were obtained from each region-specific library.

### Example 2

### Yeast Mutant Screening

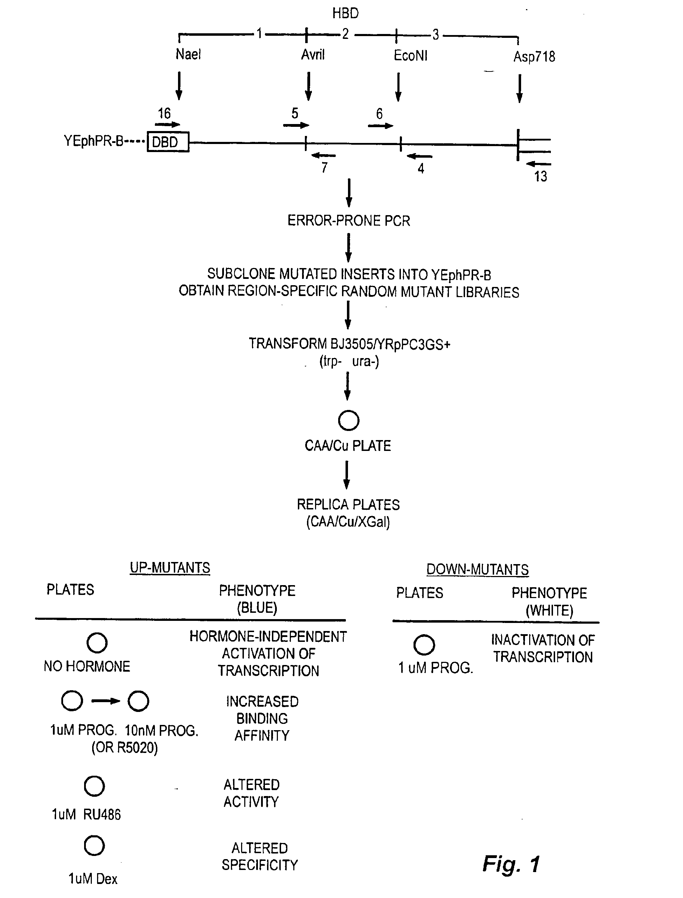

Colonies of each library of hPR molecules mutated in specific subregions were pooled, large amounts of DNA were prepared and used to transform yeast cells carrying the reporter plasmid YRpPC3GS+, which contains two GRE/PRE elements upstream of the CYC1 promoter linked to the Lac-Z gene of *E*. *coli* (Mak, et al., J. Biol. Chem. 265:20085-20086, 1989). The transformed cells were plated on 1.5% agar plates containing 2% glucose, 0.5% casamino acids (5% stock solution of casamino acids is always autoclaved before use to destroy tryptophan), 6.7 g/l yeast nitrogen base (without amino acids) and 100 µM CuSO4 (CAA/Cu plates) and grown for 2 days at 30°C. These colonies were then replica-plated on CAA/Cu plates containing 0.16 g/l of 5-bromo-4-chloro-3-indolyl-β-D-galactoside (X-Gal, an indicator of β-galactosidase activity) with or without the hormones as indicated in Fig. 1 and allowed to grow for one day at 30°C, then two days at room temperature in the dark.

### Example 3

### Growth of Yeast Culture for In Vitro Assay

*Saccharomyces cerevisiae* cells containing YEphPRB and the reporter plasmid were grown overnight at 30°C in minimal media containing 2% glucose. The cells were subcultured in fresh medium and allowed to grow until early mid-log phase (O.D.₆₀₀ₙₘ=1.0). Induction of receptor was initiated by the addition of 100 µM copper sulfate to the culture. Cells were harvested by centrifugation at 1,500 xg for 10 minutes and resuspended in the appropriate buffer. This and all subsequent steps of analysis of the yeast extracts were done at 4°C.

### Example 4

### Transcription Assay

Yeast cells containing the reporter and expression plasmids were grown overnight as described above in Example 3 in the presence of 100 µM copper sulfate. When the cell density reached O.D.₆₀₀ₙₘ=1.0, hormones were added to the cultures. After a 4 hour incubation, yeast extracts were prepared and assayed for β-galactosidase activity as described previously (Miller, J. M. Miller ed., 352-355 (1972)).

Generally, reporters useful in the present invention are any which allow for appropriate measurement of transcription levels. Preferable reporter systems include reporter vectors comprised of the yeast iso-1-cytochrome C proximal promoter element fused to a structural gene, wherein said structural gene is selected from the group consisting of β-galactosidase, galactokinase and URA3. More preferably, the vector is comprised of an insertion site for a receptor response element. The vectors which include β-galactokinase as an indicator of transcriptional activity are derived from the parent vector PC2 while the vectors which include galactokinase are derived from YCpR1 vector. Preferably, the structural genes originate from *E*. *coli.*

### Example 5

### Western Immunoblotting

Yeast cells were grown as discussed above for the transcription assay. Yeast extracts for Western blot analysis were prepared by resuspending the cell pellet in TEDG+salts. The cell suspension was mixed with an equal volume of glass beads and disrupted by vortexing in a microcentrifuge tube. The homogenate was centrifuged at 12,000 x g for 10 minutes. The supernatant was collected and the protein concentration was estimated using bovine serum albumin as standard. Yeast extracts were resolved on a 0.1% sodium dodecyl sulfate-7% polyacrylamide gel and transferred to Immobilon membrane as described previously (McDonnell, et al., Mol. Cell. Biol. 9:3517-3523, 1989). Solid phase radioimmunoassay was performed using a monoclonal antibody (JZB39) directed against the N-terminal domain of A and B forms of hPR.

### Example 6

### Hormone Binding Competition Assays

Induction of PR synthesis was initiated by the addition of 100 µM CuSO₄ to the culture and incubation was continued for 6 hours. The cell pellet was resuspended in TESH buffer containing 1 µg/ml leupeptin, 10 µg/ml PMSF and 10 µg/ml pepstatin. The cell suspension was mixed with an equal volume of glass beads (0.5 mm; B. Braun Instruments) and disrupted by vortexing in a microcentrifuge tube. The homogenate was centrifuged at 12,000 x g for 10 minutes and the supernatant was further centrifuged at 100,000 x g for 30 minutes to obtain a cytosol fraction. Diluted yeast extracts (200 µl) containing 100 µg of total protein were incubated overnight at 4°C with [³H]ligand in the absence (total binding) or presence (nonspecific binding) of a 100-fold excess of unlabeled ligand. Bound and free steroids were separated by addition of 500 µl of dextran-coated charcoal suspension (0.5% Norit A, 0.05% dextran, 10 mM Tris HC1, pH 7.4 and 1 mM EDTA). Specific binding was determined by subtracting nonspecific from total binding. Scatchard analysis was carried out as described previously by Mak, et al., *J*. *Biol. Chem.* 264:21613:21618 (1989).

### Example 7

### Site-Directed Mutagenesis

Mutants YEphPR-B879 and YEphPR-B891 were prepared following the procedure described by Dobson, et al., J. Biol. Chem. 264:4207-4211 (1989). CJ236 cells were infected with mpPR90 (an M13 plasmid containing hPR cDNA). The resulting uridine-containing single-stranded DNA was annealed to 20-mer oligonucleotides containing a TGA stop codon corresponding to amino acids 880 and 892, respectively.

### Example 8

### Construction of Mammalian Expression Vectors

The mammalian expression vector phPR-B contains the SV40 enhancer sequence upstream of the human growth hormone promoter linked to the hPR-B cDNA. This vector was digested with Sall and EcoRl. The 6.1kb fragment (containing the vector sequences and the 5'-1.5 kb of the hPR) was gel-purified and ligated to the 2.1 kb fragment of YEphPR-B891 (containing the 3'-end of the receptor) previously cut with Sall and EcoRl. The resulting plasmid, phPR-B891, encodes a 42 amino acid truncated version of hPR form B.

### Example 9

### Mammalian Cell Transient Transfections and CAT-Assays

Five micrograms of chloramphenicol acetyltransferase (CAT) reporter plasmid, containing two copies of a PRE/GRE from the tyrosine amino transferase gene linked to the thymidine kinase promoter (PRETKCAT), were used in transient cotransfection experiments together with 5 µg of wild type or mutant receptor DNAs. Transient cotransfections and CAT-assays were performed as described by Tsai et al., Cell 57:443-448 (1989).

### Example 10

### Mutagenesis of the Hormone Binding Domain of hPR-B

In order to characterize amino acids within the hPR HBD which are critical for ligand binding and hormone-dependent transactivation, libraries of mutated hPR molecules were created and the mutants introduced into a reconstituted progesterone-responsive transcription system in yeast. This system allowed the screening of large numbers of mutant clones and the direct, visual identification of phenotypes.

Unique restriction sites for NaeI, AvrII and EcoNI were created in the cDNA of hPR, obtaining three cassettes of 396, 209 and 400 nucleotides (regions 1, 2 and 3, respectively). For PCR mutagenesis three sets of primers (16 + 7 for region 1, 5 + 4 for region 2 and 6 + 13 for region 3) were used in the polymerization reaction using YEphPR-B as DNA template. The fragments obtained after PCR were digested with the appropriate enzymes, gel-purified and ligated into the parental plasmid YEphPR-B. Ligation mixes were used to transform bacterial cells and to obtain libraries of hPR molecules randomly point-mutated in the HBD. 5 µg of DNA were used from each library to transform yeast cells carrying the reporter plasmid YRpPC3GS+ and transformants were selected for tryptophan and uracil auxotrophy on CAA plates containing 100 µM CuSO₄. These were then replicated on CAA plates containing the hormones. The screening for "up-mutations" allowed identification of receptor mutants with hormone-independent transcriptional activity, or increased affinity for the ligand (these clones should remain blue when grown with 100-fold less hormone), or with an altered response to RU486 or a glucocorticoid analogue. In the "down-mutation" screening, receptor mutants that were transcriptionally inactive in the presence of the ligand were detected.

Because of the nature of the method used to generate the mutated DNA templates, it was necessary, firstly, to determine the quality of the libraries obtained. This was assessed by estimating the number of null-mutations generated by mutagenesis. We estimated the frequency of occurrence of transcriptionally inactive receptors (white colonies) compared to the total number of colonies. This frequency was about 7%.

The primary transformants were replica-plated onto plates containing the antiprogestin RU486. The wild type receptor is not activated by this hormone (Fig. 1). Using this screening strategy, a single colony was identified that displayed considerable transcriptional activity in response to the antihormone. Interestingly, the same colony did not display transcriptional activity when replica-plated in the presence of progesterone. The colony was purified and the phenotype was confirmed. Eviction of the expression vector from the clone, followed by reintroduction of the unmutated receptor, demonstrated that the phenotype was indeed related to the expression vector and was not the result of a secondary mutation. In addition, the mutated plasmid called UP-1, was rescued from yeast by passage through *E.coli* (as described in Ward, Nucl. Acids Res. 18:5319 (1990)) and purified. This DNA was then reintroduced into yeast that contained only the reporter plasmid. As expected, the mutant phenotype was stable and related directly to the receptor expression plasmid.

### Example 11

### Characterization of the UP-1 Mutant

The plate assays used to identify the receptor mutants are qualitative in nature. To further characterize the properties of UP-1, the activity of the receptor mutants was compared with that of the wild type receptor in a transcription assay. In this method, yeast cells transformed with either the wild type or the mutant receptor and a progesterone responsive reporter were grown overnight in the presence of 100µM CuSO₄. When the cells had reached an O.D.₆₀₀ₙₘ of 1.0, they were supplemented with progesterone or RU486 and harvested by centrifugation after four hours. The β-galactosidase activity in the cell cytosol was then measured.

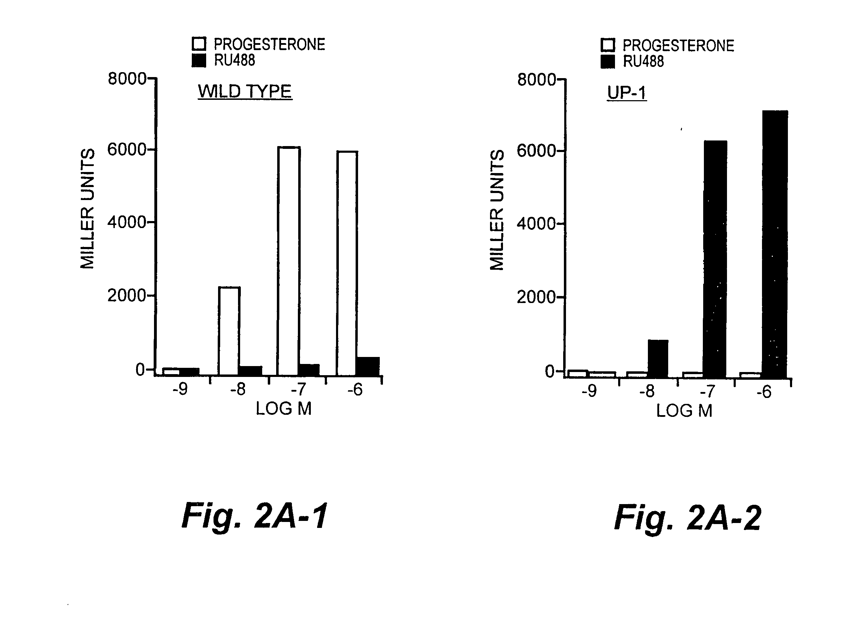

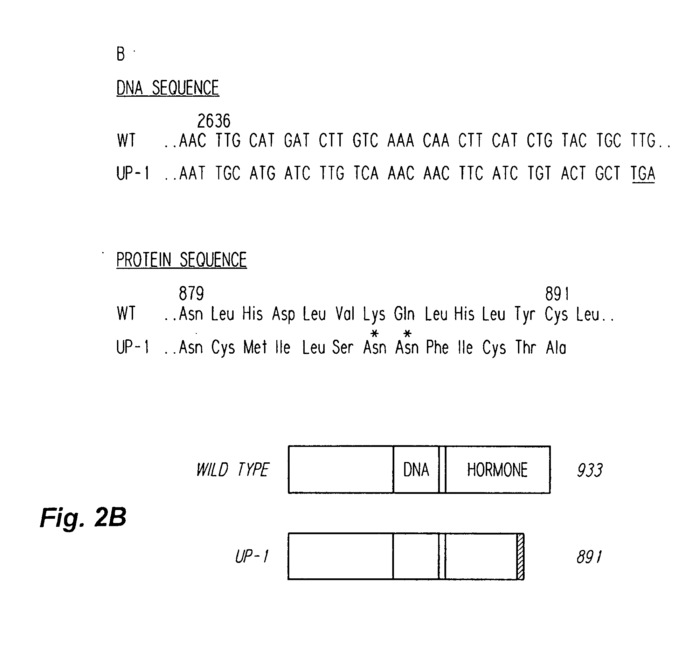

With reference to Figure 2, panel (A), when assayed with the wild type receptor, 1 µM RU486 is a weak inducer of transcription, whereas progesterone caused a greater than 60-fold induction of transcription at 1 µM. However, this situation was reversed when the mutant was analyzed. In this case, RU486 was an extremely potent activator, whereas progesterone was ineffective. Interestingly, the activity achieved by the mutant in the presence of RU486 was of the same order of magnitude as that of the wild type assayed in the presence of progesterone. This reversal in specificity clearly indicates that the mechanism by which these ligands interact with the receptor is basically different.

Figure 2 shows the DNA and amino acid sequences of the wild type and mutant DNAs. The cytosine at position 2636 was missing in the mutant DNA, therefore, a shifted reading frame was created and a stop codon was generated 36 nucleotides downstream of the C-2636 deletion. A schematic structure of the wild type and UP-1 receptors is also presented with a depiction of the 12 C-terminal amino acids unique to the mutant receptor. Conserved and structurally similar amino acids are marked by an apostrophe and asterisk, respectively.

DNA sequence analysis of UP-1 identified a single nucleotide deletion at base 2636 (Fig. 2B). This mutation results in a shift of the reading frame which generates a stop codon 36 nucleotides downstream. As a result, the wild type receptor is truncated by 54 authentic amino acids and 12 novel amino acids are added at the C-terminus.

### Example 12

### Western Analysis of the Mutant Human Progesterone Receptor

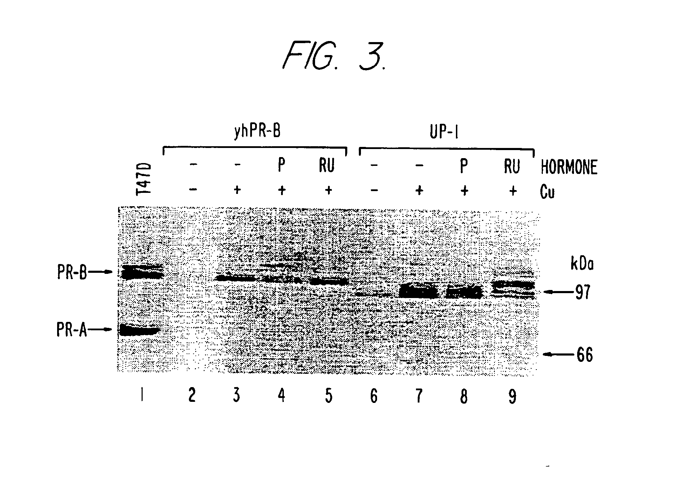

Figure 3 shows a western analysis of mutant hPR. Yeast cells carrying the reporter plasmid and wild type (yhPR-B or mutant (UP-1) hPR were grown overnight in CAA medium with (lanes 3 to 5 and 7 to 9) or without (lanes 2 and 6) 100 µM CuSO₄. 1 µM progesterone or 1 µM RU486 were added as indicated and cells were grown for another 4 hours. Yeast extracts were prepared as described above. 50 µg of protein extract were run on a 0.1% SDS-7% polyacrylamide gel. 50 µg of a T47D nuclear extract containing the A and B forms of hPR were also loaded (lane 1) as a positive control. The positions of molecular weight markers are indicated.

A Western immunoblot analysis of UP-1 and wild type receptors was performed in order to verify that the mutant receptor was synthesized as predicted from its DNA sequence and to eliminate the possibility that some major degradation products were responsible for the mutant phenotype. As shown in Fig. 3, the mutant receptor migrated faster in the gel, confirming the molecular weight predicted by DNA sequencing. The wild type receptor (yhPR-B) ran as a 114 kDa protein, while the mutant receptor was 5kDa smaller (compare lanes 2 and 3 with 6 and 7). The addition of 100µM CuSO₄ to the cell cultures increased synthesis of both the wild type and mutant hPR to the same extent. No major degradation products were detected. In the presence of progesterone and RU486, yhPR-B bands were upshifted due to hormone-induced phosphorylation of the receptor. In contrast, RU486 induced upshifting of wild type PR to a lesser extent (lanes 4 and 5). For the UP-1 mutant this hormone-dependent upshifting was seen upon treatment with RU486 (lanes 8 and 9). Thus, the C-terminus of PR may be responsible for the inactivity of RU486. Consequently, removal of this sequence would enable RU486 to become an agonist.

### Example 13

### Hormone Binding Analysis

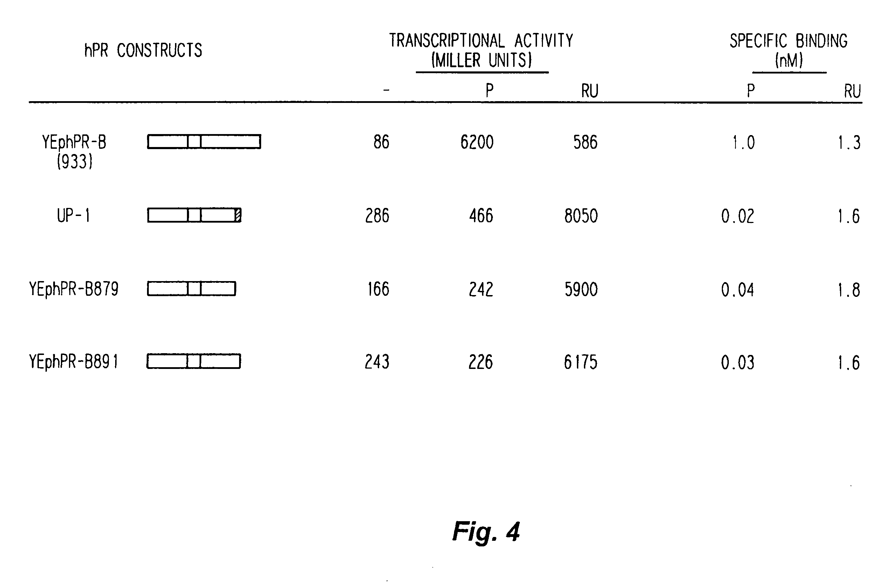

Figure 4 shows the transcriptional activity and hormone binding analysis of wild type and mutant hPR constructs. The hPR constructs are reported to the left side together with a schematic representation of the receptor molecules. Yeast cells were grown in the presence of 100 µM CuSO₄. Transcriptional analysis was done as described above. Experiments were done in triplicate and transcriptional activities were normalized with respect to protein. Hormone binding assays were performed in the presence of 20 nM [³H] progesterone or 20 nM [³H] RU486.

A saturation binding analysis of the UP-1 mutant receptor was performed in order to determine if its affinity for RU486 and progesterone was altered. Scatchard analysis of the binding data demonstrated that both the wild type and mutant receptors had a similar affinity for RU486 of 4 and 3 nM, respectively. As seen in Figure 4, the mutant receptor molecule had lost the ability to bind progesterone. Thus, the amino acid contacts for progesterone and RU486 with hPR are different.

### Example 14

### Generation of Deletion Mutants of hPR-B

As shown in Fig. 2B, DNA sequencing revealed that the frameshift mutation in the UP-1 clone created a double mutation in the receptor protein. That is, a modified C-terminal amino acid sequence and a 42 amino acid truncation. In order to identify which mutation was ultimately responsible for the observed phenotype, two new receptor mutants were constructed *in vitro:* YEphPR-B879, containing a stop codon corresponding to amino acid 880, and YEphPR-B891, containing a stop codon at amino acid 892. Hormone binding data (see Fig.4) demonstrated that both of these truncated receptors could bind RU486 but not progesterone. When examined *in vivo,* both mutant receptors activated transcription in the presence of RU486 to levels comparable to those of the mutant UP-1 generated in yeast. As expected, both mutants were inactive in the presence of progesterone. Thus, the observed phenotype was not due to second site mutations in the UP-1 molecule. Also, 12 additional amino acids, from 880 to 891, were not responsible for the mutant activity. In addition, it is clear the C-terminal 42 amino acids are required for progesterone to bind to the receptor while the last 54 amino acids are unnecessary for RU486 binding. Thus, the antagonist is contacting different amino acids in the native receptor molecule and may induce a distinct receptor conformation relative to agonists.

In addition to the above deletion mutations, other deletions in the C-terminal amino acid sequence have provided binding activity with RU486 and not with progesterone. Such deletions include: (1) a 16 amino acid deletion leaving amino acids 1-917 of the progesterone receptor; and (2) a 13 amino acid deletion leaving amino acids 1-920 of the progesterone receptor. Use of the receptor binding region with TATA-CAT expression in transient transfection assays showed CAT expression with the 16 amino acid deletion, *i.e*., amino acids 640-917, and the 13 amino acid deletion, *i.e*., amino acids 640-920.

### Example 15

### Steroid Specificity for Activation of Transcription of the UP-1 Mutant

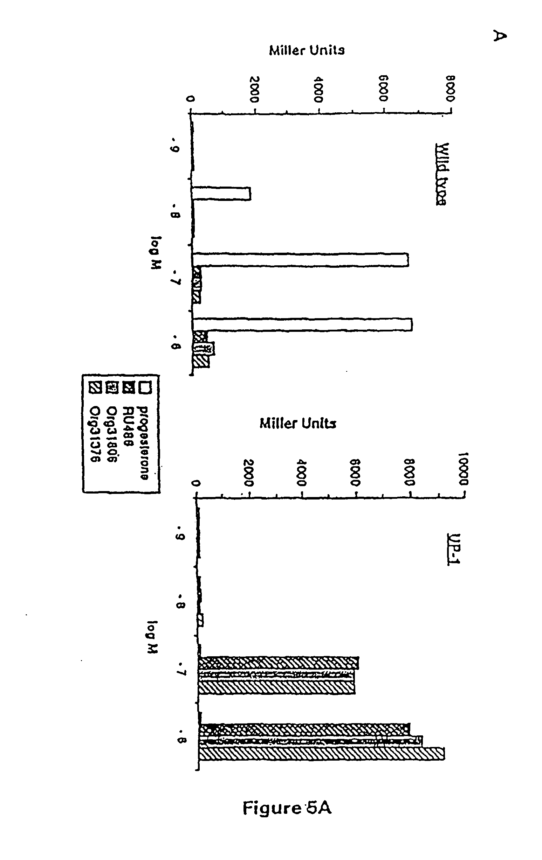

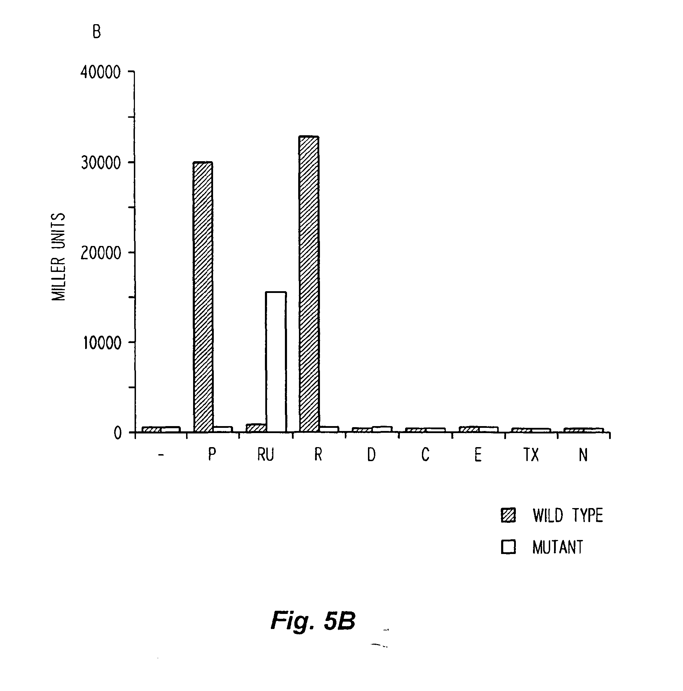

Figure 5 shows the specificity of the transcriptional activity of the mutant hPR. In panel (A), wild type and UP-1 mutant receptor transcriptional activities were assayed in the presence of different concentrations of progesterone, RU486, Org31806 and Org31376 as indicated.

A transcription assay was performed using two synthetic antagonists, Org31806 and Org31376, which are potent antiprogestins. As shown in Fig. 5A, the mutant receptor was activated by both of these compounds. The curve of the concentration-dependent activity was similar to that obtained with RU486, suggesting that the affinity of these two antagonists for the mutant receptor is similar to that of RU486. When assayed with the wild type receptor, these compounds had minimal transcriptional activity and behaved like partial agonists (3-10% of progesterone activity) only at concentrations of 1 µM, as does RU486. Thus, the inhibitory effect of the C-terminus of hPR extends to other receptor antagonists.

In panel (B), transcriptional activities of wild type and UP-1 mutant receptors were assayed in the presence of 1 µM progesterone (P), RU486 (RU), R5020 (R), dexamethasone (D), cortisol (C), estradiol (E), tamoxifen (TX) or nafoxidine (N) (see Fig. 5B). The synthetic agonist R5020 had no effect on the UP-1 mutant, suggesting that agonists, such as progesterone and R5020, require the C-terminus of the native receptor for binding and consequently fail to recognize the truncated UP-1 receptor. Other steroids known to enter yeast cells, such as estradiol, the antiestrogens tamoxifen and nafoxidine, dexamethasone and cortisol, might possibly activate the mutated receptor. All steroids tested were found to be inactive with either the wild type or mutant receptor. Thus, the activation of the mutant receptor is specific to antiprogestins.

### Example 16

### Transcriptional Activity of Mutant Receptors in Mammalian Cells

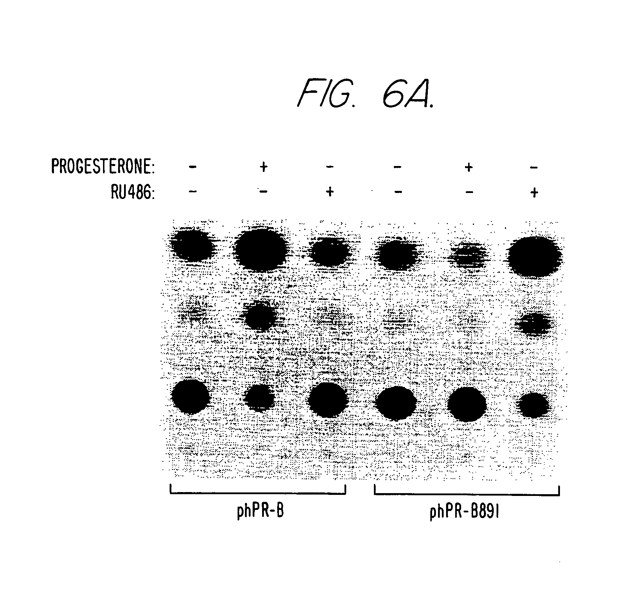

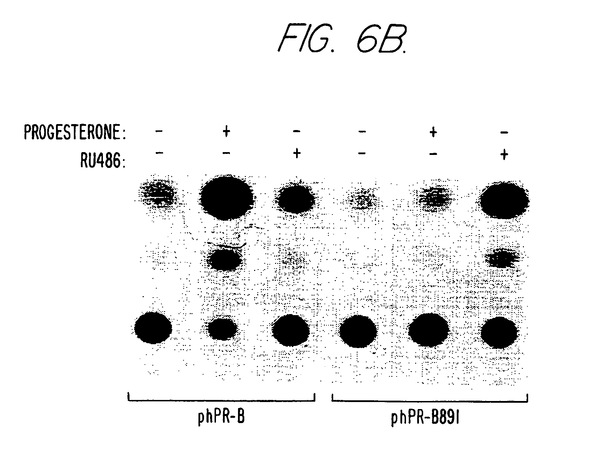

Figure 6 shows the transient transfection of mutant hPR into mammalian cells. In panel (A), HeLa cells were transiently transfected with phPR-B and pHPR-B891 receptors together with PRETKCAT receptor plasmid using the polybrene method of transfection as described (Tsai, et al. 1989). Cells were grown with or without 100 nM progesterone or RU486 for 48 hours prior to harvesting. CAT assays were performed as described above. In panel (B), CV-1 cells were transiently transfected as in (A).

With reference to Figure 6, mutant receptor activity was assayed in both human endometrial HeLa cells and monkey kidney CV-1 fibroblasts. A mutant, phPR-891, was constructed by replacing the full-length PR insert of phPR-B vector with the truncated PR cDNA of YEphPR-B891. The resulting receptor mutant, phPR-B891, is a 42 amino acid truncation of hPR-B form. Mutant 891 and wild type receptors were transfected into HeLa cells together with the PRETKCAT reporter plasmid, which contains two copies of a GRE/PRE element.

As expected, wild type PR activated transcription of the CAT gene reporter in the presence of 10⁻⁷M progesterone (Fig. 6A). Although basal transcription level was high, a 3- to 4-fold induction of transcription was detected when progesterone was added to the media. In contrast, no induction occurred in the presence of RU486. The high basal level of transcription detected in these experiments may mask or alter an RU486 effect on wild type hPR.

On the other hand, an induction of CAT activity was observed when the 891 mutant was incubated in the presence of 10⁻⁷M RU486 (Fig. 6A). The same concentration of progesterone had no activity.

Cell-type specific factors can influence the activity of the transactivating domains of steroid receptors. In order to evaluate this possibility, wild type and mutant receptors were transfected into CV-1 cells. Similar results were obtained, i.e., progesterone activated the wild type receptor while RU486 activated 891 mutant receptor (Fig. 6B).

The protein synthesized from phPR-B891 plasmid was of the correct molecular weight in mammalian cells. The mutant receptor was transfected into COSM6 cells. Western analysis on cell extracts showed that the 891 mutant was synthesized, as expected, as a protein of 109 kDa, which corresponds to a protein 42 amino acids shorter than the wild type hPR. Thus, RU486 acts as an agonist of the truncated B-receptor in a yeast reconstituted system and also in mammalian cells. The mechanism of transactivation does not require the C-terminal tail of the mutant receptor and is conserved between the three species tested.

### Example 17 (not of the invention)

### Construction of poly-glutamine stretch insertion into the LBD

The poly-glutamine stretch containing multiple repeats of CAG was constructed by a method developed by S. Rusconi (Seipel et al., Nucl. Acid Res. 21:5609-5515) utilizing multimerization of DNA fragment (BsaI and BbsI digested) coding glutamine repeats leading to poly-Qₙ. Plasmid pBluscript-KS(II) was digested with Acc65I and SacI, the linearlized vector was gel purified and ligated with the annealed oligonucleotide pair R3/R4 to create plasmid pPAP. The oligonucleotide sequence for R3 (upper strand) is: 5'-GTACGTTTAAACGCGGCGCGCCGTCGACCTGCAGAAGCTTACT AGTGGTACCCCATGGAGATCTGGATCCGAAT TCACGCGTTCTAGATTAATTAAGC-3' (Seq. ID No. 2) and the sequence for R4 (lower strand) is: 5'-GGCCGCTTAATTAATCTAGAACGCGTGAATTCGGATCCAGATCTCCATG GGGTACCACTAGTAAGCTTCTGCAGGTCGACGGCGCGCCGCGTTTAAAC-3' (Seq. ID No. 3).

The following restriction sites are incorporated into pPAP as the multiple cloning sites (from T3 to T7): PmeI, AscI, SAlI, PstI, HindIII, SpeI, Acc65I, NcoI, BglII, BamHI, EcoRI, MluI, XbaI, PacI, NotI, SacI. Oligonucleotides coding for 10 glutamines were annealed and subcloned into the BglII and BamHI site of plasmid pPAP. The sequence for the upper and lower strand oligonucleotide are, 10QU 5'-GATCTCGGTCTCCAACAGCAACAGCAACAGCAACAGCAACAG GGTCTTCTG-3' (Seq. ID No. 4) and 10QL: 5'-GATCCAGAAGACCCTG TTGCTGTTGCTGTTGCTGTTGCTGTTGGAGACCGA-3' (Seq. ID No. 5), respectively. The insert was confirmed by restriction digestion and sequencing.

The plasmid with 10Q insert (pPAP-10Q) was digested with BsaI and BbsI (New England Biolab) overnight and precipitated. One tenth of the precipitated DNA (containing both vector and fragment) was religated to create plasmid pPAP-18Q. Each ligation step results in pAP-2(n-1)Q from the previous vector pPAP-nQ. In this way various expansion of poly-Q was achieved and resulting plasmids pPAP-39Q, pPAP-66Q and pPAP132Q were created and confirmed by sequencing. The BglII and BamHI fragment (coding for poly-Q stretch) from these plasmids were purified and cloned into BglII site of pRSV-GLVP to generate GLVP with various poly-Q insert at the N-terminus. These GLVP-nQ were reinserted into the pCEP4 vector creating pCEP4-GLVP-nQ.

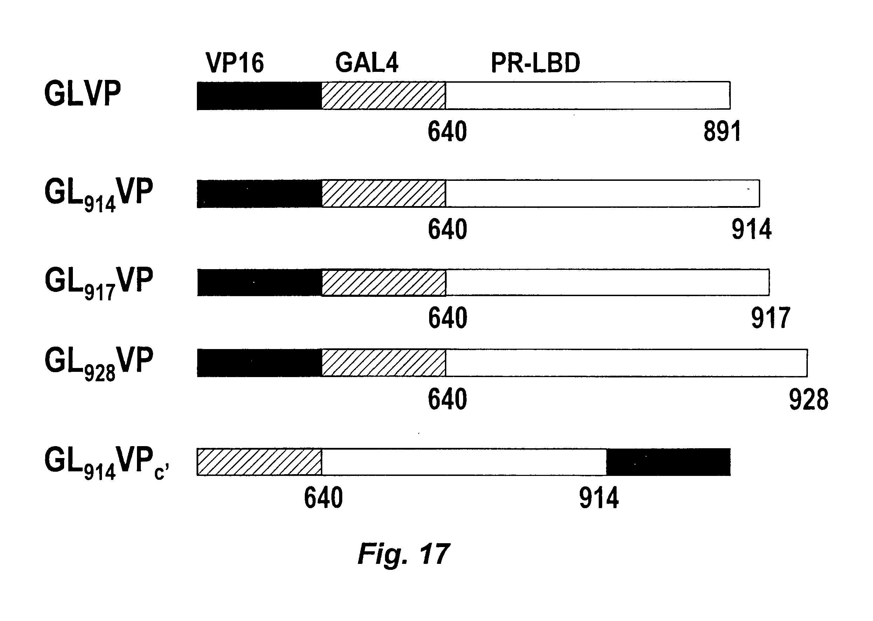

Lengthening the C-terminal ligand binding domain from 879 to 914 (Figure 17), gradually increased RU486 induced activation of target gene expression. Importantly, these mutants responded specifically to RU486, but not to the progesterone agonist R5020. Further extension of the C-terminal LBD beyond aa 914 resulted in a decrease of GLVP response to RU486.

### Construction Characterization and Analysis of Mutant Human GR-PR Fusion Protein Receptors

### Example 18 (not of the invention)

### Plasmid Construction

A mutated human Progesterone Receptor was constructed and characterized as discussed above. Mutagenesis of the ligand binding domain of the human PR was carried out under the same procedures outlined above. Characterization of the mutant progesterone receptor identified a single nucleotide deletion at base 2636. This mutation resulted in a shift of the reading frame which generates a stop codon 36 nucleotides downstream. As a result, the wild type receptor is truncated by 54 authentic amino acids and 12 novel amino acids are added at the c-terminus. The 42 amino acid truncation to the c-terminus was capable of binding RU486 and characterized as discussed above.

Plasmid DNA encoding the GR-PR fusion protein receptor and the wild type GR were constructed as follows. Each insertional mutant was digested with the restriction enzymes BamH1 and Xhol, which flanked the 3' side of the SV40 polyadenylation signal. The resulting fragments were isolated from an agarose gel. The large fragment of the insertional mutant containing the amino-terminal coding portion of the GR, i.e., the transregulatory and DNA binding region, and the bulk of the plasmid were ligated with the small fragment of another insertional mutant containing the carboxyl terminal coding sequence of the hPR deletion mutant prepared above. The resulting plasmids carrying the deletion in the hPR ligand binding domain were sequenced to ensure the integrity of the GR-PR mutant constructs.

In addition, plasmid DNA encoding a mutated rat or human GR and the wild type rat or human GR were also constructed. The plasmids for rat pGR0385 (or prCS1.C) and its wild type pGR0384 were constructed using the above methods. Details regarding construction, mutation and characterization of the above plasmid can be found in Lanz and Rusconi, Endocrinology 135:2183-2195 (1994), all of which is hereby incorporated by reference, including drawings. Characterization of the rat and human mutant GR identified a double point mutation in the ligand binding domain. In the rat construct, amino acids 770, 771, methonine and leucine, were substituted with alanine and alanine. Amino acids 780 and 781 were deleted. In the human constructs, amino acids 762 and 763 were deleted. Amino acids 752 and 753 were substituted with alanines. Both the substitution and deletion changes were at the carboxyl terminus portion of the rat or human GR ligand binding domain. The insertional mutant was digested with the restriction enzymes BamH1 and Xhol, which flank the 3' side of the SV40 polyadenylation signal and the resulting fragment was isolated from agarose gel. The large fragment of one insertional mutant containing the amino-terminal coding portion of the rat or human GR and the bulk of the plasmid were ligated with the small fragment of another insertional mutant containing the carboxy-terminal coding sequences of the mutated ligand binding domain. The resulting plasmids carrying the deletion in the ligand binding domain were sequenced to ensure the integrity of the rat or human GR mutants.

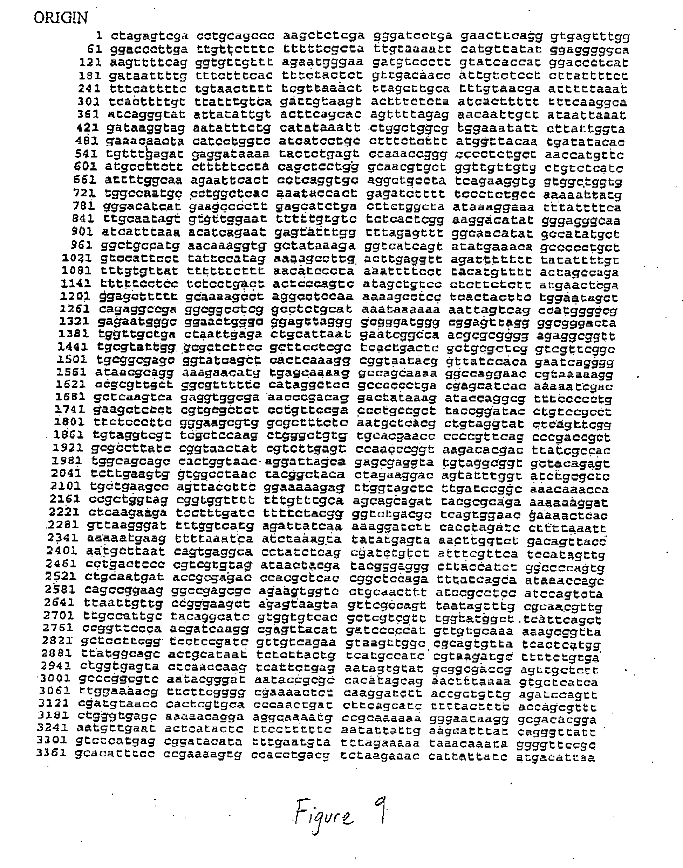

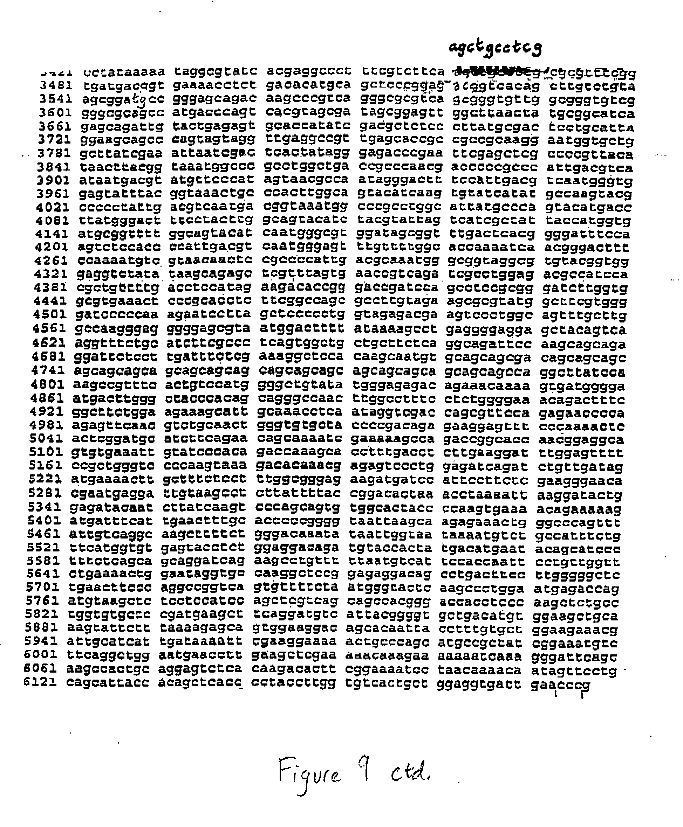

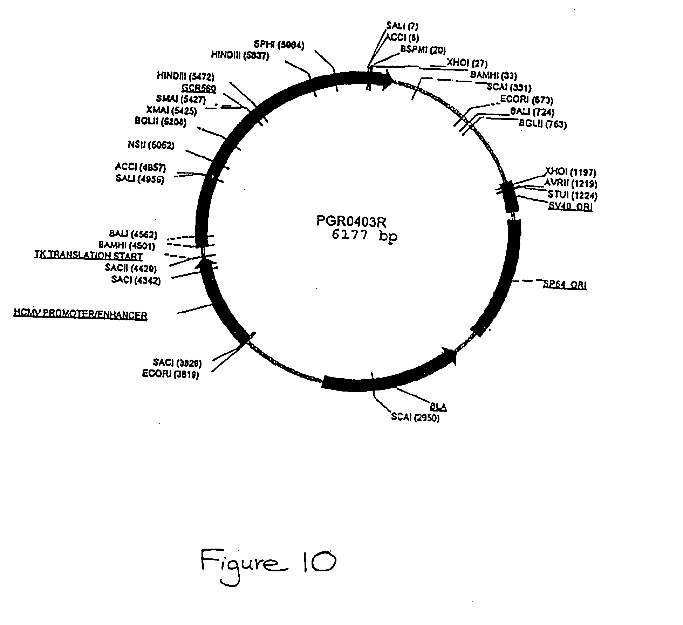

In addition, the above procedures were also used to construct plasmid DNA encoding a GR mutant with a constitutively active receptor, *i.e*., pGR0403R (Figures 9 and 10). The insertional mutant was digested with the appropriate restriction enzyme. The resulting fragments were isolated from agarose gel. The large fragment of the insertional mutant containing the amino-terminal coding portion of the GR, *i.e*., the transregulatory domains and DNA binding domains, and the bulk of the plasmid were ligated with the small fragment of another insertional mutant containing the mutated GR ligand binding domain. The resulting plasmid was sequenced (Figure 9) to ensure integrity of the mutant construct.

### Example 19

### Cell Culture, Transfection and Assay of CAT and Luciferase Activities

CV-1 cells were maintained at 37°C in Dulbecco modified Eagle medium containing 10% fetal bovine serum ("FBS") in a humidified atmosphere containing 5% CO₂. Cells were transfected using the commercially available cationic agent lipofectamine. Briefly, DNA was mixed with the lipofectamine reagent and added to cells. After 5 hours, the DNA mix was removed and replaced with growth medium containing 10% FBS and cells were returned to an atmosphere containing 5% CO2. Eighteen hours later, cells were treated with steroids at various concentrations for approximately 24 hours, then harvested.

In this method, the CV1 cells are transformed with either the wild-type receptor or the mutant receptor and a glucocorticoid responsive reporter construct. To measure transcriptional activation, a CAT reporter containing two synthetic GRE's and a TATA box was used. To measure transcriptional repression, two constructs were used. The first contains two copies of the binding site for the inflammation-inducible transcription factor AP-1, following by the thymidine kinase (tk) promoter, linked to CAT. The second contains two copies of the binding site for the inflammation-inducible transcription factor NF_{K}-B, followed by a TATA box, linked to the luciferase gene. CAT expression was quantified using an ELISA assay following the manufacturer's recommended procedure. Luciferase activity was measured using a commercially-available luciferase assay following the manufacturer's recommended procedure.

### Example 20 (not of the invention)

### In vitro Transfections Using CV1 Cells

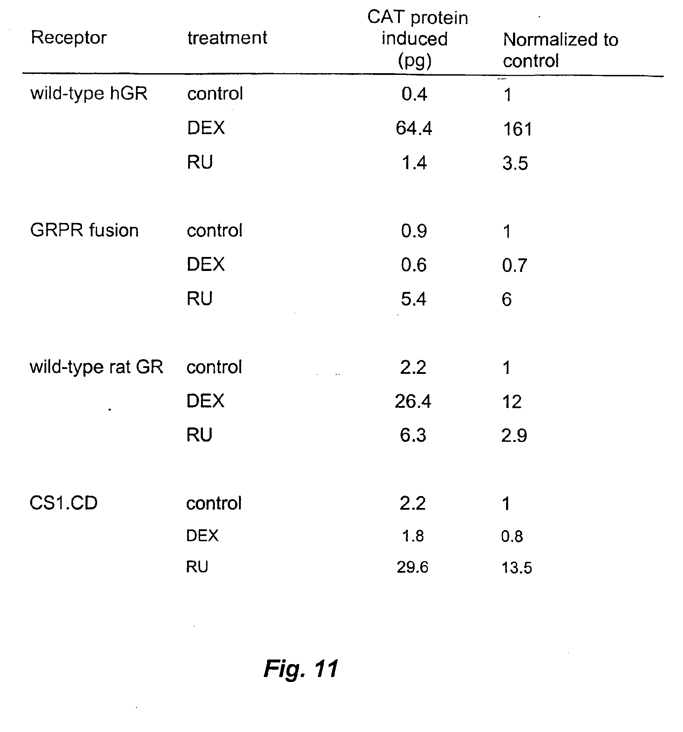

The GR-PR fusion protein receptor and the mutant rat GR were tested for biological activity through in vitro transfection into CV1 cells. As controls vectors expressing the wild type human GR and the wild type rat GR were used. Results from these experiments demonstrate that the wild type human and wild type rat GR are transcriptionally activated in response to dexamethasone and minimally by RU486. In contrast, the mutant rat GR (CS1.CD) is transcriptionally activated by RU486 and not by dexamethasone. Similarly, the GR-PR fusion protein receptor is also activated by RU486 and not by dexamethasone. Figure 11 illustrates the amount of CAT protein produced in response to the particular ligand.

### Example 21 (not of the invention)

### In vitro Transcriptional Repression Studies

The transcriptional repression mediated by the mutant rat GR and human GR-PR construct were examined. The amount of CAT protein produced under the transcriptional control of synthetic activation elements was determined.

Specifically two reporters were examined TRE2tkCAT, which contains AP-1 fused to the thymidine kinase promoter linked to CAT. The second reporter used was NF_{K}-B-luc plasmid, which contains 2 NF_{K}-B binding sites fused to luciferase. These promoters contain inflammation-inducible promoters, and were used to evaluate the ability of the wild-type and mutant GR constructs to repress transcription.

Cells were transfected into CV1 cells along with either the wild type rat or human GR or the mutant rat (CS1.CD) or human GR. Cells pretreated with dex or RU486 to allow binding to the steroid receptor, were then stimulated with phorbol ester TPA to activate AP-1 and NF_{K}-B. Companion cells were not stimulated with TPA, and control cells also received neither dex nor RU486.

The results demonstrate that RU486 treatment resulted in a decrease in the level of CAT protein and luciferase activity in CSI.CD transfected cells. Dex treatment had no effect on CAT levels or luciferase. These results were not expected since dex does not bind to the ligand binding domain of the mutant rat GR CSI.CD or human GR. In cells transfected with the wild type GR both dex and RU486 caused a decrease in the level of CAT protein and luciferase activity. Such results are not unexpected because the wild type GR binds both dex and RU486.

RU486 acts through the mutated GR to repress transcription of AP1 driven genes. Since AP-1 and NF_{K}-B drive expression of pro-inflammatory genes, and RU486 acts through mutant or represses transcription of the AP-1 and NF_{K}-B driven genes, there was mediation of the anti-inflammation.

### Example 22 (not of the invention)

### Mutant GR Expression and Detection

Three antibodies were obtained and used to recognize recombinant partially purified GR in a Western blot analysis. Studies were performed to detect wild type GR and mutant GR protein from transfected cells or GR from rat synovial tissue using the above antibodies.

The antibodies also were able to detect human GR obtained from HeLa cell extracts. Significant levels of GR were detected with as low as 200ug of whole cell extract. Immunoreactivity was also detected with synovial tissue, and antibodies are being prepared to distinguish between wild type and mutant GR proteins.

### Example 23 (not of the invention)

### Transactivation and Transrepression Studies

In addition to the experiments above, the vector with NF_{K}-B binding sites fused to the luciferase gene, was injected into synovial joints in rats and treated with and without TNF-α. TNF-α is a cytokine which induces inflammation and promotes NF_{K}-B binding to its appropriate DNA sequences. With the DNA construct, TNF-α treatment results in an increase in transcription of TNF-α and exogenously-introduced luciferase gene. No luciferase activity in synovial tissue is detected without plasmid transfection. Also, there is no luciferase activity in synovial tissue injected with plasmid in the absence of TNF. A six-fold increase in the level of luciferase occurred when tissue was exposed to 0.1 or 1nm TNF. This serves as an easily detectable in vivo marker for wild-type or mutant GR function.

### Construction, Characterization and Analysis of Double Point Mutations in the Ligand Binding Domain of GR

### Example 24 (not of the invention)

### Mutagenesis of the ligand binding domain of human GR

A plasmid was constructed containing the human GR cDNA with amino acids 752 and 753 changed to alanines and amino acids 762 and 763 deleted. This plasmid, pSTC-hGR-CS1/CD, was constructed as follows. The wild type glucocorticoid hormone receptor plasmid was digested with the restriction enzymes NsiI and XbaI, which flank the region to be mutated. The resulting fragments were isolated from agarose gel. The smaller fragment was digested with the restriction enzymes EcoRI and SspI, generating three fragments. The fragments were isolated from an agarose gel.

A synthetic fragment was synthesized: 5'-AAT TCC CCG AGG CGG CAG CTG AAA TCA TCA CCA ATC AGA TCT-3' (Seq. ID No. 6) to replace the EcoRI-SspI fragment. The larger plasmid fragment, the NsiI-EcoRI fragment, the SspI-XbaI fragment and the synthetic EcoRI-SspI fragment were ligated together. The resulting plasmid carries the substitution and deletion as described above.

### Example 25 (not of the invention)

### Characterization of GR Mutants in the Ligand Binding Domain

To ensure the integrity of the mutation, the plasmid containing the mutant human GR was sequenced. Further experiments, as discussed above, were done to characterize the mutant human GR. Western analysis and hormone binding as discussed above were performed to ensure character of the constructs, *e.g*., cell expression of the protein and steroid specificity for activation or repression of transcription.

### Example 26 (not of the invention)

### Transcriptional Activity of the Mutant Receptors in Mammalian Cells

LMTK⁻ cells were maintained at 37°C in Bulbecco's modified Eagle's medium containing 10% fetal Bovine serum ("FBS") in a humidified atmosphere containing 5% CO2. Cells were transfected with the polybrene method described in Kawai et al., Mol. Cell. Bio. 4:91-1172 (1984), hereby incorporated by reference, including drawings. After a 25% glycerol shock in Hank's buffered saline solution ("HBSS"), the cells were washed twice with HBSS and medium was added containing hormones or solvent. The cells were cultured for 48 hours. Extracts were made by freeze-thawing. CAT activity was assayed with 25 *µ*g protein and an incubation time of 16 hours. CAT activity assayed as described by Seed et al., Gene 67:271 (1988), hereby incorporated by reference, including drawings.

### Construction Characterization and Analysis of Constitutively Active Mutant GR

### Example 27 (not of the invention)

### Mutagenesis of the ligand binding domain of human GR

Deletion of the steroid ligand binding domain was prepared as follows. This deletion removed a large portion of the carboxyl-terminal portion of the protein eliminating all steroid binding properties. Using the procedures discussed above, the pGR0403R plasmid (Figures 9 and 10) was constructed. This mutation gave rise to a constitutively active receptor. This mutant was able to activate transcription of the CAT reporter gene in the presence or absence of glucocorticoid hormone. In addition, this mutant is also able to repress transcription of the NF_{K}-B-luciferase construct.

### Example 28 (not of the invention)

### Characterization of GR Mutants in the Ligand Binding Domain

To ensure the integrity of the mutation, the plasmid containing the mutant human GR, pGR0403R (Figure 10) was sequenced (Figure 9). Further experiments, as discussed above, were done to characterize the mutant human GR. Western analysis and hormone binding as discussed above were performed to ensure character of the constructs, *e.g.,* cell expression of the protein, lack of steroid specificity for activation or repression of transcription and base level of gene expression as compared to constitutive expression.

### Example 29 (not of the invention)

### Transcriptional Activity of the Mutant Receptors in Mammalian Cells

The constitutively active mutant GR construct was prepared as discussed above. The receptor has no ligand binding domain and, when expressed in cells, represses transcription of AP-1 driven genes in the absence of dex or RU486. In vitro testing shows that the constitutively active GR mutant when transfected constitutively activates promoters with glucocorticoid responsive elements and represses AP-1 containing promoters.

### Construction, Characterization and Analysis of Mutations in the DNA Binding or Transregulatory Domains of GR

### Example 30 (not of the invention)

### Mutagenesis of the DNA Binding or Transregulatory Domains of GR

For obtaining transactivation activity without transrepression activity the following construct was made. The mutated ligand binding domain is mutated as described above. Procedure details from Lanz, et al., Endocrinology 135:2183-2195 (1994) are hereby incorporated by reference, including drawings. The mutated DNA binding domain is mutated by changing the serine at position 425 to glycine, the leucine at position 436 to valine and the tyrosine and asparagine at positions 478 and 479 to leucine and glycine.

For obtaining transrepression activity without transactivation, the following construct was made. The mutated ligand binding domain is mutated as described above. The mutated transregulatory domain is mutated by changing the alanine at position 458 to threonine, the asparagine and alanine at positions 454 and 458 to aspartic acid and threonine, respectively, and the arginine and aspartic acid at positions 460 and 562 to aspartic and cysteine, respectively.

### Example 31 (not of the invention)

### Characterization of GR Mutants in the DNA Binding or Transregulatory Domains

To ensure the integrity of the mutation, the plasmids containing the mutant GR were sequenced. Further experiments, as discussed above, were done to characterize the mutant GR constructs. Western analysis and hormone binding as discussed above were performed to ensure character of the constructs, *e.g.*, protein expression in cells and steroid specificity for activation or repression of transcription.

### Example 32 (not of the invention)

### Transcriptional Activity of the Mutant Receptors in Mammalian Cells

The above mutant GR constructs were prepared. The two different receptor constructs have either a mutated DNA binding domain or a mutated transregulatory domain. When expressed in cells, the transrepression only construct with a DNA binding domain mutation represses transcription of AP-1 and NF_{K}-B driven genes in the presence of dex or RU486. No activation of transcription was observed. *In vitro* testing shows that the GR mutant when transfected represses AP-1 and NF_{K}-B containing promoters and does not activate the glucocorticoid responsive genes.

As for the transactivation only construct with a mutated transregulatory domain, activation of transcription was observed in the presence of various steroids. In the presence of dex or RU486 no transrepression of AP-1 or NF_{K}-B driven genes was detected. *In vitro* testing shows that the GR mutant when transfected activates glucocorticoid responsive genes in response to ligand stimulation but no repression of AP-1 or NF_{K}-B genes was observed.

### Example 33

### Chicken, Rat and Mammalian Progesterone Receptors

Chicken, rat and mammalian progesterone receptors are readily available and function by binding to the same DNA regulatory sequence. Chicken and rat progesterone receptors, however, bind a different spectrum of ligands, possessing affinities different from those interacting with human progesterone receptor. Thus, the chicken and rat progesterone receptor can be used as a transgene regulator in humans. Further, it can be used to screen for specific ligands which activate chicken or rat progesterone receptor but not endogenous human progesterone receptor. An example of a ligand is 5α-pregnane-3,20-dione (dihydroprogesterone) which binds extremely well to chicken and rat progesterone receptor but does not bind or binds very poorly to human progesterone receptor.

Although the unmodified chicken or rat progesterone receptors are already endowed with a different spectrum of ligand binding affinities from the human or other mammals and can be used in its native form, it is important to try to select additional mutated progesterone receptor to create a more efficacious receptor. The differences in chicken, rat and human progesterone receptors are due to a few amino acid differences. Thus, other mutations could be artificially introduced. These mutations would enhance the receptor differences. Screening receptor mutations for ligand efficacy produces a variety of receptors in which alterations of affinity occur. The initial screening of progesterone mutants was carried out using intermediate levels of ligands. One mutant had lost progesterone affinity entirely, but bound a synthetic ligand RU486 with nearly wild-type efficiency. RU486 is normally considered an antagonist of progesterone function, but had become an agonist when tested using this specific mutant. Because the ligand is synthetic, it does not represent a compound likely to be found in humans or animals to be treated with gene therapy. Although RU486 works as an agonist in this case, it is not ideal because of its potential side effects as an anti-glucocorticoid. Further, it also binds to the wild-type human progesterone. Thus, it has the undesirable side effect of reproductive and endocrine disfunction.

This approach is not limited to the progesterone receptor, since it is believed that all ligand activated transcription factors act through similar mechanisms. One skilled in the art recognizes that similar screening of other members of the steroid superfamily will provide a variety of molecular switches. For example, the compound 1,25-dihydroxy-Vitamin D₃ activates the Vitamin D receptor but the compound 24,25-dihydroxy-Vitamin D does not. Mutants of the Vitamin D receptor can be produced which are transcriptionally activated when bound to 24,25-dihydroxy-Vitamin D, but not by 1,25-Vitamin D₃.

One skilled in the art recognizes that the ligands are designed to be physiologically tolerated, easily cleared, non-toxic and have specific effects upon the transgene system rather than the entire organism.

### Example 34

### Transgenic Animals

A modified glucocorticoid receptor can be used in the production of non-human transgenic animals. A variety of procedures are known for making transgenic animals, including that described in Leder and Stewart, U.S. Patent No. 4,736,866 issued April 12, 1988, and Palmiter and Bannister, Annual Review of Genetics, 20:465-499. For example, the mutated glucocorticoid receptors described above can be combined with the nucleic acid cassette containing the recombinant gene to be expressed. For example, lactoferrin can be placed under the control of a basal promoter, such as thymidine kinase promoter with adjacent glucocorticoid responsive elements. This vector is introduced into the animal germ lines, along with the vector constitutively expressing the mutant glucocorticoid receptor. The two vectors can also be combined into one vector. The expression of the recombinant gene in the transgenic animal is turned on or off by administering a pharmacological dose of RU 38486 to the transgenic animal. This hormone serves to specifically activate transcription of the transgene. The dose can be adjusted to regulate the level of expression. One skilled in the art will readily recognize that this protocol can be used for a variety of genes and, thus, it is useful in the regulation of temporal expression of any given gene product in transgenic animals.

### Location of Transregulatory Domains at the C-Terminal

### Example 35

### Chimeric Fusion Protein with Various C-terminus Deletion

To construct GLVP chimeras with various C-terminal deletions of the human progesterone receptor ligand binding domain, the HindIII to BamHI fragment containing these various deletions in pRSV-hPR plasmids (Xu et al. (1996) (unpublished)) was gel purified with QIAEX II gel extraction kit (Qiagen). The purified fragments were subcloned into-HindIII and BamHI sites of pRSV-GLVP (Wang et al., Proc. Natl. Acad. Sci. 91:8180-8184 (1994)) replacing the amino acid region 610 to 891 of the GLVP.

### Example 36

### GLVP_{c'} Chimeras with VP16 Activation at the C-terminus

Two-step clonings were used to move VP16 activation to the C-terminus of the chimeric fusion protein. First, the hPR -LBD region (from amino acid 800 to various C-terminus) was amplified using 5' primer (5'-TATGCCTTACCA TGTGGC-3' (Seq. ID No. 7)) with a different 3' primer as a pair and digested with HindIII to SalI to prepare the fragment for ligation. For a different position of amino acid truncation, the 3' primers incorporating the SalI site are: P3S-879: 5'-TTGGTCGACAAGATCATGCATTATC-3' (Seq. ID No. 9); P3S-891: 5'-TTGTCGACCCGCAGTACAGATGAAGTTG-3' (Seq. ID No. 10) and P3S-914: 5'-TTGGTCGACCCAGCAATAACTTCA GACATC-3'. The DNA fragment containing the VP16 activation domain (amino acid 411-490) was isolated from pMSV-VP16-Δ3'-β58N' with SalI and BamHI.

The digested PCR fragment and VP16 activation were ligated together into the HindIII and BamHI sites of expression vector pCEP4 (Invitrogen). The ligated vector pCEP4-PV (LBD 810-879 and VP16), -C3 (LBD 810-891 and VP16), -C2 (LBD 810-914 and VP16), respectively, now contain C-terminal fragments of hPR-LBD from the HindIII site (amino 810) to various truncations of LBD fused 3' to VP16 activation domain with BamHI after the termination codon of VP16. The HindIII-BamHI fragment from pGL (in pAB vector) was then replaced with PV, C3, and C2 fragment, respectively, to yield pGL₈₇₉VP_{C',} pG₈₉₁VP_{C'}, and pGL₉₁₄VP_{C'}. These chimeric fusion proteins were then subcloned into Acc65I and BamHI sites of pCEP4 expression and were named as pCEP4-GL₈₇₉VP_{C'}, pCEP4-GI₈₉₁VP_{C'}, pCEP4-GL₉₁₄VP_{C'} (Fig. 17).

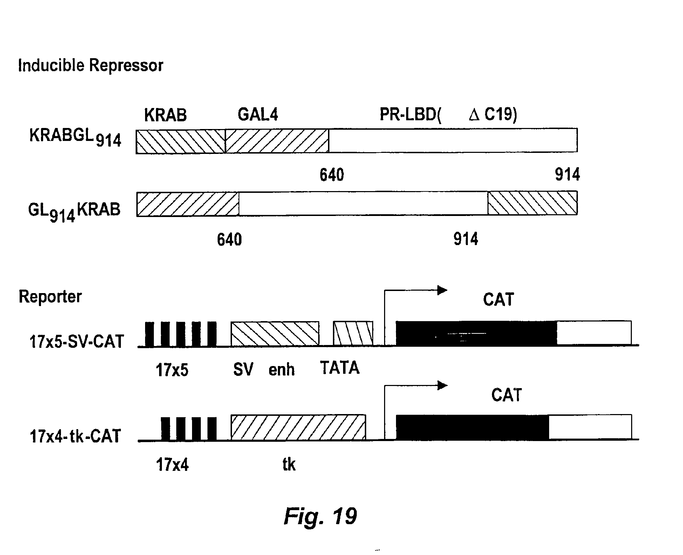

The regulator with a C-terminally located VP16 is more potent than its N-terminal counterpart (Fig. 18). In addition, extension of the C-terminal LBD from amino acid 879 to amino acid 914 further increased transactivational activity of the regulator in this C-terminally located VP16 chimera. Thus, extension of the LBD to amino acid 914 further enhances the RU486-dependent transactivation, irrespective of whether VP16 is located in the N- or C-terminus, suggesting the existence of a weak dimerization and activation function between amino acid 879 and 914 of the PR-LBD. By transferring the VP16 activation domain from the N-terminus to the C-terminus, a much more potent transactivator GL₉₁₄VP_{C'} was generated.

The modified GL₉₁₄VP_{C'} is not only more potent but also activates the reporter gene at a lower concentration of ligand as compared to GL₉₁₄VP where VP16 is located at the N-terminus. GL₉₁₄VP activity occurred at an RU486 concentration of 0.1 nM and reached a maximal level at 1 nM. In contrast, GL₉₁₄VP_{C'}, increased reporter gene expression at an RU486 concentration 10 fold lower (0.01 nM) than that of GL₉₁₄VP. This newly discovered character of GL₉₁₄VP_{C'} is important for its use in inducible target gene expression, since it would allow use of a concentration which has no anti-progesterone or anti-glucocorticoid activity. This represents a significant advantage when the inducible system is applied in *in vivo* situations, as exemplified by transgenic mice and gene therapy.

### Example 37

### Inducible Repressor Containing the Kid-1 KRAB domain

The Kid-1 gene containing the KRAB domain (aa. 1-70) was amplified with 2 sets of primers for insertion into the N- or C-terminus of GL₉₁₄, respectively. For the KRAB domain to be inserted at the N-terminus of the fusion protein, the Kid-1 cDNA was amplified with the set of primers as follows: Kid3: 5'-CGACAGATCTGGCTCCTGAGCAAAGA GAA-3' (Seq. ID No. 11), Kid4: 5'-CCAGGGATCCTCTCCTTGCTG CAA-3' (Seq. ID No. 12). The PCR products were digested with BglII and BamHI and subcloned into pRSV-GL₈₉₁ to create pRSV-KRABGL₈₉₁. The KpnI-SalI fragment of KRABGL ₈₉₁ was then purified and subcloned into KpnI-SalI sites in pRSV-GL₉₁₄VP to create pRSV-KRABGL₉₁₄. The entire KRABGL₉₁₄ fragment (KpnI-BamHI) was then inserted into the KpnI and BamHI digested pCEP4 generating pCEP4-KRABGL₉₁₄ (Fig. 19).

For C-terminally located KRAB domain, the Kid-1 gene was amplified with the following set of primers: Kid1: 5'-TCTAGTCGACGATGGCTCCTGAGCAAAGAGAAG-3' (Seq. ID No. 13), Kid2: 5'-CCAGGGATCCTATCCTTGCTGCAACAG (Seq. ID No. 14). The primer Kid2 also contains a termination codon (TAG) after aa. 70. The PCR products were digested with SalI and BamHI and purified using QIAEX II gel extraction kit (Qiagen). The HindIII and SalI fragment (317 bp) from pBS-GL₉₁₄VP_{C'}, was isolated as is the vector fragment of pCEP4-GL₉₁₄VP_{C'} digested with HindIII and BamHI. These three piece fragments were ligated to create pCEP4-GL₉₁₄KRAB.

The chimeric regulator GL₉₁₉KRAB, with the KRAB repression domain inserted in the C-terminus, strongly repressed expression (6-8 fold) of both reporters in an RU486-dependent manner. However, the N-terminally located KRAB repression domain (KRABGL₉₁₄) did not repress target gene expression in the presence of RU486 to the degree of that achieved with KRAB located in the C-terminus (GL₉₁₄KRAB) .

### Example 38

### Transient Transfection, CAT Assay, hGH assay and Western Blot

HeLa and CV1 cells were transfected with the described amount of DNA using the polybrene mediated Ca₂PO₄ precipitation method and CAT assay was performed and quantified as described above (Wang et al., Proc. Natl. Acad. Sci. 91:8180-8184 (1994)). HepG2 cells (10⁶) were grown in DMEM with 10% fetal bovine serum and 1X Penicillin-Streptomycin-Glutamine (Gibco BRL) and transfected with polybrene mediated Ca₂PO₄ precipitation method. Aliquots of the cell culture media were taken at different time intervals and hGH production was measured using the hGH clinical assay kit (Nichols Institute) according to the manufacture's instruction. For Western blot analysis, protein extracts (20 µg) were prepared from transiently transfected HeLa cells, separated on SDS polyacrylamide gel and trans-blotted onto nylon membrane as described above. The blot was probed with anti-GAL4-DBD (aa. 1-147) monoclonal antibody (Clontech) and developed with an ECL kit (Amersham).

These analyses confirmed that the two regulator proteins are expressed at a similar level. Together, these results suggest that through modification of the PR-LBD within the chimeric regulator we could further improve its response to a ligand by at least one order of magnitude.

### Example 39

### Stable Cell Line Generation and Neurite Outgrowth Assay

To demonstrate the use of the inducible system in a biological situation, a regulatable expression model for nerve growth factor (NGF) was designed. NGF has been shown to stimulate neurite (axon) outgrowth of PC12 cells (from rat adrenal pheochromocytoma) when added to the cell culture media (Greene et al., Proc. Natl. Acad. Sci. 73:2429-2928 (1976)).

Rat FR cells, derived from rat fetal skin cells (American Type Culture Collection, CRL 1213) were transfected with pCEP4-GLVP₉₁₄VP_{C'} by the ₂Ca₄PO method as described previously (Wang et al., Proc. Natl. Acad. Sci. 91:8180-8184 (1994)). Cells were grown in DMEM with 10% fetal bovine serum and selected with 50 µg/ml hygromycin-B (Boehringer Mannheim). After 2-3 weeks colonies were picked and subsequently expanded. Each clone was then transiently transfected with 2 µg of the p17X4-TATA-CAT plasmid utilizing Lipofectin (GIBCO-BRL). Twenty-four hours later, the cells were treated with either RU486 (10⁻⁸M) or 80% ethanol vehicle. Cells were harvested 48 hours later and CAT activity was measured using 50 µg of cell extracts. Clones showing RU486 inducible CAT activity were subsequently transfected with the vector p17X4-TATA-rNGF(Neo).

Stable cells containing both genes were selected with hygromycin (50 µg/ml) and G418 (100 µg/ml) for 2-3 weeks and subsequently expanded. Each colony was then seeded into a 10 cm culture dish and treated with 10⁻⁸M RU486 or vehicle control (80% ethanol). After 48 hours, the conditioned media was collected and frozen. Subsequently, the conditioned media was thawed and diluted two-fold in DMEM with 10% horse serum and 5% fetal bovine serum. The diluted conditioned media was then placed on PC12 cells, with new diluted conditioned media added every two days After 5-7 days, PC12 cells were observed for neurite outgrowth.

When conditioned media (from C4FRNGF cells treated with RU486) was added to PC12 cells, strong neurite outgrowth from PC12 cells was observed after 48 hrs of incubation. Little if any neurite outgrowth was observed in PC12 cells incubated with the conditioned media that was collected from stable cells treated with vehicle only (85% ethanol). These results demonstrate that the inducible system can be used to control various biological phenomenon.

### Mutated Glucocorticoid Receptors as Gene Switch (not of the invention)

In addition to the above methods, the mutated glucocorticoid receptors can be used as gene switches. The above constructs can be used to express a co-transfected target therapeutic gene using a glucocorticoid response element ("GRE") containing promoter. The GRE promoter will drive, activate or transactivate expression of the therapeutic gene upon activation of the ligand binding domain of the constructs of the present invention. The therapeutic protein can be a secreted protein, e.g., an anti-inflammatory cytokine. Such methods allow more global effect on the transfected tissue.

One skilled in the art would readily appreciate that the present invention is well adapted to carry out the objects and obtain the ends and advantages mentioned, as well as those inherent therein. The mutated steroid receptors along with the methods, procedures, treatments, molecules, specific compounds described herein are presently representative of preferred embodiments are exemplary and are not intended as limitations on the scope of the invention. Changes therein and other uses will occur to those skilled in the art.

It will be readily apparent to one skilled in the art that varying substitutions and modifications may be made to the invention disclosed herein.

## Claims

1. A modified steroid hormone receptor protein, wherein said receptor protein responds to a conventional antagonist of a wild-type progesterone receptor protein counterpart with an agonistic response, and wherein said modified receptor protein comprises a DNA binding domain, a transregulatory domain, and a mutated progesterone receptor ligand binding region, **characterized in that** the transregulatory domain is heterologous to the ligand binding domain and is located in a C-terminal position in said modified steroid hormone receptor protein.

2. The modified steroid hormone receptor protein of claim 1, wherein the progesterone receptor ligand binding region is mutated by an alteration of a primary sequence in about 13 to 42 carboxyl terminal amino acids of a wild-type progesterone receptor ligand binding domain.

3. The modified steroid hormone receptor protein of claim 2, wherein the progesterone receptor ligand binding region consists essentially of amino acids selected from the group consisting of 640 through 914, 640 through 917 or 640 through 920 of a progesterone receptor ligand binding domain.

4. The modified steroid hormone receptor protein of any of the preceding claims, wherein said transregulatory domain is capable of transactivating or transrepressing gene expression.

5. The modified steroid hormone receptor protein of any of the preceding claims, wherein the DNA binding domain is replaced with a DNA binding domain that is not associated normally with the progesterone ligand binding domain.

6. The modified steroid hormone receptor protein of any of the preceding claims, wherein the DNA binding domain is a GAL-4 DNA binding domain.

7. The modified steroid hormone receptor protein of any of the preceding claims, wherein the transregulatory domain is a transactivation domain.

8. The modified steroid hormone receptor protein of any of the preceding claims, wherein the transregulatory domain is a transrepression domain.

9. The modified steroid hormone receptor protein of any of the preceding claims, wherein said transregulatory domain comprises a Krüppel-associated box-A (KRAB) transrepressing domain.

10. The modified steroid hormone receptor protein of claim 9, wherein the KRAB transrepression domain is a Kid-1 or a Koxl KRAB transrepression domain.

11. The modified steroid hormone receptor protein of claim 10, wherein the KRAB transrepression domain is a Kid-1 KRAB transrepression domain.

12. The modified steroid hormone receptor protein of any one of the preceding claims, wherein the mutated progesterone receptor ligand binding domain binds a synthetic antagonist selected from the group consisting of: RU486, Org31806 and Org 31376.

13. The modified steroid hormone receptor protein of claim 12 wherein said mutated progesterone receptor ligand binding region is capable of responding to RU486 at a concentration as low as 0.01 nM.

14. The modified steroid hormone receptor protein of any of the preceding claims, wherein the receptor protein comprises in order from N-terminus to C-terminus, the DNA binding domain, the mutated progesterone receptor ligand binding region, and the transregulatory domain.

15. A nucleic acid sequence encoding a modified steroid hormone receptor protein of any one of the preceding claims.

16. The nucleic acid sequence of claim 15, further comprising a tissue specific promoter.

17. A vector containing the nucleic acid sequence of claim 15 or claim 16.

18. A cell transfected or transformed with a vector of claim 17.

19. An in vitro method of making a transformed cell comprising the step of contacting said cell with a vector of claim 17 for sufficient time to transform said cell, wherein said transformed cell expresses a modified steroid hormone receptor protein encoded by said vector.

20. The use of the vector of claim 17, in the manufacture of a medicament for delivering the nucleic acid sequence encoding the modified steroid hormone receptor protein to a mammal in *vivo.*

21. The use of the vector of claim 17, in the manufacture of a medicament for delivering the nucleic acid sequence encoding the modified steroid hormone receptor protein to a mammal in *vivo* for regulating expression of neurite growth factor.

22. A transgenic non-human animal whose cells contain the nucleic acid of claim 15 or claim 16, or the vector of claim 17.

## Patentansprüche

1. Modifiziertes Steroidhormonrezeptorprotein, wobei das Rezeptorprotein auf einen herkömmlichen Antagonisten eines Wildtyp-Progesteronrezeptorprotein-Gegenstücks mit einer agonistischen Reaktion reagiert und wobei das modifizierte Rezeptorprotein eine DNA-Bindungsdomäne, eine Transregulationsdomäne und eine mutierte Progesteronrezeptor-Ligandenbindungsregion umfasst, **dadurch gekennzeichnet, dass** die Transregulationsdomäne zu der Ligandenbindungsregion heterolog ist und sich in einer C-terminalen Position in dem modifizierten Steroidhormonrezeptorprotein befindet.

2. Modifiziertes Steroidhormonrezeptorprotein nach Anspruch 1, wobei die Progesteronrezeptor-Ligandenbindungsregion durch eine Veränderung einer primären Sequenz in etwa 13 bis 42 carboxyterminalen Aminosäuren einer Wildtyp-Progesteronrezeptor-Ligandenbindungsdomäne mutiert ist.

3. Modifiziertes Steroidhormonrezeptorprotein nach Anspruch 2, wobei die Progesteronrezeptor-Ligandenbindungsregion im Wesentlichen aus Aminosäuren besteht, die aus der Gruppe bestehend aus 640 bis 914, 640 bis 917 oder 640 bis 920 einer Progesteronrezeptor-Ligandenbindungsdomäne ausgewählt sind.

4. Modifiziertes Steroidhormonrezeptorprotein nach einem der vorhergehenden Ansprüche, wobei die Transregulationsdomäne die Genexpression transaktivieren oder transreprimieren kann.

5. Modifiziertes Steroidhormonrezeptorprotein nach einem der vorhergehenden Ansprüche, wobei die DNA-Bindungsdomäne durch eine DNA-Birtdungsdomäne ersetzt wird, die normalerweise nicht mit der Progesteron-Ligandenbindungsdomäne assoziiert wird.

6. Modifiziertes Steroidhormonrezeptorprotein nach einem der vorhergehenden Ansprüche, wobei es sich bei der DNA-Bindungsdomäne um eine GAL-4-DNA-Bindungsdomäne handelt.

7. Modifiziertes Steroidhormonrezeptorprotein nach einem der vorhergehenden Ansprüche, wobei es sich bei der Transregulationsdomäne um eine Transaktivierungsdomäne handelt.

8. Modifiziertes Steroidhormonrezeptorprotein nach einem der vorhergehenden Ansprüche, wobei es sich bei der Transregulationsdomäne um eine Transrepressionsdomäne handelt.

9. Modifiziertes Steroidhormonrezeptorprotein nach einem der vorhergehenden Ansprüche, wobei die Transregulationsdomäne eine Krüppel-assoziierte Box-A-Transrepressionsdomäne (KRAB-Transrepressionsdomäne) umfasst.

10. Modifiziertes Steroidhormonrezeptorprotein nach Anspruch 9, wobei es sich bei der KRAB-Transrepressionsdomäne um eine Kid-1- oder Kox1-KRAB-Transrepressionsdomäne handelt.

11. Modifiziertes Steroidhormonrezeptorprotein nach Anspruch 10, wobei es sich bei der KRAB-Transrepressionsdomäne um eine Kid-1-KRAB-Transrepressionsdomäne handelt.

12. Modifiziertes Steroidhormonrezeptorprotein nach einem der vorhergehenden Ansprüche, wobei die mutierte Progesteronrezeptor-Ligandenbindungsdomäne einen synthetischen Antagonisten bindet, der ausgewählt ist aus der Gruppe bestehend aus: RU486, Org31806 und Org31376.

13. Modifiziertes Steroidhormonrezeptorprotein nach Anspruch 12, wobei die mutierte Progesteronrezeptor-Ligandenbindungsregion auf RU486 in einer Konzentration von nur 0,01 nM reagieren kann.

14. Modifiziertes Steroidhormonrezeptorprotein nach einem der vorhergehenden Ansprüche, wobei das Rezeptorprotein in folgender Reihenfolge vom N-Terminus zum C-Terminus die DNA-Bindungsdomäne, die mutierte Progesteronrezeptor-Ligandenbindungsregion und die Transregulationsdomäne umfasst.

15. Nukleinsäuresequenz, die ein modifiziertes Steroidhormonrezeptorprotein nach einem der vorhergehenden Ansprüche kodiert.

16. Nukleinsäuresequenz nach Anspruch 15, die weiterhin einen gewebespezifischen Promotor umfasst.

17. Vektor, der die Nukleinsäuresequenz nach Anspruch 15 oder 16 enthält.

18. Zelle, die mit einem Vektor nach Anspruch 17 transfziert oder transformiert wurde.

19. In-vitro-Verfahren zum Herstellen einer transformierten Zelle, das den Schritt des Inkontaktbringens der Zelle mit einem Vektor nach Anspruch 17 für einen Zeitraum, der dazu ausreicht, die Zelle zu transformieren, umfasst, wobei die transformierte Zelle ein modifiziertes Steroidhormonrezeptorprotein exprimiert, das von dem Vektor kodiert wird.

20. Verwendung des Vektors nach Anspruch 17 bei der Herstellung eines Arzneimittels zum Abgeben der Nukleinsäuresequenz, die das modifizierte Steroidhormonrezeptorprotein kodiert, an einen Säuger *in vivo.*

21. Verwendung des Vektors nach Anspruch 17 bei der Herstellung eines Arzneimittels zum Abgeben der Nukleinsäuresequenz, die das modifizierte Steroidhormonrezeptorprotein kodiert, an einen Säuger *in vivo* zum Regulieren der Expression von Neuritwachstumsfaktor.

22. Transgenes, nicht menschliches Tier, dessen Zellen die Nukleinsäure nach Anspruch 15 oder 16 oder den Vektor nach Anspruch 17 enthalten.

## Revendications

1. Protéine réceptrice d'hormone stéroïdienne modifiée, dans laquelle ladite protéine réceptrice répond à un antagoniste conventionnel d'une contrepartie de protéine réceptrice de progestérone de type sauvage avec une réponse agoniste, et dans laquelle ladite protéine réceptrice modifiée comprend un domaine de liaison à l'ADN, un domaine transrégulateur et une région de liaison au ligand du récepteur de progestérone mutée, **caractérisée en ce que** le domaine transrégulateur est hétérologue au domaine de liaison au ligand et est situé en une position C-terminale dans ladite protéine réceptrice d'hormone stéroïdienne modifiée.

2. Protéine réceptrice d'hormone stéroïdienne modifiée selon la revendication 1, dans laquelle la région de liaison au ligand du récepteur de progestérone est mutée par une altération d'une séquence primaire dans environ 13 à 42 acides aminés carboxyl-terminaux d'un domaine de liaison au ligand du récepteur de progestérone de type sauvage.

3. Protéine réceptrice d'hormone stéroïdienne modifiée selon la revendication 2, dans laquelle la région de liaison au ligand du récepteur de progestérone est constituée essentiellement d'acides aminés sélectionnés parmi le groupe constitué de 640 à 914, 640 à 917 ou 640 à 920 d'un domaine de liaison au ligand du récepteur de progestérone.

4. Protéine réceptrice d'hormone stéroïdienne modifiée selon l'une quelconque des revendications précédentes, dans laquelle ledit domaine transrégulateur est capable de transactiver ou transréprimer l'expression génique.

5. Protéine réceptrice d'hormone stéroïdienne modifiée selon l'une quelconque des revendications précédentes, dans laquelle le domaine de liaison à l'ADN est remplacé par un domaine de liaison à l'ADN qui n'est pas normalement associé au domaine de liaison au ligand de progestérone.

6. Protéine réceptrice d'hormone stéroïdienne modifiée selon l'une quelconque des revendications précédentes, dans laquelle le domaine de liaison à l'ADN est un domaine de liaison à l'ADN GAL-4.

7. Protéine réceptrice d'hormone stéroïdienne modifiée selon l'une quelconque des revendications précédentes, dans laquelle le domaine transrégulateur est un domaine de transactivation.

8. Protéine réceptrice d'hormone stéroïdienne modifiée selon l'une quelconque des revendications précédentes, dans laquelle le domaine transrégulateur est un domaine de transrépression.

9. Protéine réceptrice d'hormone stéroïdienne modifiée selon l'une quelconque des revendications précédentes, dans laquelle ledit domaine transrégulateur comprend un domaine de transrépression box-A associé à Krüppel (KRAB).

10. Protéine réceptrice d'hormone stéroïdienne modifiée selon la revendication 9, dans laquelle le domaine de transrépression KRAB est un domaine de transrépression KRAB Kid-1 ou Koxl.

11. Protéine réceptrice d'hormone stéroïdienne modifiée selon la revendication 10, dans laquelle le domaine de transrépression KRAB est un domaine de transrépression KRAB Kid-1.

12. Protéine réceptrice d'hormone stéroïdienne modifiée selon l'une quelconque des revendications précédentes, dans laquelle le domaine de liaison au ligand du récepteur de progestérone muté se fixe à un antagoniste synthétique sélectionné parmi le groupe constitué de : RU486, Org31806 et Org 31376.

13. Protéine réceptrice d'hormone stéroïdienne modifiée selon la revendication 12, dans laquelle ladite région de liaison au ligand du récepteur de progestérone mutée est capable de répondre à RU486 à une concentration aussi faible que 0,01 nM.

14. Protéine réceptrice d'hormone stéroïdienne modifiée selon l'une quelconque des revendications précédentes, dans laquelle la protéine réceptrice comprend, en ordre de la terminaison N à la terminaison C, le domaine de liaison à l'ADN, la région de liaison au ligand du récepteur de progestérone mutée et le domaine transrégulateur.

15. Séquence d'acide nucléique codant pour une protéine réceptrice d'hormone stéroïdienne modifiée selon l'une quelconque des revendications précédentes.

16. Séquence d'acide nucléique selon la revendication 15, comprenant en outre un promoteur spécifique au tissu.

17. Vecteur contenant la séquence d'acide nucléique selon la revendication 15 ou la revendication 16.

18. Cellule transfectée ou transformée avec un vecteur selon la revendication 17.

19. Procédé in vitro de fabrication d'une cellule transformée comprenant l'étape consistant à mettre en contact ladite cellule avec un vecteur selon la revendication 17 pendant une durée suffisante pour transformer ladite cellule, dans lequel ladite cellule transformée exprime une protéine réceptrice d'hormone stéroïdienne modifiée codée par ledit vecteur.

20. Utilisation du vecteur selon la revendication 17 dans la fabrication d'un médicament pour la fourniture de la séquence d'acide nucléique codant pour la protéine réceptrice d'hormone stéroïdienne modifiée à un mammifère in vivo.

21. Utilisation du vecteur selon la revendication 17 dans la fabrication d'un médicament pour la fourniture de la séquence d'acide nucléique codant pour la protéine réceptrice d'hormone stéroïdienne modifiée à un mammifère in vivo pour réguler l'expression du facteur de croissance de neurite.

22. Animal non humain transgénique dont les cellules contiennent l'acide nucléique selon la revendication 15 ou la revendication 16 ou le vecteur selon la revendication 17.
